# EUROPEAN PATENT SPECIFICATION

(11) **EP 2 518 110 B1**
(45) Date of publication and mention of the grant of the patent: **13.12.2017**
(21) Application number: 10839615.1
(22) Date of filing: 20.12.2010
(51) Int. Cl.: C08L 65/00, C08K 5/07, C08K 5/151, C08K 5/28, C08K 5/3412, H01L 51/30, H01L 51/42, H01L 51/50, C07C 25/24, C07D 307/91

(54) **COMPOSITION AND LIGHT EMITTING ELEMENT USING THE COMPOSITION**
ZUSAMMENSETZUNG UND DIE ZUSAMMENSETZUNG VERWENDENDES LICHTEMITTIERENDES ELEMENT
COMPOSITION ET ÉLÉMENT ÉLECTROLUMINESCENT UTILISANT LA COMPOSITION

(30) Priority: 25.12.2009 JP 2009294385
(43) Date of publication of application: 31.10.2012
(73) Proprietor: Sumitomo Chemical Company, Limited, Tokyo 104-8260 (JP)
(72) Inventor: NAKATANI, Tomoya, Tsukuba-shi Ibaraki 305-0821 (JP); KAWAGUCHI, Keiko, Karatsu-shi Saga 847-0832 (JP); SASAKI, Shigeru, Tsukuba-shi Ibaraki 300-3261 (JP)
(74) Representative: J A Kemp
(86) International application number: PCT/JP2010/073650
(87) International publication number: WO 2011/078387

(56) References cited:
- EP-A1- 2 067 805
- WO-A1-2010/013724
- WO-A1-2010/016555
- JP-A- 2007 302 886
- JP-A- 2008 106 241
- JP-A- 2008 517 135
- JP-A- 2008 522 238
- US-A1- 2008 026 250

## Description

### TECHNICAL FIELD

The present invention relates to a composition and a light emitting device using the composition.

### BACKGROUND ART

Materials used in light emitting devices such as an organic electroluminescent device and the like are variously investigated recently. Of them, a compound containing a crosslinkable group having a benzocyclobutene structure is known as a material useful for a light emitting device of lamination type (JP-A No. 2008-106241).

JP2007302886 describes an organic electronic material containing a compound having an epoxy group or an oxetane group and a polymer.

### DISCLOSURE OF THE INVENTION

The above-described material, however, has an insufficient curable property in a low temperature region.

The present invention has an object of providing a material showing an excellent curable property in a low temperature region.

The present invention provides a composition, a film, a light emitting device, a surface light source, a display, an organic transistor, an organic photoelectric conversion device and the like described below.
[1] A composition comprising
   (i) a crosslinkable polymer compound comprising a crosslinkable group and being capable of showing at least one of a light emitting property and charge transportability, and
   (ii) a crosslinkable low molecular weight compound having a single molecular weight which is 1x10² or more and less than 1x10⁴, comprising a crosslinkable group and being capable of showing at least one of a light emitting property and charge transportability,
   characterised in that one of said crosslinkable polymer compound and said crosslinkable low molecular weight compound has a group represented by any of formulae (Z-1) and (Z-2) as said crosslinkable group, and the other has a group represented by formula (Z-5) as said crosslinkable group: 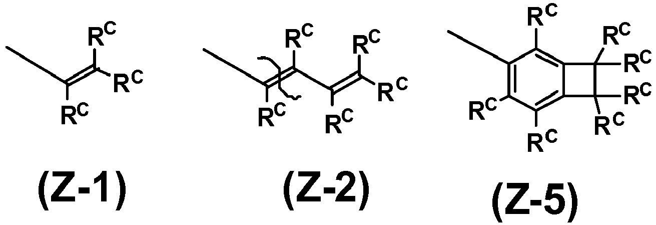 in the formulae (Z-1), (Z-2) and (Z-5), each R^{c} represents a hydrogen atom, an alkyl group, an alkoxy group, an alkylthio group, an aryl group, an aryloxy group, an arylthio group, an arylalkyl group, an arylalkoxy group, an arylalkylthio group, an amino group, a silyl group, a halogen atom, an acyl group, an acyloxy group, an imine residue, a carbamoyl group, an acid imide group, a monovalent heterocyclic group, a carboxyl group, a cyano group or a nitro group, and the group represented by R^{c} may have a substituent, wherein the plurality of R^{c} may be the same or different.
[2] The composition according to [1], wherein said crosslinkable low molecular weight compound is an aromatic hydrocarbon.
[3] The composition according to [1], wherein said crosslinkable low molecular weight compound is a heterocyclic compound.
[4] The composition according to any one of [1] to [3], wherein said crosslinkable polymer compound has, as a repeating unit, at least one member selected from the group consisting of an arylene group comprising 1 to 4 groups represented by the following formula (I) and optionally comprising a substituent, a divalent heterocyclic group comprising 1 to 4 groups represented by the following formula (I) and optionally comprising a substituent and a divalent aromatic amine residue comprising 1 to 4 groups represented by the following formula (I) and optionally comprising a substituent: 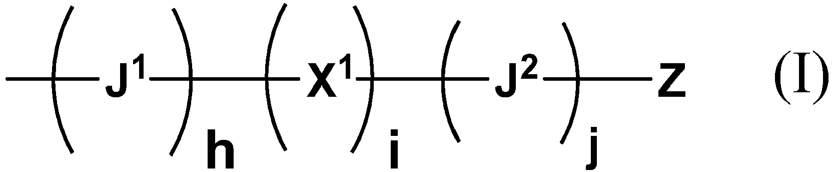 in the formula (I), Z represents a group represented by any of said formulae (Z-1) to (Z-12), J¹ represents a phenylene group optionally comprising a substituent, J² represents an alkylene group optionally comprising a substituent, X¹ represents an oxygen atom or a sulfur atom, h and i are each independently 0 or 1, and j is an integer of 0 to 3.
[5] The composition according to any one of [1] to [4], wherein the weight ratio of said crosslinkable polymer compound to said crosslinkable low molecular weight compound is 99:1 to 50:50.
[6] The composition according to any one of [1] to [5], wherein the total amount of said crosslinkable groups contained in one gram of the composition is 1.0x10⁻⁶ to 1.0x10⁻² mol.
[7] The composition according to any one of [1] to [6], wherein the composition further comprises a solvent.
[8] A film; or
   an organic transistor; or
   an organic photoelectric device; or
   a light emitting device comprising electrodes consisting of an anode and a cathode and a layer that is disposed between the electrodes;
   wherein the film, the organic transistor, the organic photoelectric device, or the layer comprise the composition according to any one of [1] to [7].
[9] A surface light source or a display comprising the light emitting device according to [8].

### MODE FOR CARRYING OUT THE INVENTION

The present invention will be illustrated in detail below.

In the present invention, when the crosslinkable polymer compound is fabricated into a film having a thickness of 30 to 60 nm and if the PL quantum yield is 5% or more (preferably, 10% or more) at an excitation wavelength of 325 nm, this compound is said to have a light emitting property. When the crosslinkable low molecular weight compound is fabricated into a film having a thickness of 30 to 60 nm and if the PL quantum yield is 5% or more (preferably, 10% or more) at an excitation wavelength of 250 nm, this compound is said to have a light emitting property. The method of fabricating a film for measurement of the PL quantum yield is a method of film formation from a solution or melted state in the case of the crosslinkable polymer compound, and is a vacuum vapor deposition method from a powder or a method of film formation from a solution or melted state in the case of the crosslinkable low molecular weight compound. For measurement of the PL quantum yield, for example, PL Quantum Yield Measurement System (c9920-02) manufactured by Hamamatsu Photonics, Japan can be used.

In the present invention, when the crosslinkable polymer compound or the crosslinkable low molecular weight compound is fabricated into a film (0.1 to 20 µm) and if the hole mobility of the film measured by the Time of Flight method at an excitation wavelength of 337 nm by nitrogen laser is 10⁻⁷ cm²/V/s (cm²/(V·s)) or more, this compound is said to have hole transportability, if the electron mobility of the film is 10⁻⁷ cm²/V/s or more, this compound is said to have electron transportability, and if the compound has at least one of hole transportability and electron transportability, this compound is said to have charge transportability. For measurement of mobility (charge transportability), for example, a photoexcitation carrier mobility measurement apparatus (TOF401) manufactured by Sumitomo Heavy Industries, Ltd. can be used.

### <Composition>

The composition of the present invention is a composition comprising (i) a crosslinkable polymer compound having a crosslinkable group and being capable of showing at least one of a light emitting property and charge transportability (hereinafter, referred to as "crosslinkable polymer compound"), and (ii) a crosslinkable low molecular weight compound having a crosslinkable group and being capable of showing at least one of a light emitting property and charge transportability (hereinafter, referred to as "crosslinkable low molecular weight compound").

The above-described crosslinkable group is a group causing a crosslinking reaction by a stimulus such as heat, light and the like, and are groups represented by the following formula (Z-1), (Z-2) or (Z-5). These are preferable from the standpoint of easy crosslinkage.

The crosslinkable group of the crosslinkable polymer compound and the crosslinkable group of the crosslinkable low molecular weight compound are different. This is preferable from the standpoint of promotion of a crosslinking reaction at low temperatures.

In the present invention, "crosslinkable groups are identical" means a case in which the kind of the crosslinkable group of the crosslinkable polymer compound and the kind of the crosslinkable group of the crosslinkable low molecular weight compound are identical (when two or more kinds of crosslinkable groups are present, the kinds and the numbers thereof are identical), and "crosslinkable groups are different" means a case other than this. "The kinds of the crosslinkable groups are identical" means that the basic structures of the crosslinkable groups are identical. For example, two different crosslinkable groups belonging to (Z-1) are identical in the kind.

The same shall apply also to two different crosslinkable groups belonging to (Z-2), and two different crosslinkable groups belonging to (Z-5).

Combinations of crosslinkable groups include a combination of a group represented by the following formula (Z-1) and a group represented by the following formula (Z-5), a combination of a group represented by the following formula (Z-2) and a group represented by the following formula (Z-5), and a combination of a group represented by the following formula (Z-1), a group represented by the following formula (Z-2) and a group represented by the following formula (Z-5). This is preferable from the standpoint of curability at low temperature. 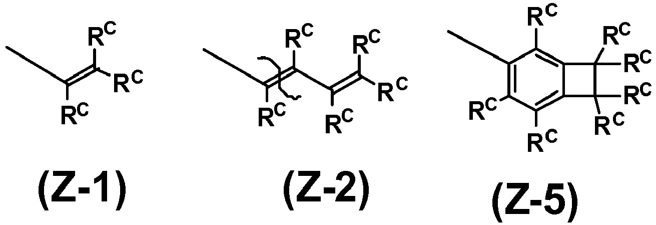 in the formulae (Z-1), (Z-2) and (Z-5), each R^{c} represents a hydrogen atom, an alkyl group, an alkoxy group, an alkylthio group, an aryl group, an aryloxy group, an arylthio group, an arylalkyl group, an arylalkoxy group, an arylalkylthio group, an amino group, a silyl group, a halogen atom, an acyl group, an acyloxy group, an imine residue, a carbamoyl group, an acid imide group, a monovalent heterocyclic group, a carboxyl group, a cyano group or a nitro group, and the group represented by RC may have a substituent, wherein the plurality of R^{c} may be the same or different.

A compound having a double bond with a wavy line represented by the following formula in the above-described formulae (Z-2): 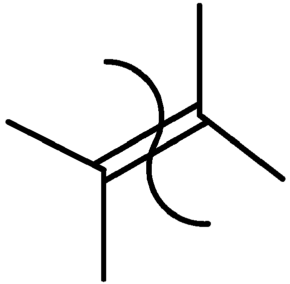 means that any of an E-isomer and a Z-isomer may be permissible. The same shall apply in the following structural formulae.

The alkyl group represented by the above-described R^{c} may be any of linear, branched or cyclic and may have a substituent. The above-described alkyl group has a carbon atom number of usually 1 to 20, and examples thereof include a methyl group, an ethyl group, a propyl group, an isopropyl group, a butyl group, an isobutyl group, a tert-butyl group, a pentyl group, a hexyl group, a cyclohexyl group, a heptyl group, an octyl group, a 2-ethylhexyl group, a nonyl group, a decyl group, a 3,7-dimethyloctyl group, a lauryl group, a trifluoromethyl group, a pentafluoroethyl group, a perfluorobutyl group, a perfluorohexyl group and a perfluorooctyl group.

The alkoxy group represented by the above-described R^{c} may be any of linear, branched or cyclic and may have a substituent. The above-described alkoxy group has a carbon atom number of usually 1 to 20, and examples thereof include a methoxy group, an ethoxy group, a propyloxy group, an isopropyloxy group, a butoxy group, an isobutoxy group, a tert-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, a lauryloxy group, a trifluoromethoxy group, a pentafluoroethoxy group, a perfluorobutoxy group, a perfluorohexyloxy group, a perfluorooctyloxy group, a methoxymethyloxy group a 2-methoxyethyloxy group.

The alkylthio group represented by the above-described R^{c} may be any of linear, branched or cyclic and may have a substituent. The above-described alkylthio group has a carbon atom number of usually 1 to 20, and examples thereof include a methylthio group, an ethylthio group, a propylthio group, an isopropylthio group, a butylthio group, an isobutylthio group, a tert-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group, a laurylthio group and a trifluoromethylthio group.

The aryl group represented by the above-described R^{c} is an atomic group obtained by removing one hydrogen atom from an aromatic hydrocarbon, and includes groups having a condensed ring, and groups having two or more independent benzene rings or condensed rings linked directly. The above-described aryl group may have a substituent. The above-described aryl group has a carbon atom number of usually 6 to 60, preferably 7 to 48, and examples thereof include a phenyl group, C₁ to C₁₂ alkoxyphenyl groups ("C₁ to C₁₂ alkoxy" means that the carbon atom number of an alkoxy portion is 1 to 12. The same shall apply hereinafter.), C₁ to C₁₂ alkylphenyl groups ("C₁ to C₁₂ alkyl" means that the carbon atom number of an alkyl portion is 1 to 12. The same shall apply hereinafter.), a 1-naphthyl group, a 2-naphthyl group, a 1-anthracenyl group, a 2-anthracenyl group, a 9-anthracenyl group and a pentafluorophenyl group, and preferable are C₁ to C₁₂ alkoxyphenyl groups and C₁ to C₁₂ alkylphenyl groups.

The C₁ to C₁₂ alkoxyphenyl group includes a methoxyphenyl group, an ethoxyphenyl group, a propyloxyphenyl group, an isopropyloxyphenyl group, a butoxyphenyl group, an isobutoxyphenyl group, a tert-butoxyphenyl group, a pentyloxyphenyl group, a hexyloxyphenyl group, a cyclohexyloxyphenyl group, a heptyloxyphenyl group, an octyloxyphenyl group, a 2-ethylhexyloxyphenyl group, a nonyloxyphenyl group, a decyloxyphenyl group, a 3,7-dimethyloctyloxyphenyl group and a lauryloxyphenyl group and the like.

The C₁ to C₁₂ alkylphenyl group includes a methylphenyl group, an ethylphenyl group, a dimethylphenyl group, a propylphenyl group, a mesityl group, a methylethylphenyl group, an isopropylphenyl group, a butylphenyl group, an isobutylphenyl group, a tert-butylphenyl group, a pentylphenyl group, an isoamylphenyl group, a hexylphenyl group, a heptylphenyl group, an octylphenyl group, a nonylphenyl group, a decylphenyl group, a dodecylphenyl group and the like.

The aryloxy group represented by the above-described R^{c} may have a substituent, and the carbon atom number thereof is usually 6 to 60, preferably 7 to 48. The above-described aryloxy group includes a phenoxy group, C₁ to C₁₂ alkoxyphenoxy groups, C₁ to C₁₂ alkylphenoxy groups, a 1-naphthyloxy group, a 2-naphthyloxy group, a pentafluorophenyloxy group and the like, and preferable are C₁ to C₁₂ alkoxyphenoxy groups and C₁ to C₁₂ alkylphenoxy groups.

The C₁ to C₁₂ alkoxyphenoxy group includes a methoxyphenoxy group, an ethoxyphenoxy group, a propyloxyphenoxy group, an isopropyloxyphenoxy group, a butoxyphenoxy group, an isobutoxyphenoxy group, a tert-butoxyphenoxy group, a pentyloxyphenoxy group, a hexyloxyphenoxy group, a cyclohexyloxyphenoxy group, a heptyloxyphenoxy group, an octyloxyphenoxy group, a 2-ethylhexyloxyphenoxy group, a nonyloxyphenoxy group, a decyloxyphenoxy group, a 3,7-dimethyloctyloxyphenoxy group, a lauryloxyphenoxy group and the like.

The C₁ to C₁₂ alkylphenoxy group include a methylphenoxy group, an ethylphenoxy group, a dimethylphenoxy group, a propylphenoxy group, a 1,3,5-trimethylphenoxy group, a methylethylphenoxy group, an isopropylphenoxy group, a butylphenoxy group, an isobutylphenoxy group, a tert-butylphenoxy group, a pentylphenoxy group, an isoamylphenoxy group, a hexylphenoxy group, a heptylphenoxy group, an octylphenoxy group, a nonylphenoxy group, a decylphenoxy group, a dodecylphenoxy group and the like.

The arylthio group represented by the above-described R^{c} may have a substituent, and the carbon atom number thereof is usually 3 to 60. Examples of the above-described arylthio group include a phenylthio group, C₁ to C₁₂ alkoxyphenylthio groups, C₁ to C₁₂ alkylphenylthio groups, a 1-naphthylthio group, a 2-naphthylthio group and a pentafluorophenylthio group.

The arylalkyl group represented by the above-described R^{c} may have a substituent, and the carbon atom number thereof is usually 7 to 60. Examples of the above-described arylalkyl group include phenyl-C₁ to C₁₂ alkyl groups, C₁ to C₁₂ alkoxyphenyl-C₁ to C₁₂ alkyl groups, C₁ to C₁₂ alkylphenyl-C₁ to C₁₂ alkyl groups, 1-naphthyl-C₁ to C₁₂ alkyl groups and 2-naphthyl-C₁ to C₁₂ alkyl groups.

The arylalkoxy group represented by the above-described R^{c} may have a substituent, and the carbon atom number thereof is usually 7 to 60. Examples of the above-described arylalkoxy group include phenyl-C₁ to C₁₂ alkoxy groups, C₁ to C₁₂ alkoxyphenyl-C₁ to C₁₂ alkoxy groups, C₁ to C₁₂ alkylphenyl-C₁ to C₁₂ alkoxy groups, 1-naphthyl-C₁ to C₁₂ alkoxy groups and 2-naphthyl-C₁ to C₁₂ alkoxy groups.

The arylalkylthio group represented by the above-described R^{c} may have a substituent, and the carbon atom number thereof is usually 7 to 60. Examples of the above-described arylalkylthio group include phenyl-C₁ to C₁₂ alkylthio groups, C₁ to C₁₂ alkoxyphenyl-C₁ to C₁₂ alkylthio groups, C₁ to C₁₂ alkylphenyl-C₁ to C₁₂ alkylthio groups, 1-naphthyl-C₁ to C₁₂ alkylthio groups and 2-napthyl-C₁ to C₁₂ alkylthio groups.

The amino group represented by the above-described R^{c} may be an unsubstituted amino group, or an amino group obtained by substitution of one or two hydrogen atoms of an amino group with one or two groups selected from the group consisting of an alkyl group, an aryl group, an arylalkyl group and a monovalent heterocyclic group (hereinafter, referred to as "substituted amino group").

The substituted amino group may further have a substituent, and the carbon atom number thereof is usually 1 to 60, preferably 2 to 48.

Examples of the above-described substituted amino group include a methylamino group, a dimethylamino group, an ethylamino group, a diethylamino group, a propylamino group, a dipropylamino group, an isopropylamino group, a diisopropylamino group, a butylamino group, a sec-butylamino group, an isobutylamino group, a tert-butylamino group, a pentylamino group, a hexylamino group, a cyclohexylamino group, a heptylamino group, an octylamino group, a 2-ethylhexylamino group, a nonylamino group, a decylamino group, a 3,7-dimethyloctylamino group, a laurylamino group, a cyclopentylamino group, a dicyclopentylamino group, a dicyclohexylamino group, a pyrrolidyl group, a piperidyl group, a ditrifluoromethylamino group, a phenylamino group, a diphenylamino group, C₁ to C₁₂ alkoxyphenylamino groups, di(C₁ to C₁₂ alkoxyphenyl)amino groups, di(C₁ to C₁₂ alkylphenyl)amino groups, a 1-naphthylamino group, a 2-naphthylamino group, a pentafluorophenylamino group, a pyridylamino group, a pyridazinylamino group, a pyrimidylamino group, a pyrazylamino group, a triazylamino group, phenyl-C₁ to C₁₂ alkylamino groups, C₁ to C₁₂ alkoxyphenyl-C₁ to C₁₂ alkylamino groups, C₁ to C₁₂ alkylphenyl-C₁ to C₁₂ alkylamino groups, di(C₁ to C₁₂ alkoxyphenyl-C₁ to C₁₂ alkyl)amino groups, di(C₁ to C₁₂ alkylphenyl-C₁ to C₁₂ alkyl)amino groups, 1-naphthyl-C₁ to C₁₂ alkylamino groups and 2-naphthyl-C₁ to C₁₂ alkylamino groups.

The silyl group represented by the above-described R^{c} may be an unsubstituted silyl group, or a silyl group obtained by substitution of one, two or three hydrogen atoms of a silyl group with one, two or three groups selected from the group consisting of an alkyl group, an aryl group, an arylalkyl group and a monovalent heterocyclic group (hereinafter, referred to as "substituted silyl group").

The above-described substituted silyl group may further have a substituent, and the carbon atom number thereof is usually 1 to 60, preferably 3 to 48.

Examples of the above-described substituted silyl group include a trimethylsilyl group, a triethylsilyl group, a tripropylsilyl group, a tri-isopropylsilyl group, a dimethyl-isopropylsilyl group, a diethyl-isopropylsilyl group, a tert-butyldimethylsilyl group, a pentyldimethylsilyl group, a hexyldimethylsilyl group, a heptyldimethylsilyl group, an octyldimethylsilyl group, a 2-ethylhexyl-dimethylsilyl group, a nonyldimethylsilyl group, a decyldimethylsilyl group, a 3,7-dimethyloctyl-dimethylsilyl group, a lauryldimethylsilyl group, phenyl-C₁ to C₁₂ alkylsilyl groups, C₁ to C₁₂ alkoxyphenyl-C₁ to C₁₂ alkylsilyl groups, C₁ to C₁₂ alkylphenyl-C₁ to C₁₂ alkylsilyl groups, 1-naphthyl-C₁ to C₁₂ alkylsilyl groups, 2-naphthyl-C₁ to C₁₂ alkylsilyl groups, phenyl-C₁ to C₁₂ alkyldimethylsilyl groups, a triphenylsilyl group, a tri-p-xylylsilyl group, a tribenzylsilyl group, a diphenylmethylsilyl group, a tert-butyldiphenylsilyl group and a dimethylphenylsilyl group.

Examples of the halogen atom represented by the above-described R^{c} include a fluorine atom, a chlorine atom, a bromine atom and an iodine atom.

The acyl group represented by the above-described R^{c} may have a substituent, and the carbon atom number thereof is usually 1 to 20, preferably 2 to 18. Examples of the above-described acyl group include an acetyl group, a propionyl group, a butyryl group, an isobutyryl group, a pivaloyl group, a benzoyl group, a trifluoroacetyl group and a pentafluorobenzoyl group.

The acyloxy group represented by the above-described R^{c} may have a substituent, and the carbon atom number thereof is usually 1 to 20, preferably 2 to 18. Examples of the above-described acyloxy group include an acetoxy group, a propionyloxy group, a butyryloxy group, an isobutyryloxy group, a pivaloyloxy group, a benzoyloxy group, a trifluoroacetyloxy group and a pentafluorobenzoyloxy group.

The imine residue represented by the above-described R^{c} is an atomic group obtained by removing one hydrogen atom from an imine compound (meaning a compound having - N=C- in the molecule. Examples thereof include aldimines, ketimines, and compounds obtained by substituting a hydrogen atom linked to a nitrogen atom contained in these compounds with an alkyl group or the like.), may have a substituent, and the carbon atom number thereof is usually 2 to 20, preferably 2 to 18.

Examples of the above-described imine residue include groups represented by the following formulae. 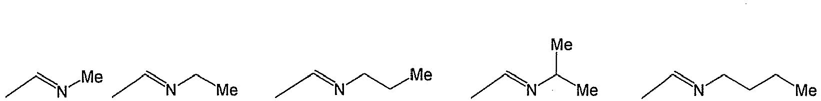 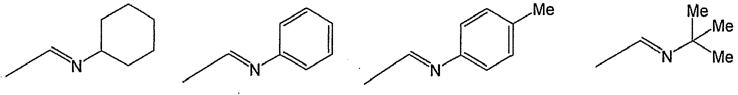 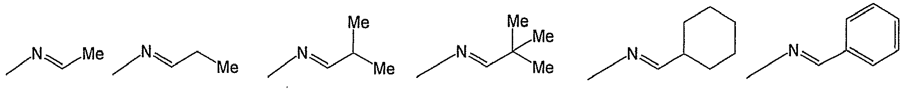 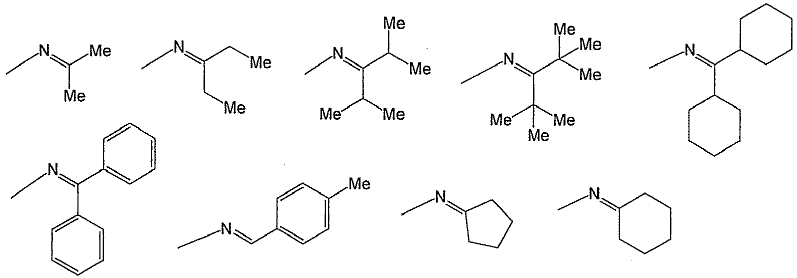 (wherein Me represents a methyl group. The same shall apply hereinafter.)

The carbamoyl group represented by the above-described R^{c} may have a substituent, and the carbon atom number thereof is usually 1 to 20, preferably 2 to 20, more preferably 2 to 18. Examples of the above-described carbamoyl group include a formamide group, an acetamide group, a propioamide group, a butyroamide group, a benzamide group, a trifluoroacetamide group, a pentafluorobenzamide group, a diformamide group, a diacetamide group, a dipropioamide group, a dibutyroamide group, a dibenzamide group, a ditrifluoroacetamide group and a dipentafluorobenzamide group.

The acid imide group represented by the above-described R^{c} is an atomic group obtained by removing from an acid imide a hydrogen atom linked to its nitrogen atom, may have a substituent, and the carbon atom number thereof is usually 4 to 20. Examples of the above-described acid imide group include following groups.

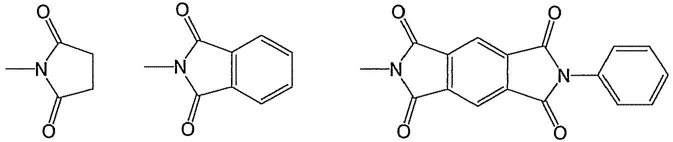

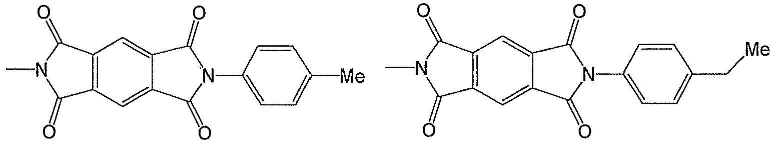

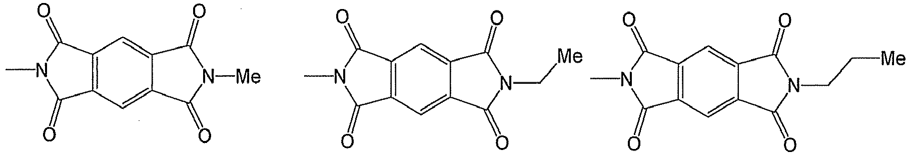

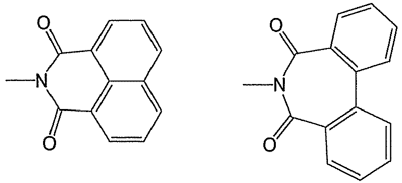

The monovalent heterocyclic group represented by the above-described R^{C} means an atomic group remaining after removal of one hydrogen atom from a heterocyclic compound, and the carbon atom number thereof is usually 4 to 60, preferably 4 to 20. Of the monovalent heterocyclic groups, monovalent aromatic heterocyclic groups are preferable. The carbon atom number of the monovalent heterocyclic group does not include the carbon atom number of the substituent. Here, the heterocyclic compound includes organic compounds having a cyclic structure in which elements constituting the ring include not only a carbon atom but also a hetero atom such as an oxygen atom, a sulfur atom, a nitrogen atom, a phosphorus atom, a boron atom and the like contained in the ring. Examples of the above-described monovalent heterocyclic group include a thienyl group, C₁ to C₁₂ alkylthienyl groups, a pyrrolyl group, a furyl group, a pyridyl group, C₁ to C₁₂ alkylpyridyl groups, a piperidyl group, a quinolyl group and an isoquinolyl group, and preferable are a thienyl group, C₁ to C₁₂ alkylthienyl groups, a pyridyl group and C₁ to C₁₂ alkylpyridyl groups.

The carboxyl group represented by the above-described R^{C} may be an unsubstituted carboxyl group, or a carboxyl group of which hydrogen atom is substituted by an alkyl group, an aryl group, an arylalkyl group or a monovalent heterocyclic group (hereinafter, referred to as "substituted carboxyl group").

The above-described substituted carboxyl group may further have a substituent, and the carbon atom number thereof is usually 2 to 60, preferably 2 to 48.

Examples of the above-described substituted carboxyl group include a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, a pentyloxycarbonyl group, a hexyloxycarbonyl group, a cyclohexyloxycarbonyl group, a heptyloxycarbonyl group, an octyloxycarbonyl group, a 2-ethylhexyloxycarbonyl group, a nonyloxycarbonyl group, a decyloxycarbonyl group, a 3,7-dimethyloctyloxycarbonyl group, a dodecyloxycarbonyl group, a trifluoromethoxycarbonyl group, a pentafluoroethoxycarbonyl group, a perfluorobutoxycarbonyl group, a perfluorohexyloxycarbonyl group, a perfluorooctyloxycarbonyl group, a phenoxycarbonyl group, a naphthoxycarbonyl group and a pyridyloxycarbonyl group. The alkyl group, the aryl group, the arylalkyl group and the monovalent heterocyclic group may have a substituent.

The substituent which can be carried on the group represented by the above-described R^{c} includes an alkyl group, an aryl group, a fluoro group and a monovalent heterocyclic group.

### -Crosslinkable polymer compound-

The above-described crosslinkable polymer compound is usually a compound having a distribution in the molecular weight and having a polystyrene-equivalent number-average molecular weight of 1×10⁴ to 1×10⁸.

The above-described crosslinkable polymer compound is preferably a polymer compound having, as a repeating unit, at least one member selected from the group consisting of an arylene group having 1 to 4 groups represented by the following formula (I) and optionally having a substituent, a divalent heterocyclic group having 1 to 4 groups represented by the following formula (I) and optionally having a substituent and a divalent aromatic amine residue having 1 to 4 groups represented by the following formula (I) and optionally having a substituent (hereinafter, this repeating unit is referred to as "first repeating unit"), from the standpoint of easiness of crosslinkage. 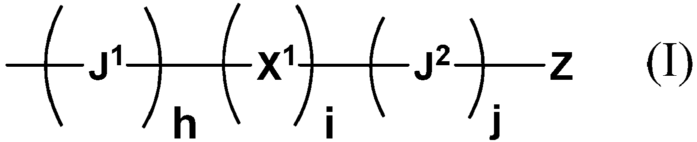 in the formula (I), Z represents a group represented by any of the above-described formulae (Z-1), (Z-2) and (Z-5), J¹ represents a phenylene group optionally having a substituent, J² represents an alkylene group optionally having a substituent, X¹ represents an oxygen atom or a sulfur atom, h and i are each independently 0 or 1, and j is an integer of 0 to 3.

The above-described arylene group is an atomic group obtained by removing two hydrogen atoms from an aromatic hydrocarbon, and includes groups having a condensed ring, and groups having two or more independent benzene rings or condensed rings linked directly or via a vinylene group or the like. The above-described arylene group may have a substituent. The substituent which can be carried on the above-described arylene group includes an alkyl group, an alkoxy group, an alkylthio group, an aryl group, an aryloxy group, an arylthio group, an arylalkyl group, an arylalkoxy group, an arylalkylthio group, an amino group, a substituted amino group, a silyl group, a substituted silyl group, a halogen atom, an acyl group, an acyloxy group, an imine residue, a carbamoyl group, an acid imide group, a monovalent heterocyclic group, a carboxyl group, a substituted carboxyl group, a cyano group and a nitro group, and preferable from the standpoint of the property of a light emitting device are an alkyl group, an alkoxy group, an aryl group, an aryloxy group, an arylalkyl group, an arylalkoxy group, a halogen atom and a cyano group.

The carbon atom number of a portion excluding the substituent in the above-described arylene group is usually 6 to 60, preferably 6 to 20, and the total carbon atom number including the substituent is usually 6 to 100.

Examples of the above-described arylene group include phenylene groups (the following formulae 1 to 3), naphthalenediyl groups (the following formulae 4 to 13), anthracene-diyl groups (the following formulae 14 to 19), biphenyl-diyl groups (the following formulae 20 to 25), terphenyl-diyl groups (the following formulae 26 to 28), condensed ring groups (the following formulae 29 to 35), fluorene-diyl groups (the following formulae 36 to 38) and benzofluorene-diyl groups (the following formulae 39 to 46), and from the standpoint of the durability of a light emitting device, preferable are phenylene groups, naphthalenediyl groups, anthracene-diyl groups, biphenyl-diyl groups, fluorene-diyl groups and benzofluorene-diyl groups, more preferable are phenylene groups, naphthalenediyl groups, anthracene-diyl groups, fluorene-diyl groups and benzofluorene-diyl groups, further preferable are phenylene groups and fluorene-diyl groups. The following groups may have the same substituent as explained and shown as the substituent which can be carried on the above-described arylene group.

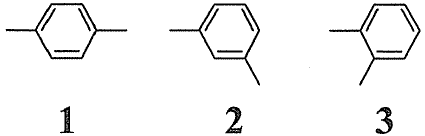

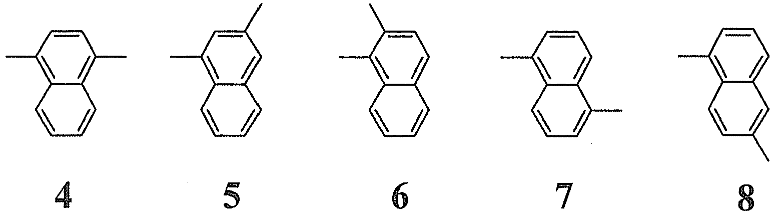

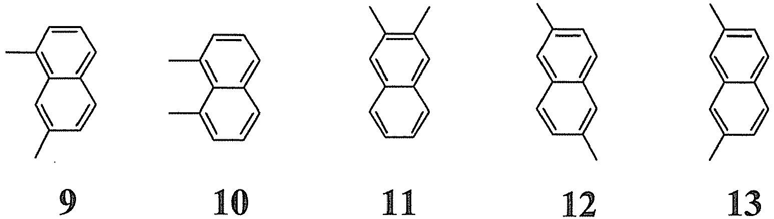

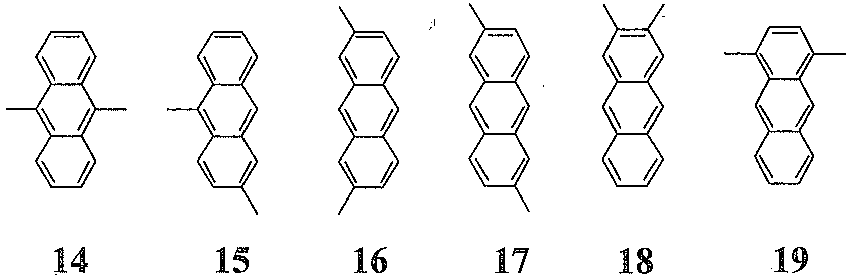

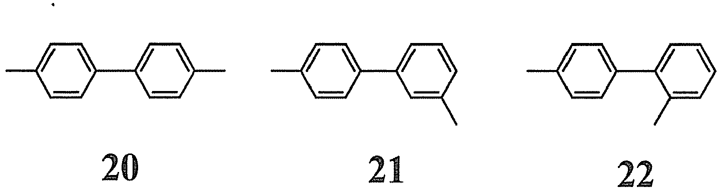

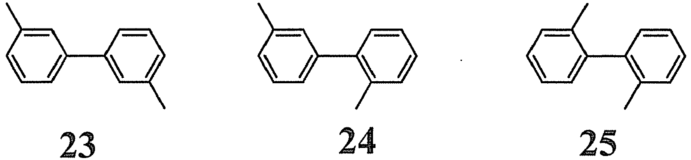

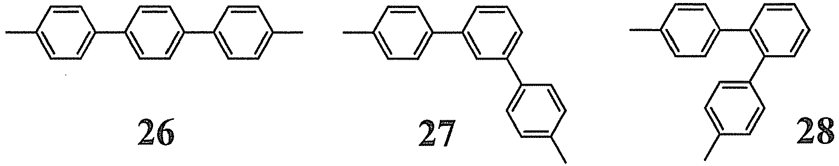

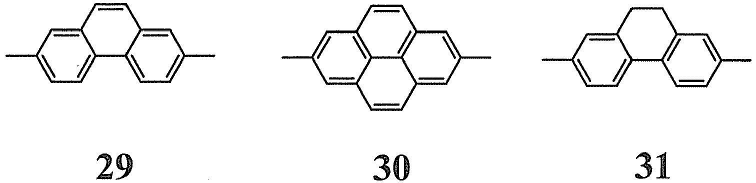

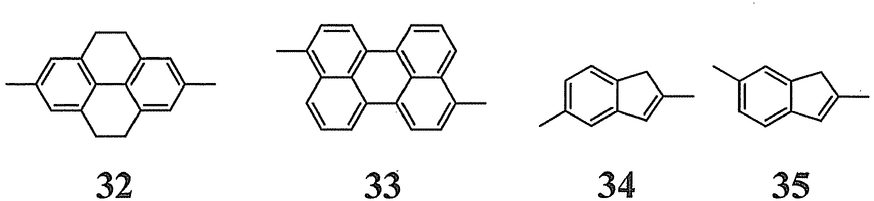

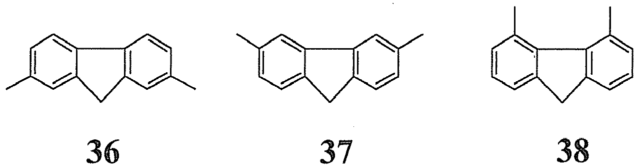

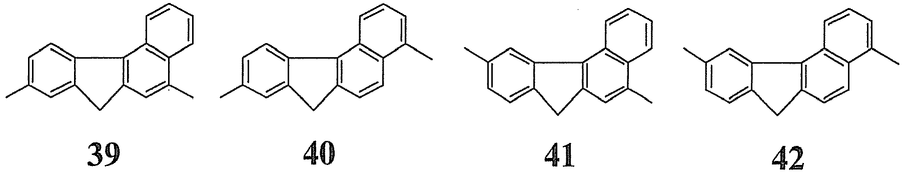

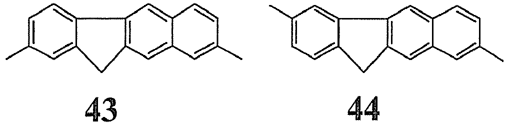

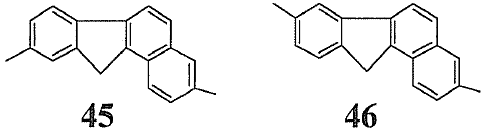

The above-described divalent heterocyclic group is an atomic group remaining after removal of two hydrogen atoms from a heterocyclic compound. The above-described divalent heterocyclic group may have a substituent. The above-described substituent is the same group as the substituent which can be carried on the above-described arylene group.

The above-described heterocyclic compound includes organic compounds having a cyclic structure in which elements constituting the ring include not only a carbon atom but also a hetero atom such as an oxygen atom, a sulfur atom, a nitrogen atom, a phosphorus atom, a boron atom, an arsenic atom and the like contained in the ring. As the above-described divalent heterocyclic group, divalent aromatic heterocyclic groups are preferable.

The carbon atom number of a portion excluding the substituent in the divalent heterocyclic group is usually 3 to 60, and the total carbon atom number including the substituent is usually 3 to 100.

Examples of the divalent heterocyclic group include the following groups. The following groups may have the same substituent as explained and shown as the substituent which can be carried on the above-described arylene group.

Divalent heterocyclic groups containing as a hetero atom a nitrogen atom: pyridine-diyl groups (the following formulae 101 to 104), diazaphenylene groups (the following formulae 105 to 108), triazine-diyl group (the following formula 109), quinoline-diyl groups (the following formulae 110 to 114), quinoxaline-diyl groups (the following formulae 115 to 119), acridinediyl groups (the following formulae 120 to 123), bipyridyl-diyl groups (the following formulae 124 to 126) and phenanthrolinediyl groups (the following formulae 127 to 128).

Groups containing as a hetero atom an oxygen atom, a sulfur atom, a nitrogen atom, a silicon atom and the like and having a fluorene structure (the following formulae 129 to 136).

5-membered heterocyclic groups containing as a hetero atom an oxygen atom, a sulfur atom, a nitrogen atom, a silicon atom and the like (the following formulae 137 to 140).

5-membered condensed heterocyclic groups containing as a hetero atom an oxygen atom, a sulfur atom, a nitrogen atom, a silicon atom and the like (the following formulae 141 to 158).

5-membered heterocyclic groups containing as a hetero atom an oxygen atom, a sulfur atom, a nitrogen atom, a silicon atom and the like and having a linkage at α-position of its hetero atom thereby forming a dimer or an oligomer (the following formulae 159 to 160).

5-membered heterocyclic groups containing as a hetero atom an oxygen atom, a sulfur atom, a nitrogen atom, a silicon atom and the like and having a linkage at α-position of its hetero to a phenyl group (the following formulae 161 to 166).

5-membered condensed heterocyclic groups containing as a hetero atom an oxygen atom, a sulfur atom, a nitrogen atom and the like and carrying thereon a substituent such as a phenyl group, a furyl group or a thienyl group (the following formulae 167 to 172).

6-membered heterocyclic groups containing as a hetero atom an oxygen atom, a nitrogen atom and the like (the following formulae 173 to 176).

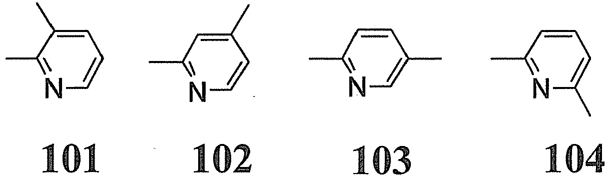

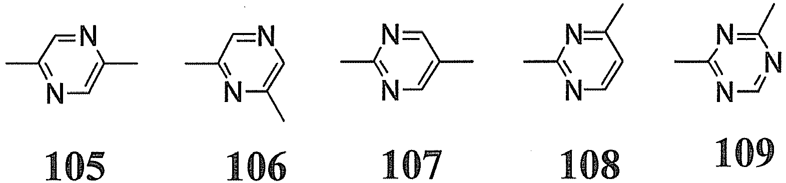

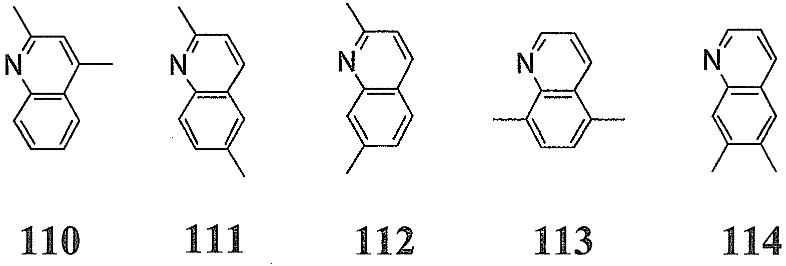

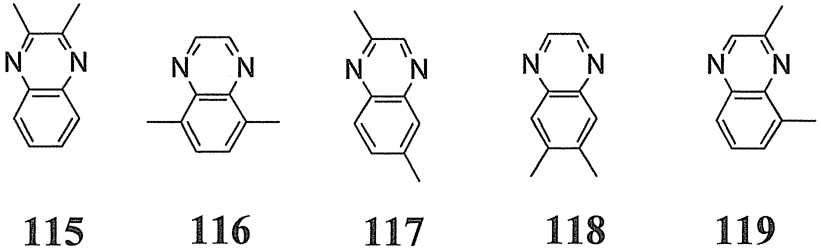

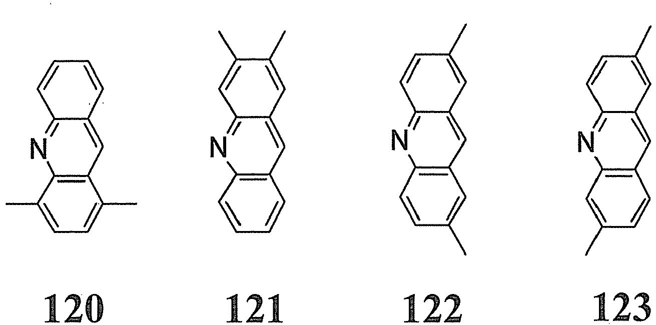

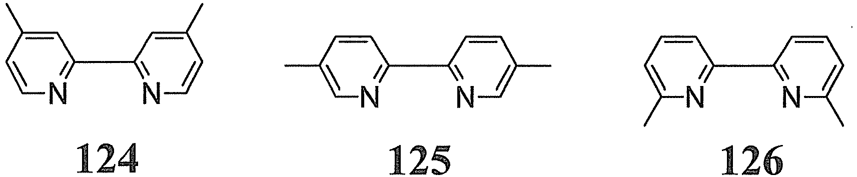

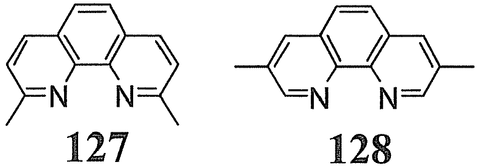

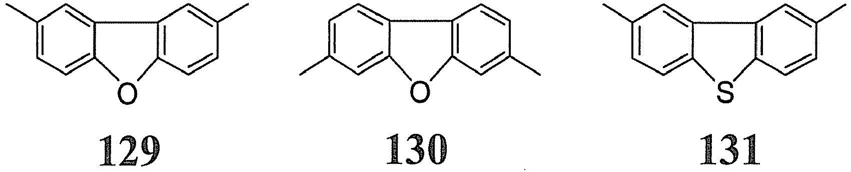

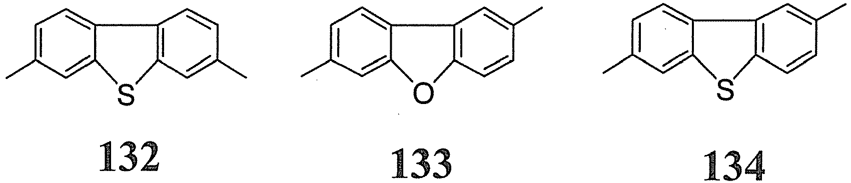

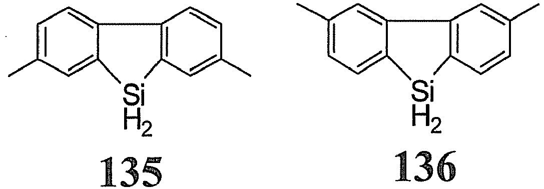

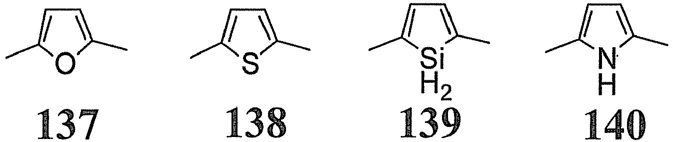

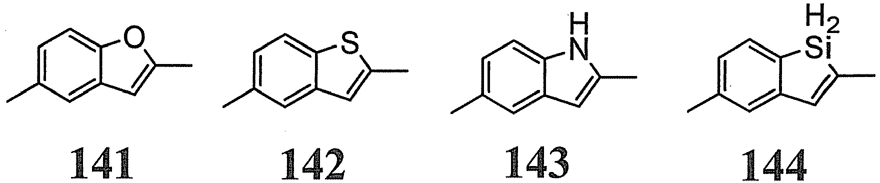

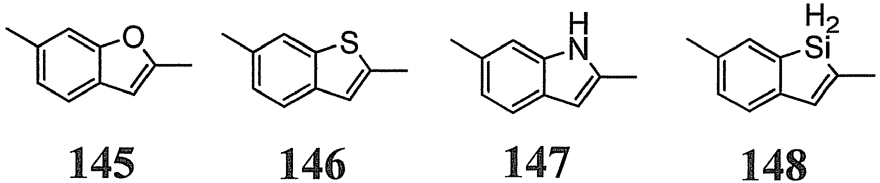

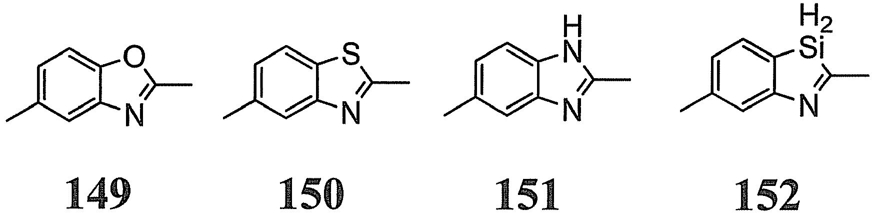

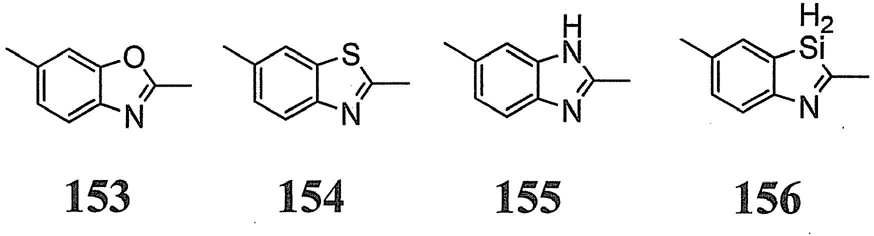

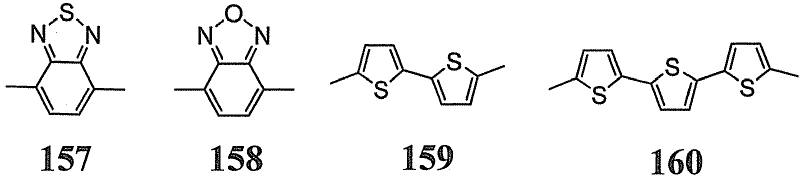

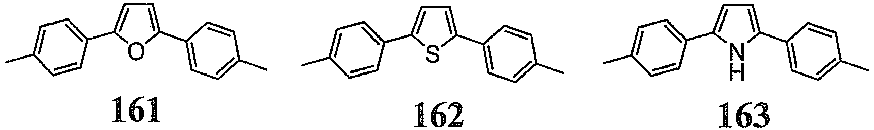

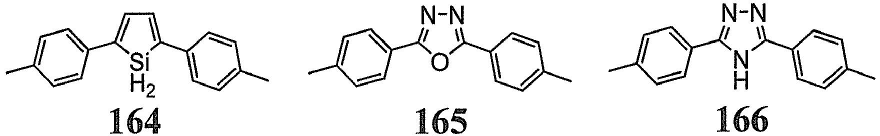

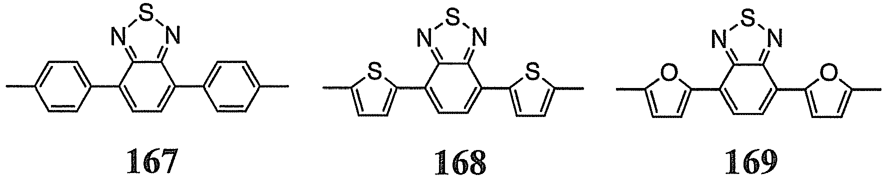

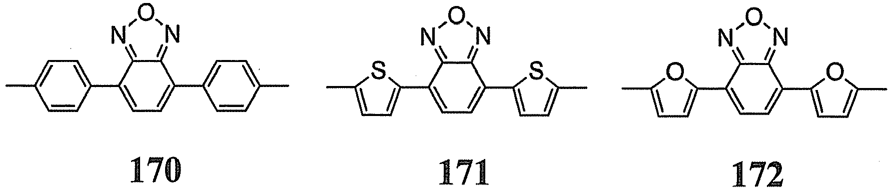

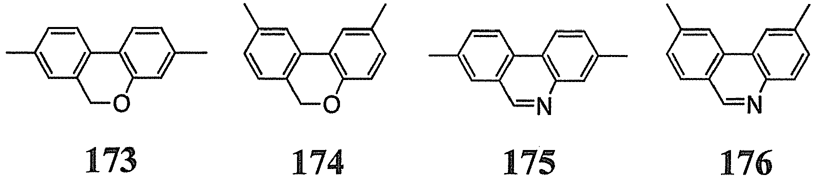

As the divalent heterocyclic group, divalent groups represented by the following formula (II): 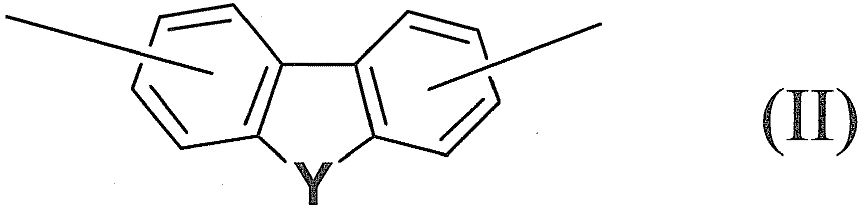 [in the formula (II), Y represents an oxygen atom, a sulfur atom, -O-C(R²) (R³)- or -Si(R⁴) (R⁵)-. R², R³, R⁴ and R⁵ represent each independently a hydrogen atom, an alkyl group, an alkoxy group, an aryl group or an arylalkyl group. The formula may have a substituent.] are preferable and divalent groups represented by the following formula (II)-1 or the following formula (II)-2 are more preferable from the standpoint of electron transportability. 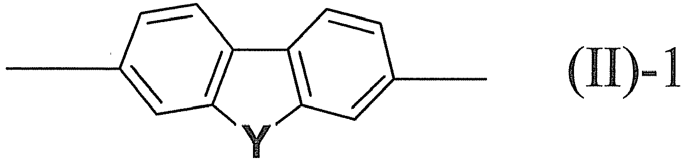 [in the formula (II)-1, Y has the same meaning as described above. The group represented by the formula may have a substituent.] 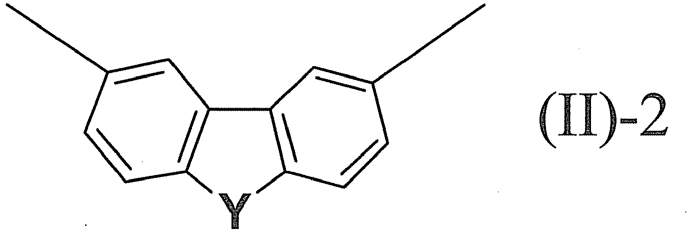 [in the formula (II)-2, Y has the same meaning as described above. The group represented by the formula may have a substituent.]

When the group represented by the above-described formula (II), (II)-1 or (II)-2 has a substituent, the substituent includes an alkyl group, an alkoxy group, an aryl group, an aryloxy group, an arylalkyl group and an arylalkoxy group. These groups have the same meaning as described above.

In the above-described formulae (II) and (II)-1, Y represents preferably an oxygen atom or a sulfur atom, more preferably an oxygen atom, from the standpoint of easiness of synthesis.

In the above-described formula (II)-2, Y represents preferably an oxygen atom or a sulfur atom, particularly preferably a sulfur atom, from the standpoint of easiness of synthesis.

The above-described divalent aromatic amine residue means an atomic group remaining after removal of two hydrogen atoms from an aromatic amine, and the carbon atom number thereof is usually 5 to 100, preferably 15 to 60, not including the carbon atom number of the substituent. The above-described divalent aromatic amine residue may have a substituent. The above-described substituent is the same group as the substituent which can be carried on the above-described arylene group.

The above-described divalent aromatic amine residue includes divalent groups represented by the following formulae 201 to 214. The following groups may have the same substituent as explained and shown as the substituent which can be carried on the above-described arylene group.

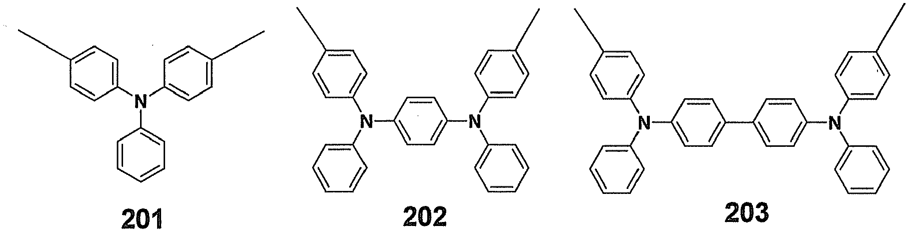

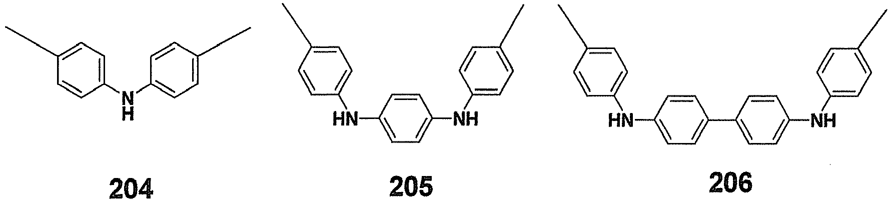

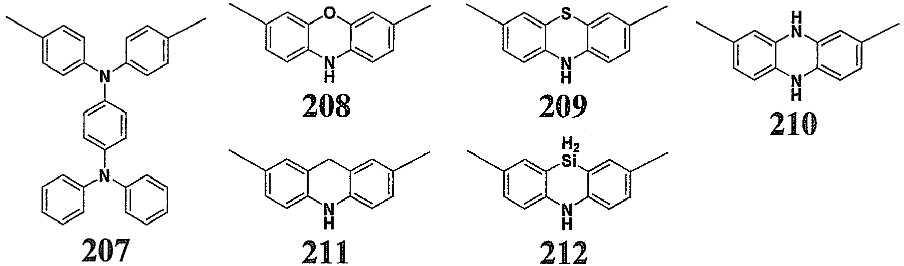

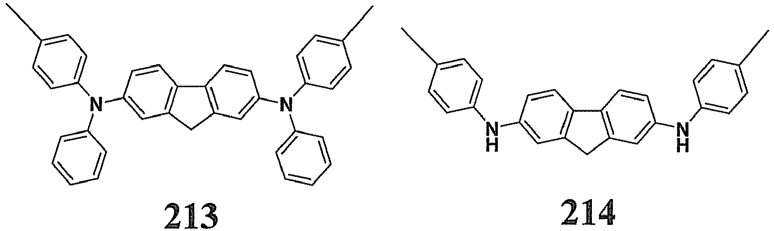

The above-described divalent aromatic amine residue is preferably a di-valent group represented by the following formula (III): 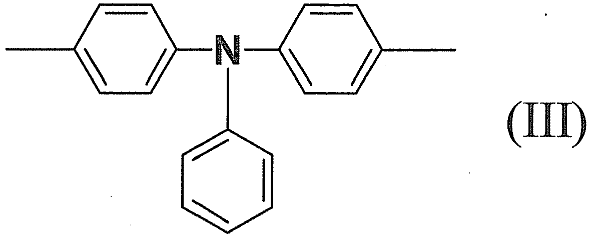 [The group represented by the formula (III) may have a substituent.]
or a di-valent group represented by the following formula (IV) : 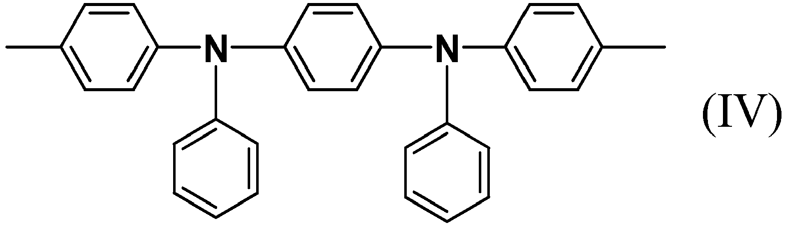 [The group represented by the formula (IV) may have a substituent.]
, from the standpoint of hole transportability.

When the group represented by the above-described formula (III) or (IV) has a substituent, the substituent includes an alkyl group, an alkoxy group, an aryl group, an aryloxy group, an arylalkyl group, an arylalkoxy group and a substituted amino group. These groups have the same meaning as described above.

In the above-described formula (I), the phenylene group represented by J¹ may have a substituent. The above-described phenylene group includes an o-phenylene, a m-phenylene, a p-phenylene and the like. The substituent which can be carried on the above-described phenylene group includes an alkyl group, an alkoxy group, a halogen atom and a cyano group. These groups have the same meaning as described above.

In the above-described formula (I), the alkylene group represented by J² may be linear or branched, and may have a substituent. The above-described alkylene group has a carbon atom number of usually 1 to 20, preferably 1 to 10, more preferably 1 to 6. The above-described alkylene group includes a methylene group, a 1,2-ethylene group, a 1,3-propylene group, a 1,3-butylene group, a 1,4-butylene group, a 1,3-pentylene group, a 1,4-pentylene group, a 1,5-pentylene group, a 1,4-hexylene group, a 1,6-hexylene group, a 1,7-heptylene group, a 1,6-octylene group, a 1,8-octylene group and the like.

In the above-described formula (I), X¹ represents preferably an oxygen atom, from the standpoint of easiness of synthesis of a crosslinkable polymer compound.

The arylene group having 1 to 4 groups represented by the above-described formula (I) and optionally having a substituent, the divalent heterocyclic group having 1 to 4 groups represented by the above-described formula (I) and optionally having a substituent and the divalent aromatic amine residue having 1 to 4 groups represented by the above-described formula (I) and optionally having a substituent include groups represented by the following formulae (Ar¹-1) to (Ar¹-8), (Ar¹-10), (Ar¹-12), (Ar¹-14), (Ar¹-16), (Ar¹-17), (Ar¹-20) and (Ar¹-21).

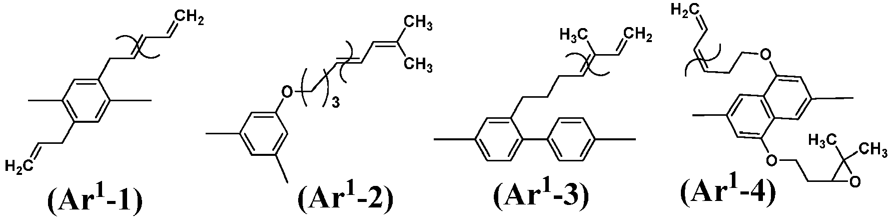

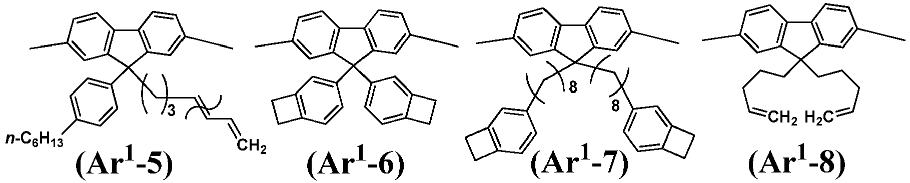

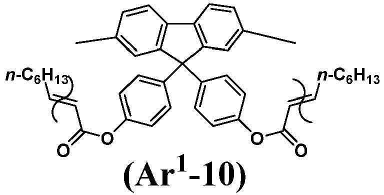

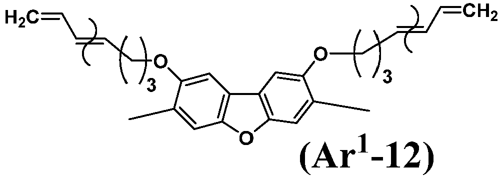

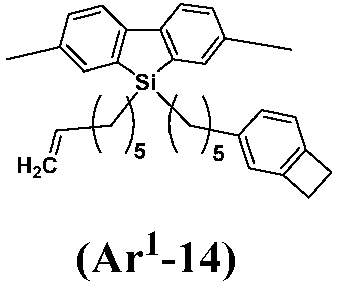

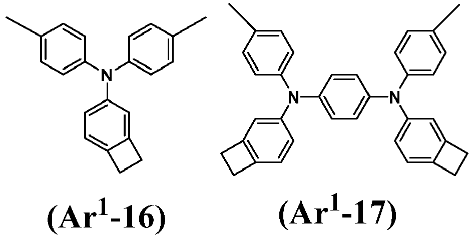

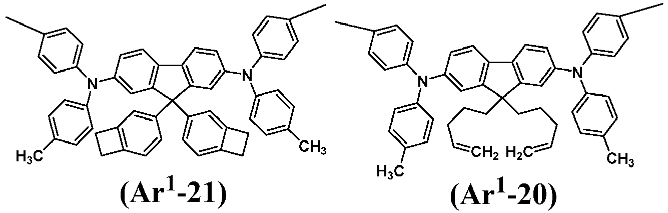

It is preferable that the above-described crosslinkable polymer compound has a repeating unit represented by the following formula (A): 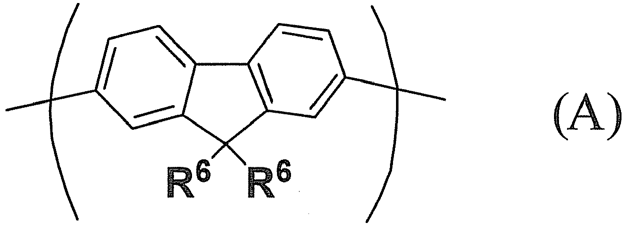 [in the formula (A), R⁶ represents an alkyl group, an aryl group, an arylalkyl group or an arylalkoxy group, and these groups may have a substituent. Two moieties of R⁶ may be the same or different, and may be mutually linked to form a ring.]
, in addition to the first repeating unit, from the standpoint of charge transportability. The repeating unit represented by the above-described formulae (A) is different from the first repeating unit.

In the above-described formula (A), the alkyl group, the aryl group, the arylalkyl group and the arylalkoxy group represented by R⁶ have the same meaning as described above, and from the standpoint of easiness of synthesis of raw material monomers, preferable are an alkyl group, an aryl group and an arylalkyl group, more preferable are an alkyl group and an aryl group, particularly preferable is an alkyl group.

When two R⁶s are mutually linked to form a ring, shown as the ring are C₄ to C₁₀ cycloalkane rings optionally having a substituent, C₄-C₁₀ cycloalkene rings optionally having a substituent and C₄ to C₁₀ heterocyclic rings optionally having a substituent.

Shown as the C₄ to C₁₀ cycloalkane ring are a cyclobutane ring, a cyclopentane ring, a cyclohexane ring, a cycloheptane ring, a cyclooctane ring, a cyclononane ring and a cyclodecane ring.

The C₄ to C₁₀ cycloalkene ring includes rings having two or more double bonds, and specific examples thereof include a cyclohexene ring, a cyclohexadiene ring, a cyclooctatriene ring and a cyclopentadiene ring.

Shown as the C₄ to C₁₀ heterocyclic ring are a tetrahydrofuran ring, a tetrahydrothiophene ring, a tetrahydroindole ring, a tetrahydroquinoline ring, a hexahydropyridine ring and a tetrahydroisoquinoline ring.

It is preferable that the above-described crosslinkable polymer compound has at least one repeating unit selected from the group consisting of a repeating unit represented by the following formula (B) and a repeating unit represented by the following formula (C), in addition to the first repeating unit, from the standpoint of hole transportability. The repeating units represented by the following formulae (B) and (C) are different from the first repeating unit. 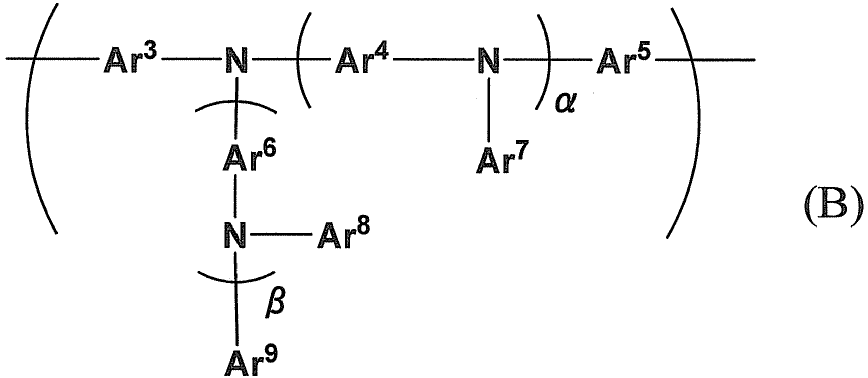 [in the formula (B), Ar³, Ar⁴, Ar⁵ and Ar⁶ represent each independently an arylene group optionally having a substituent or a divalent heterocyclic group optionally having a substituent, Ar⁷, Ar⁸ and Ar⁹ represent each independently an aryl group optionally having a substituent or a monovalent heterocyclic group optionally having a substituent, and α and β represent each independently 0 or 1.] 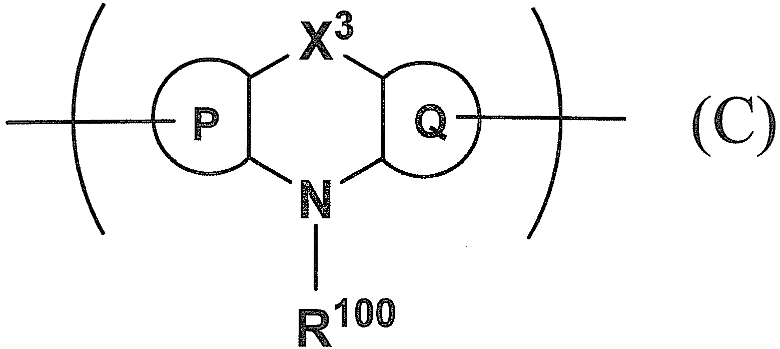 [in the formula (C), the ring P and the ring Q represent an aromatic hydrocarbon ring optionally having a substituent, X³ represents a single bond, an oxygen atom or a sulfur atom, R¹⁰⁰ represents an alkyl group, an aryl group, an aryloxy group, an arylalkyl group, an acyl group or a monovalent heterocyclic group, and these groups may have a substituent.]

In the above-described formula (B), the arylene group, the divalent heterocyclic group, the aryl group and the monovalent heterocyclic group have the same meaning as described above.

The substituent which can be carried on the groups represented by Ar³, Ar⁴, Ar⁵, Ar⁶, Ar⁷, Ar⁸ and Ar⁹ includes an alkyl group, an alkoxy group, an alkylthio group, an aryl group, an aryloxy group, an arylthio group, an arylalkyl group, an arylalkoxy group, an arylalkylthio group, an amino group, a substituted amino group, a silyl group, a substituted silyl group, a halogen atom, an acyl group, an acyloxy group, an imine residue, a carbamoyl group, an acid imide group, a monovalent heterocyclic group, a carboxyl group, a substituted carboxyl group, a cyano group and a nitro group. These groups and atoms have the same meaning as described above.

In the above-described formula (C), the aromatic hydrocarbon ring includes rings shown in the section of the above-described arylene group in which two connecting bonds are substituted by hydrogen atoms.

In the above-described formula (C), the substituent which can be carried on the ring P and the ring Q is the same as the group and the atom explained and shown as the substituent which can be carried on the group represented by the above-described Ar³, Ar⁴, Ar⁵, Ar⁶, Ar⁷, Ar⁸ and Ar⁹.

In the above-described formula (C), the alkyl group, the aryl group, the aryloxy group, the arylalkyl group, the acyl group and the monovalent heterocyclic group represented by R¹⁰⁰ have the same meaning as described above. The substituent which can be carried on the group represented by R¹⁰⁰ is the same as the group and the atom explained and shown as the substituent which can be carried on the group represented by the above-described Ar³, Ar⁴, Ar⁵ , Ar⁶ , Ar⁷, Ar⁸ and Ar⁹.

It is preferable that the repeating unit represented by the above-described formula (B) is a repeating unit represented by the following formula (B)-1, (B)-2, (B)-3 or (B)-4, from the standpoint of hole transportability. 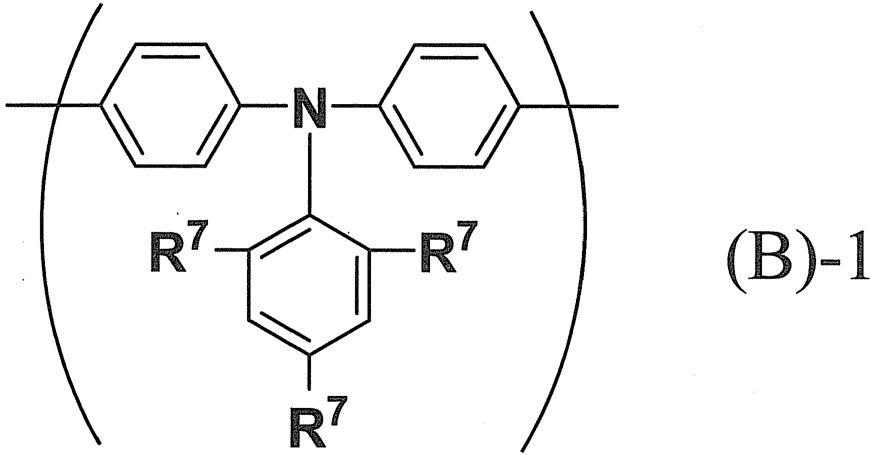 [in the formula (B)-1, R⁷ represents a hydrogen atom, an alkyl group or an alkoxy group, and these groups may have a substituent. Three moieties of R⁷ may be the same or different.] 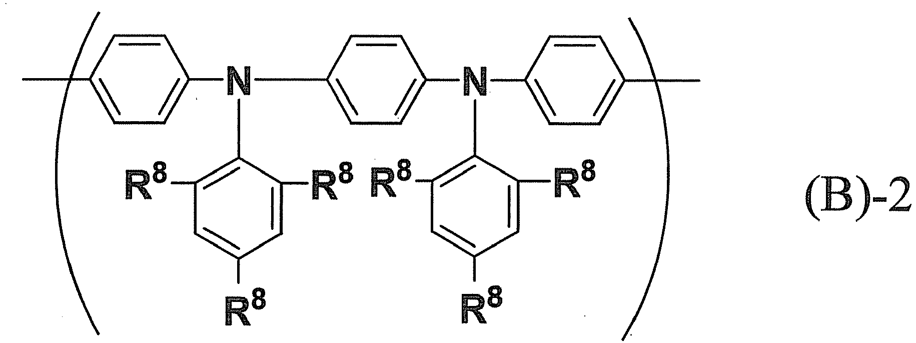 [in the formula (B)-2, R⁸ represents a hydrogen atom, an alkyl group or an alkoxy group, and these groups may have a substituent. Six moieties of R⁸ may be the same or different.] 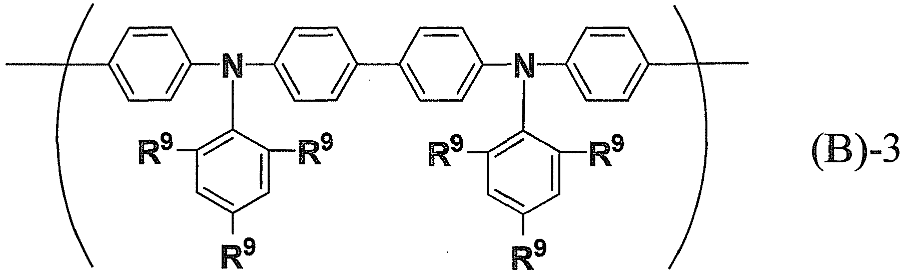 [in the formula (B)-3, R⁹ represents a hydrogen atom, an alkyl group or an alkoxy group, and these groups may have a substituent. Six moieties of R⁸ may be the same or different.] 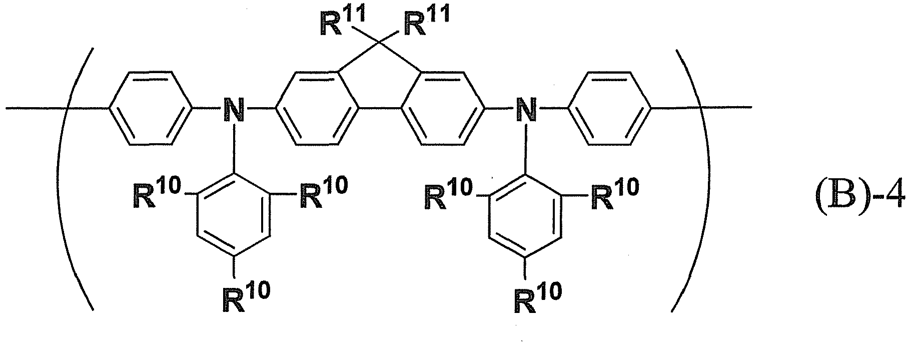 [in the formula (B)-4, R¹⁰ represents a hydrogen atom, an alkyl group or an alkoxy group, and these groups may have a substituent. R¹¹ represents an alkyl group, an aryl group, an arylalkyl group or an arylalkoxy group, and these groups may have a substituent. Six moieties of R¹⁰ may be the same or different, and two moieties of R¹¹ may be the same or different.]

The alkyl group and the alkoxy group represented by R⁷ in the above-described formula (B)-1, by R⁸ in the above-described formula (B)-2 and by R⁹ in the above-described formula (B)-3 have the same meaning as described above.

The alkyl group and the alkoxy group represented by R¹⁰ and the alkyl group, the aryl group, the arylalkyl group and the arylalkoxy group represented by R¹¹ in the above-described formula (B)-4 have the same meaning as described above.

It is preferable that the repeating unit represented by the above-described formulae (C) is a repeating unit represented by the following formula (C)-1, from the standpoint of hole transportability. 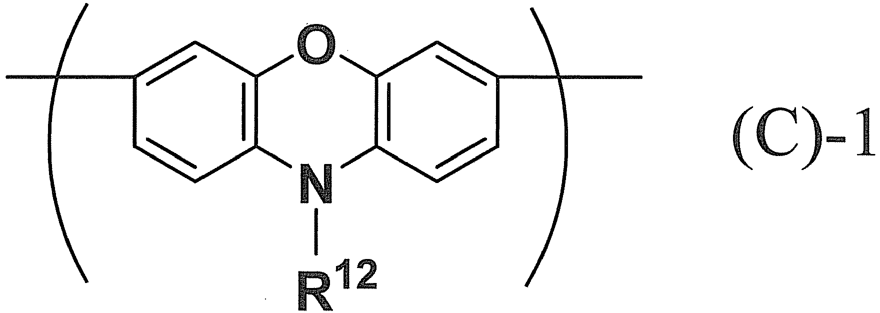 [in the formula (C)-1, R¹² represents an alkyl group, an aryl group or an arylalkyl group, and these groups may have a substituent.]

In the above-described formula (C)-1, the alkyl group, the aryl group and the arylalkyl group represented by R¹² have the same meaning as described above.

It is preferable that the above-described crosslinkable polymer compound has at least one repeating unit selected from the group consisting of repeating units represented by the following formulae (D), (E), (F) and (G), in addition to the first repeating unit, from the standpoint of charge transportability. The repeating units represented by the following formulae (D), (E), (F) and (G) are different from the first repeating unit. 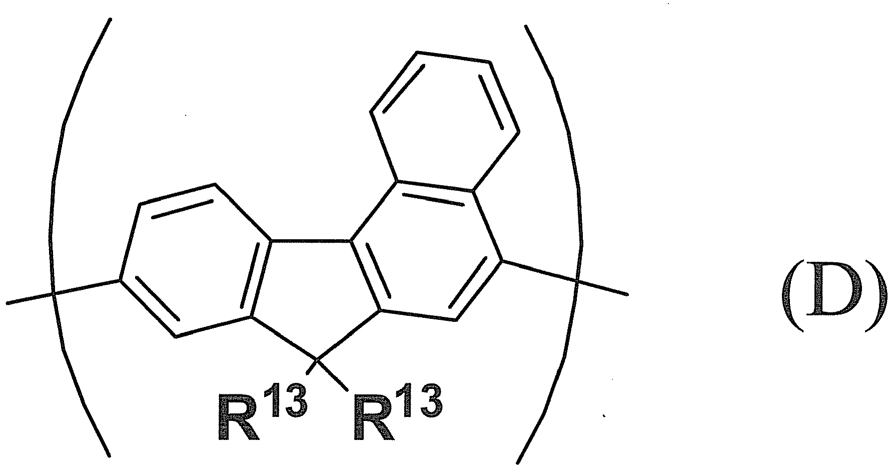 [in the formula (D), R¹³ represents an alkyl group, an aryl group, an arylalkyl group or an arylalkoxy group, and these groups may have a substituent. Two moieties of R¹³ may be the same or different, and may be mutually linked to form a ring.] 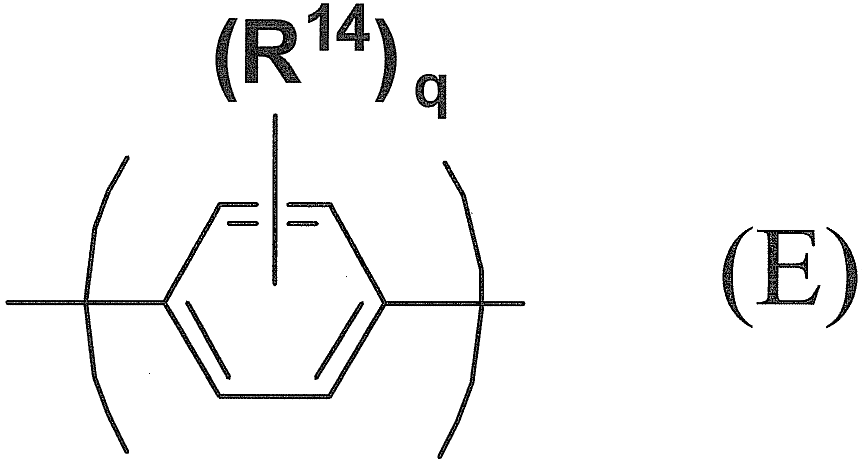 [in the formula (E), R¹⁴ represents an alkyl group, an alkoxy group, an aryl group, an arylalkyl group or an arylalkoxy group, and these groups may have a substituent. q is an integer of 0 to 4. When a plurality of R¹⁴s are present, these may be the same or different.] 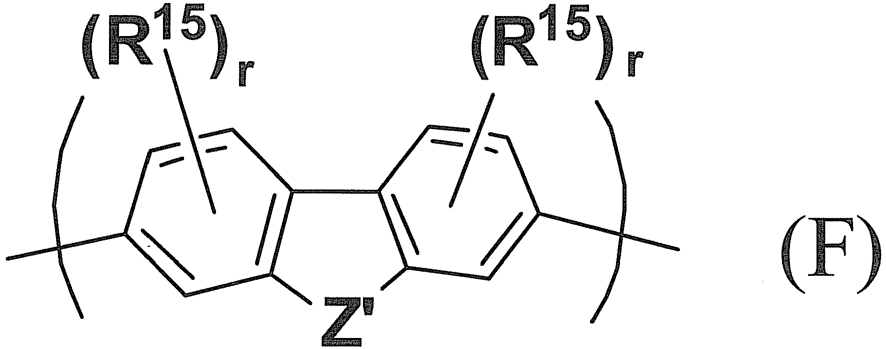 [in the formula (F), R¹⁵ represents an alkyl group, an alkoxy group, an aryl group, an arylalkyl group or an arylalkoxy group, and these groups may have a substituent. Z' represents an oxygen atom or a sulfur atom. r is an integer of 0 to 3. When a plurality of R¹⁵s are present, these may be the same or different.] 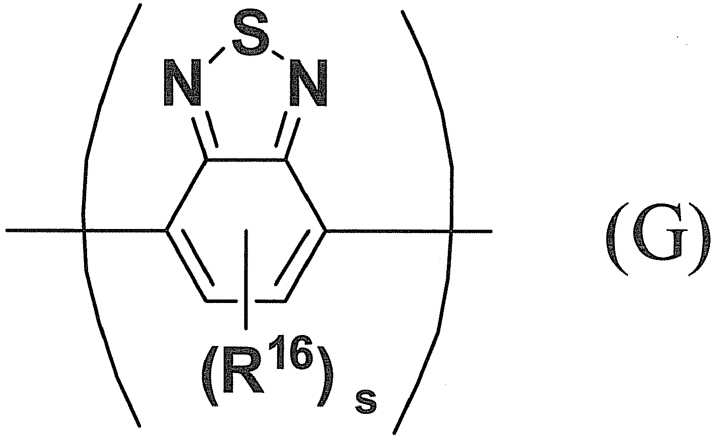 [in the formula (G), R¹⁶ represents an alkyl group, an alkoxy group, an aryl group, an arylalkyl group or an arylalkoxy group, and these groups may have a substituent. s is an integer of 0 to 2. When a plurality of R¹⁶s are present, these may be the same or different.].

In the above-described formula (D), the alkyl group, the aryl group, the arylalkyl group and the arylalkoxy group represented by R¹³ have the same meaning as described above.

When the groups represented by R¹³ are mutually linked to form a ring in the above-described formula (D), shown as the ring are C₄ to C₁₀ cycloalkane rings optionally having a substituent, C₄-C₁₀ cycloalkene rings optionally having a substituent and C₄ to C₁₀ heterocyclic rings optionally having a substituent.

The C₄ to C₁₀ cycloalkane ring, the C₄-C₁₀ cycloalkene ring optionally having a substituent and the C₄ to C₁₀ heterocyclic ring optionally having a substituent which can be formed by mutual linkage of the groups represented by R¹³ are the same as those explained and shown as the group which can be formed by mutual linkage of the groups represented by R⁶.

The alkyl group, the alkoxy group, the aryl group, the arylalkyl group and the arylalkoxy group represented by R¹⁴ in the above-described formulae (E), by R¹⁵ in the above-described formulae (F) and by R¹⁶ in the above-described formulae (G) have the same meaning as described above.

It is preferable that the above-described crosslinkable polymer compound has at least one repeating unit selected from the group consisting of repeating units represented by the following formula (J), (K) or (N), in addition to the first repeating unit,
from the standpoint of the light emission efficiency of a light emitting device, when the composition of the present invention is used as a light emitting material. The repeating units represented by the following formulae (J), (K) and (N) are different from the first repeating unit. 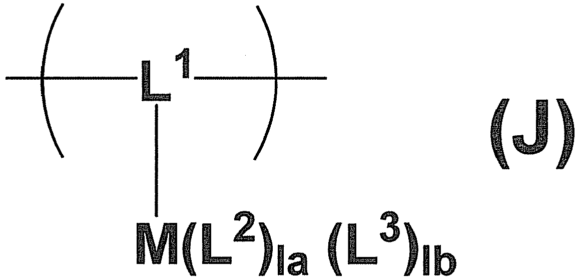 [in the formula (J), L¹ represents a residue obtained by removing two hydrogen atoms from a ligand represented by any of the following formulae (L-1) to (L-5), L² and L³ represent a ligand represented by any of (L-1) to (L-5) or a halogen atom, M represents a metal atom having an atomic number of 50 or more and capable of causing intersystem crossing between the singlet state and the triplet state in the present compound by the spin-orbital interaction, and la and lb are each independently 0 or 1.] 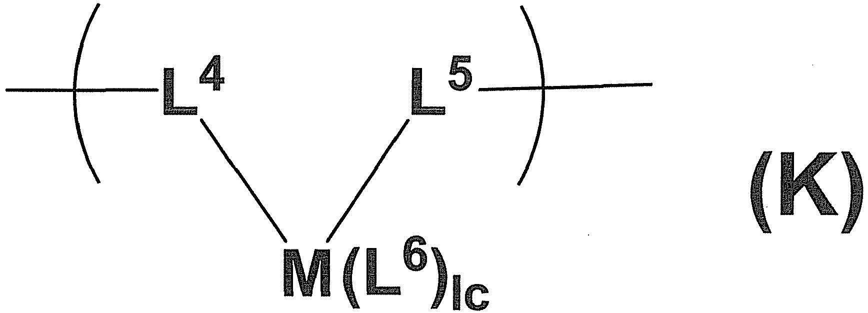 [in the above-described formula (K), L⁴ and L⁵ represent each independently a structure obtained by removing one hydrogen atom from a ligand represented by any of the following formulae (L-1) to (L-5), L⁶ represents a ligand represented by any of the following formulae (L-1) to (L-5) or a halogen atom, M has the same meaning as described above, and lc is 0 or 1.] 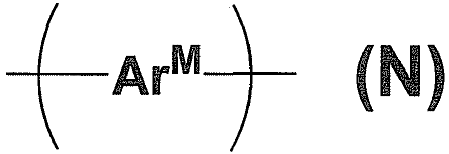 [in the formula (N), Ar^{M} represents an arylene group, a divalent heterocyclic group or a divalent aromatic amine residue and has one or two groups represented by the following formula (P): 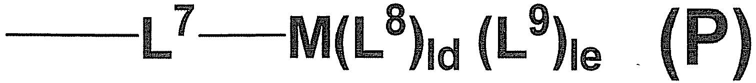 [in the formula (P), L⁷ represents a residue obtained by removing one hydrogen atom from a ligand represented by any of the following formulae (L-1) to (L-5), L⁸ and L⁹ represent each independently a ligand represented by any of the following formulae (L-1) to (L-5) or a halogen atom, M has the same meaning as described above, and ld and 1e are each independently 0 or 1.]] 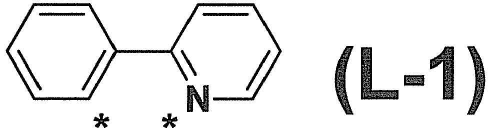 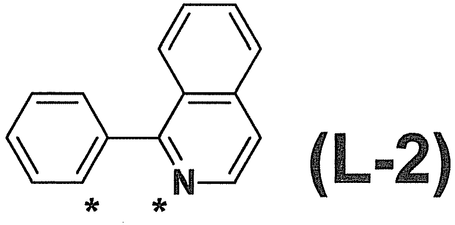 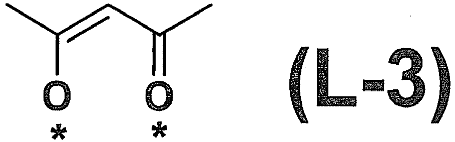 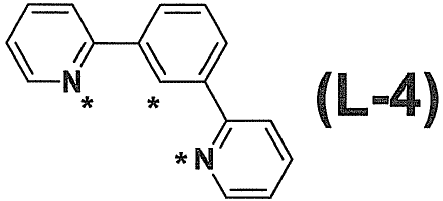 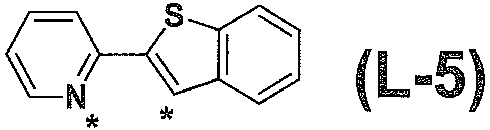 [in the formulae (L-1) to (L-5), * represents an atom linked to a metal complex.]

The ligand represented by the above-described formulae (L-1) to (L-5) may have the same substituent as explained and shown as the substituent which can be carried on the group represented by the above-described Ar³, Ar⁴, Ar⁵, Ar⁶ , Ar⁷, Ar⁸ and Ar⁹.

As the metal atom represented by M in the above-described formulae (J), (K) and (P), shown are a rhenium atom, an iridium atom, an osmium atom, a platinum atom, a gold atom and a europium atom, and preferable are an iridium atom, a platinum atom and a gold atom and more preferable are an iridium atom and a platinum atom, from the standpoint of the light emission efficiency of a light emitting device.

In the above-described formula (J), relating to la and lb, la+lb is preferably 1 or 2 and more preferably 2, since the light emission efficiency of a light emitting device is enhanced.

In the above-described formula (K), lc is preferably 1, since the light emission efficiency of a light emitting device is enhanced.

In the above-described formula (P), ld+le is preferably 1 or 2 and more preferably 2, since the light emission efficiency of a light emitting device is enhanced.

In the above-described formula (N), the arylene group, the divalent heterocyclic group and the divalent aromatic amine residue represented by Ar^{M} are the same groups as described above.

It is preferable that the above-described crosslinkable polymer compound has the first repeating unit, a repeating unit represented by the above-described formula (A), and at least one repeating unit selected from the group consisting of a repeating unit represented by the above-described formula (B) and a repeating unit represented by the above-described formula (C), from the standpoint of the light emission efficiency of a light emitting device.

In the above-described crosslinkable polymer compound, the upper limit of the proportion of the first repeating unit is usually 100 mol%, preferably 50 mol%, more preferably 30 mol% and particularly preferably 15 mol%, from the standpoint of the chemical stability of the crosslinkable polymer compound, and the lower limit of the proportion of the first repeating unit is usually 1 mol%, preferably 2 mol% and more preferably 5 mol%, from the standpoint of the curability of the crosslinkable polymer compound, with respect to all repeating units in the crosslinkable polymer compound.

If the above-described crosslinkable polymer compound is a polymer compound having the first repeating unit, a repeating unit represented by the above-described formula (A), and at least one repeating unit selected from the group consisting of a repeating unit represented by the above-described formula (B) and a repeating unit represented by the above-described formula (C), the amount of the repeating unit represented by the above-described formulae (A) is usually 10 to 90 mol%, preferably 30 to 90 mol% and the sum of the amounts of the repeating units represented by the above-described formulae (B) and (C) is usually 1 to 50 mol%, preferably 5 to 30 mol%, with respect to all repeating units.

The above-described crosslinkable polymer compound has a polystyrene-equivalent number-average molecular weight of preferably 1×10⁴ to 1×10⁷, from the standpoint of the luminance life of a light emitting device in the case of use for fabrication of the light emitting device.

The above-described crosslinkable polymer compound has a polystyrene-equivalent weight-average molecular weight of preferably 1×10⁴ to 1×10⁷, from the standpoint of curability.

The above-described crosslinkable polymer compound may be any of a homopolymer, an alternative copolymer, a random copolymer, a block copolymer and a graft copolymer, or may also be a polymer compound having an intermediate structure thereof, for example, a random copolymer having a block property, and from the standpoint of fluorescent or phosphorescent quantum yield, a random copolymer having a block property, a block copolymer and a graft copolymer are more preferable than the complete random copolymer. The above-described crosslinkable polymer compound includes also polymer compounds having a branch in the main chain and thus having three or more end parts and dendrimers.

It is preferable that the end group of the above-described crosslinkable polymer compound is protected by a stable group since if the end group is a polymerization active group, the light emitting property and the life of a light emitting device lower in some cases when used for fabrication of the light emitting device. When the above-described crosslinkable polymer compound has a conjugated structure, a group having a conjugated bond consecutive to the conjugated structure of the main chain is preferable as the above-described end group, and such a group includes groups having a linkage to an aryl group or a monovalent heterocyclic group via a carbon-carbon bond, and substituents described in JP-A No. 9-45478, chemical formula 10.

The above-described crosslinkable polymer compound includes the following polymer compounds. In the formulae, v, w, x, y and z represent the composition ratio (molar ratio).

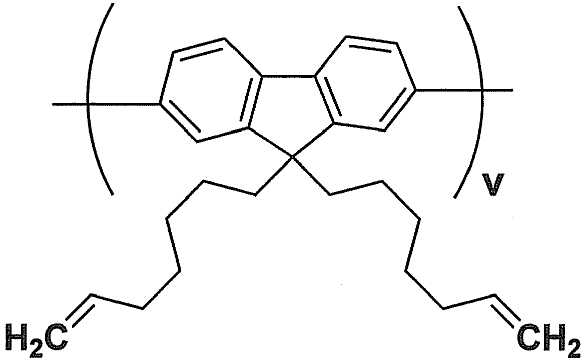

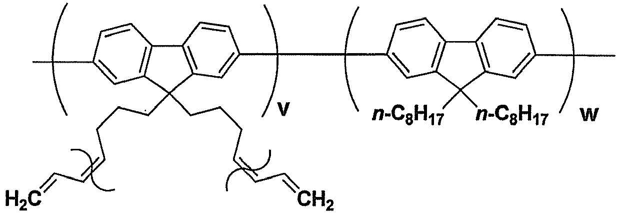

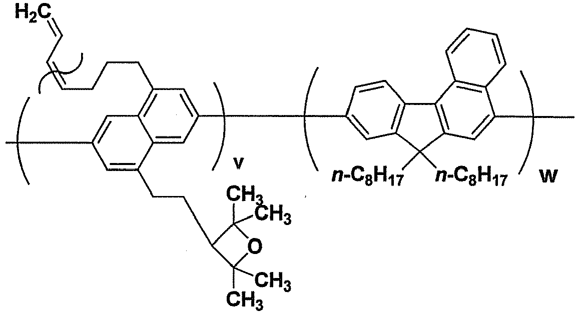

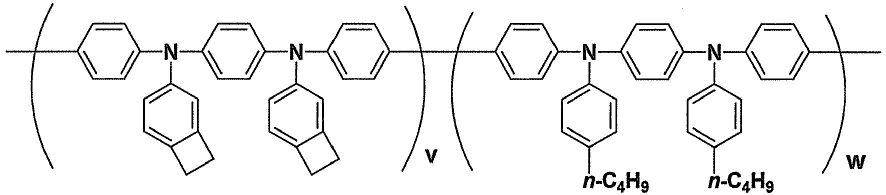

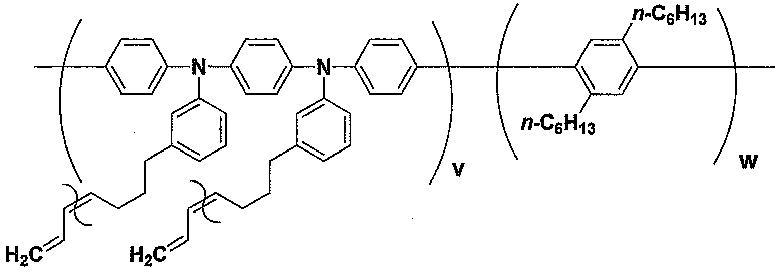

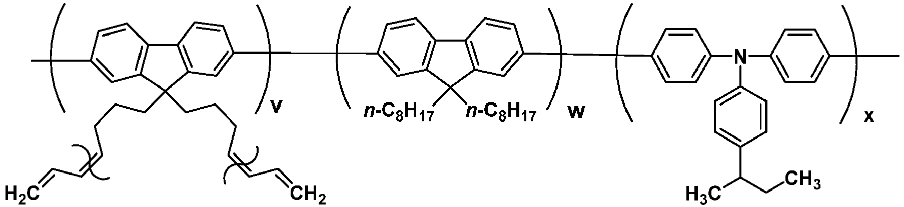

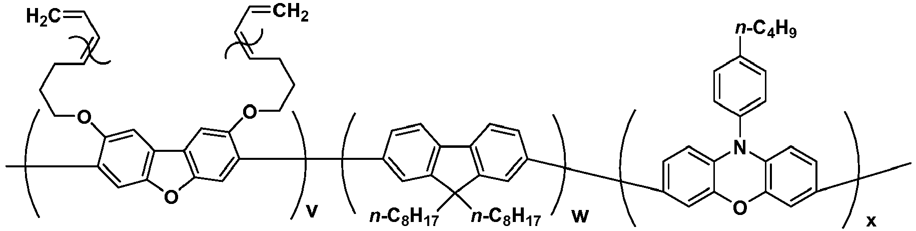

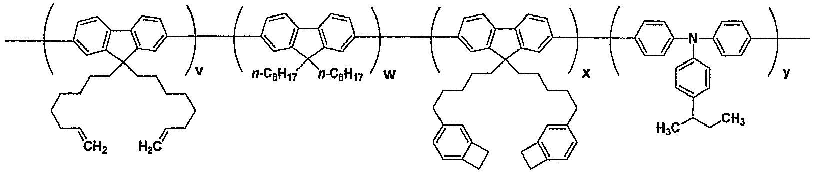

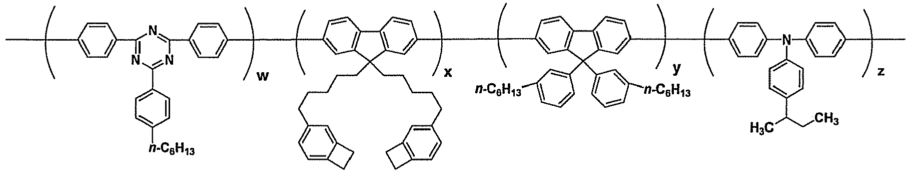

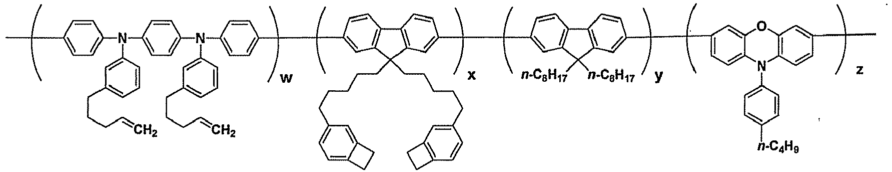

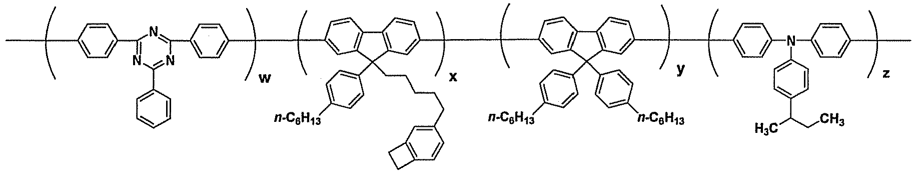

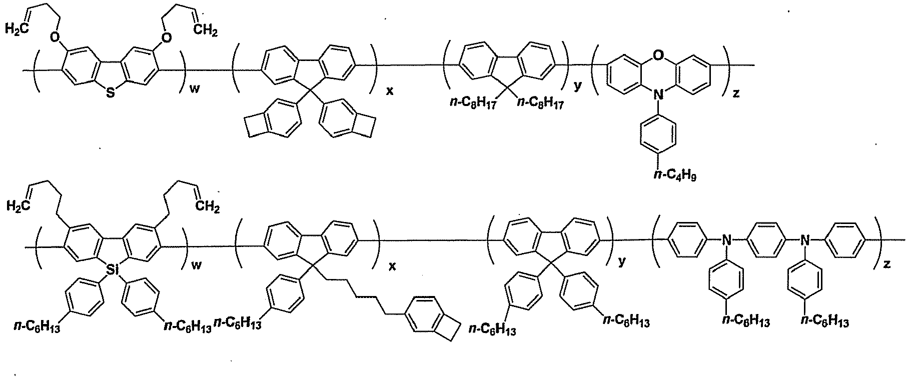

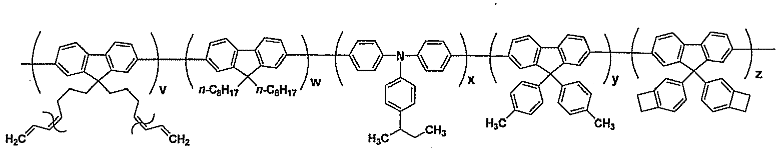

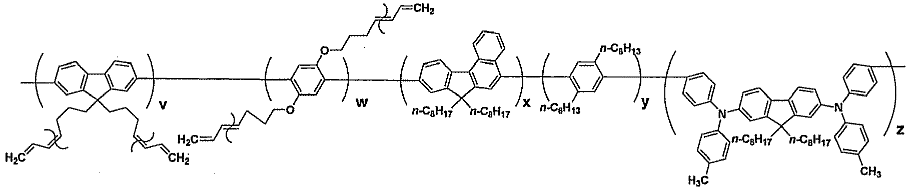

The copolymers among the polymer compounds shown above may be any of random copolymers, alternative copolymers and block copolymers.

### -Crosslinkable low molecular weight compound-

The above-described crosslinkable low molecular weight compound is a compound having a single molecular weight which is 1×10² or more and less than 1×10⁴.

As the above-described crosslinkable low molecular weight compound, compounds represented by the following formula (R) are preferable. 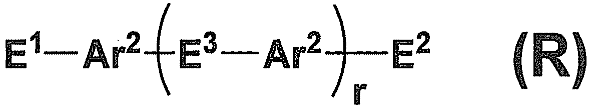 [in the formula (R), E¹ and E² represent each independently a hydrogen atom, an alkyl group, an alkoxy group, an alkylthio group, an aryl group, an aryloxy group, an arylthio group, an arylalkyl group, an arylalkoxy group, an arylalkylthio group, an amino group, a substituted amino group, a silyl group, a substituted silyl group, a halogen atom, an acyl group, an acyloxy group, an imine residue, a carbamoyl group, an acid imide group, a carboxyl group, a substituted carboxyl group, a cyano group or a nitro group, and these groups may have a substituent. E³ represents a direct bond, an arylene group or a divalent aromatic amine residue, and these groups may have a substituent. r is an integer of 0 to 2. Ar² represents an arylene group, a divalent aromatic amine residue, a divalent heterocyclic group having an oxygen atom or a sulfur atom, or a divalent group having a metal complex structure, and these groups may have a substituent, Ar² having 2 to 4 groups represented by the following formula (V): 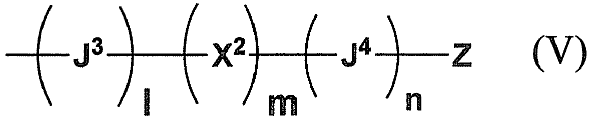 (in the formula (V), Z has the same meaning as described above, J³ represents a phenylene group optionally having a substituent, J⁴ represents an alkylene group optionally having a substituent, and X² represents an oxygen atom or a sulfur atom. 1 and m are each independently 0 or 1, and n is an integer of 0 to 3.)
When a plurality of Ar²s and a plurality of E³s are present, two or more moieties of each of them may be the same or different.]

The group and the atom represented by E¹ and E² in the above-described formula (R) represent the same meaning as described above, and from the standpoint of easiness of synthesis of a crosslinkable low molecular weight compound, E¹ and E² represent preferably a hydrogen atom, an alkyl group, an aryl group, an arylalkyl group, a substituted amino group, a substituted silyl group or a halogen atom, more preferably a hydrogen atom, an aryl group, an arylalkyl group, a substituted amino group or a halogen atom, further preferably an aryl group, a substituted amino group or a halogen atom.

The arylene group represented by Ar² and E³ in the above-described formula (R) is the same group as described above, and from the standpoint of the light emission efficiency of a light emitting device, preferable are a naphthalenediyl group, an anthracene-diyl group, a condensed ring group, a fluorene-diyl group and a benzofluorene-diyl group, more preferable are an anthracene-diyl group, a condensed ring group and a fluorene-diyl group, further preferable are an anthracene-diyl group and a fluorene-diyl group.

The divalent heterocyclic group having an oxygen atom or a sulfur atom represented by Ar² includes groups represented by the above-described formulae 129 to 134, 137, 138, 141, 142, 145, 146, 149, 150, 153, 154, 157 to 162, 165, 167 to 174.

The divalent heterocyclic group having an oxygen atom or a sulfur atom represented by Ar² includes preferably groups represented by the above-described formula (II) in which Y is an oxygen atom or a sulfur atom and includes preferably groups represented by the above-described formulae (II)-1 and (II)-2 in which Y¹ is an oxygen atom or a sulfur atom, from the standpoint of electron transportability.

The examples and the preferable range of the divalent aromatic amine residue represented by Ar² and E³ are the same as described above.

The divalent group having a metal complex structure represented by Ar² is a divalent group remaining after removal of two hydrogen atoms from an organic ligand of a metal complex having the organic ligand, or a divalent group remaining after removal of one hydrogen atom from one organic ligand and removal of one hydrogen atom from another organic ligand. This organic ligand has a carbon atom number of usually 4 to 60, and examples thereof include 8-quinolinol and derivatives thereof, benzoquinolinol and derivatives thereof, 2-phenyl-pyridine and derivatives thereof, 2-phenyl-benzothiazole and derivatives thereof, 2-phenyl-benzoxazole and derivatives thereof and porphyrin and derivatives thereof, and from the standpoint of the light emission efficiency of a light emitting device fabricated using a crosslinkable low molecular weight compound, preferable are 8-quinolinol and derivatives thereof, benzoquinolinol and derivatives thereof and 2-phenyl-pyridine and derivatives thereof, further preferable are benzoquinolinol and derivatives thereof and 2-phenyl-pyridine and derivatives thereof.

Examples of the central metal of the above-described metal complex include aluminum, zinc, beryllium, iridium, platinum, gold, europium and terbium, and from the standpoint of the light emission efficiency of a light emitting device, preferable are aluminum, zinc, iridium and platinum, more preferable are iridium and platinum, further preferable is iridium.

The above-described metal complex having an organic ligand includes metal complexes, triplet light emitting complexes and the like known as low molecular weight fluorescent and phosphorescent materials, and triplet light emitting complexes are preferable from the standpoint of the light emission efficiency of a light emitting device.

Shown as the above-described metal complex having an organic ligand are metal complexes represented by the following formulae 301 to 308. These metal complexes having an organic ligand may have a substituent. The substituent which this metal complex may have includes an alkyl group, an alkoxy group, an alkylthio group, an aryl group, an aryloxy group, an arylthio group, an arylalkyl group, an arylalkoxy group, an arylalkylthio group, an amino group, a substituted amino group, a silyl group, a substituted silyl group, a halogen atom, an acyl group, an acyloxy group, an imine residue, a carbamoyl group, an acid imide group, a monovalent heterocyclic group, a carboxyl group, a substituted carboxyl group, a cyano group and a nitro group, and from the standpoint of the property of a light emitting device, an alkyl group, an alkoxy group, an aryl group, an aryloxy group, an arylalkyl group, an arylalkoxy group, a halogen atom and a cyano group are preferable.

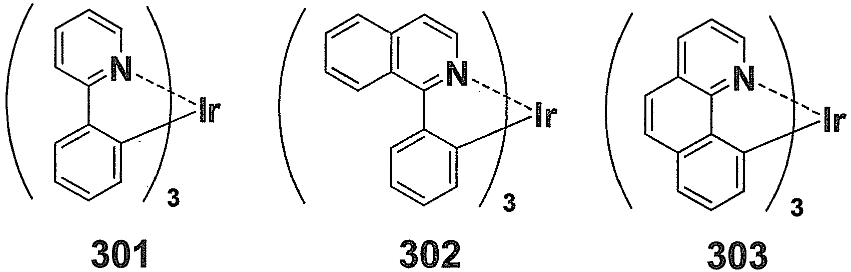

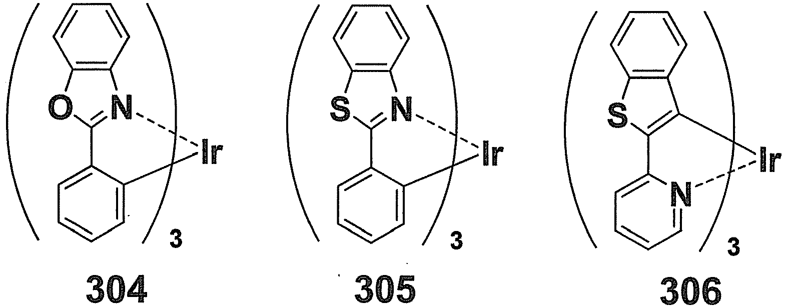

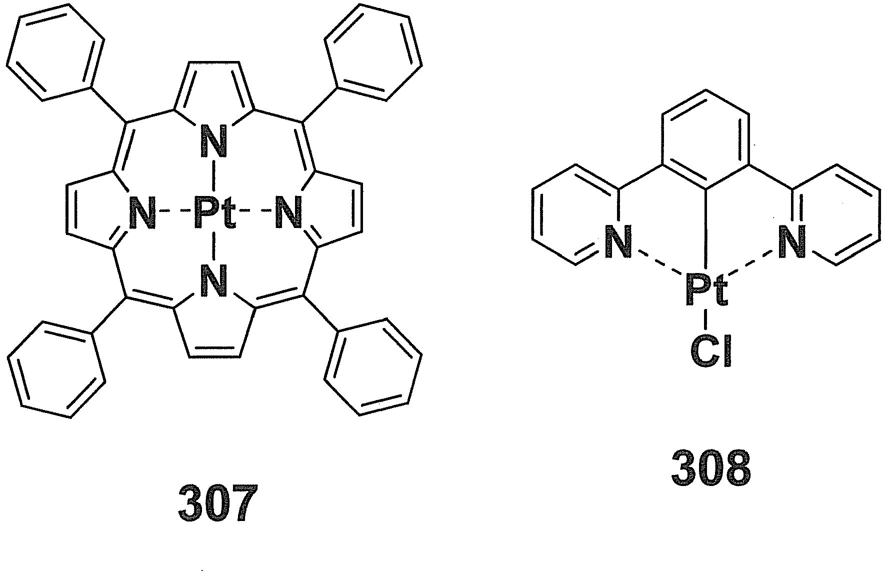

The phenylene group represented by J³ in the above-described formula (V) includes o-phenylene, m-phenylene, p-phenylene and the like. The substituent which can be carried on the above-described phenylene groups includes an alkyl group, an alkoxy group, a halogen atom and a cyano group. These groups have the same meaning as described above.

The alkylene group represented by J⁴ in the above-described formula (V) may be linear or branched. The above-described alkylene group is the same as the alkylene group represented by the above-described J².

X² in the above-described formula (V) is preferably an oxygen atom, from the standpoint of easiness of synthesis of a crosslinkable low molecular weight compound.

The above-described Ar² includes groups represented by the following formulae (Ar²-1), (Ar²-2), (Ar²-4), (Ar²-6) to (Ar²-11), (Ar²-13), (Ar²-14), (Ar²-16), (Ar²-17) and (Ar²-19) to (Ar²-22).

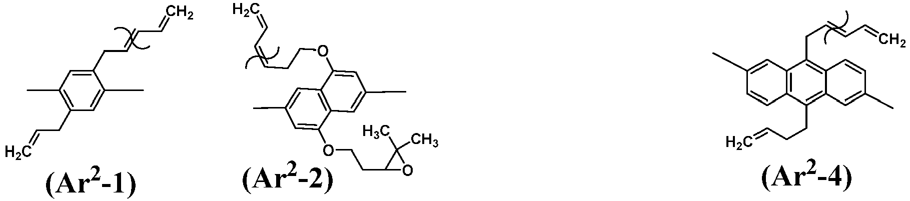

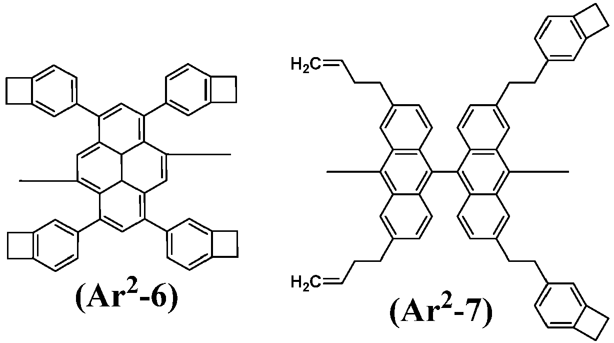

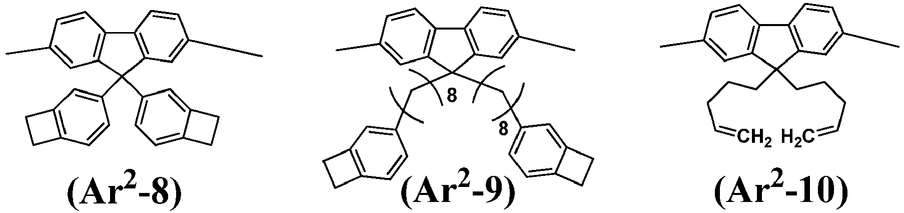

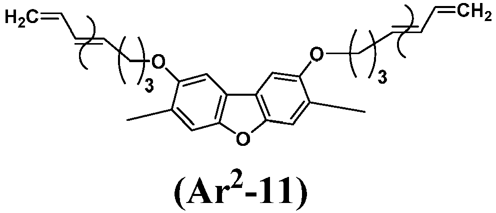

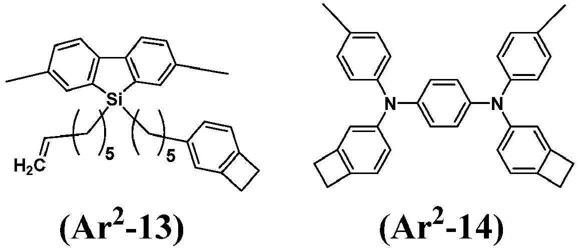

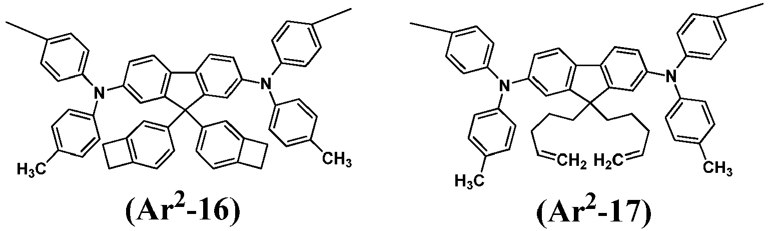

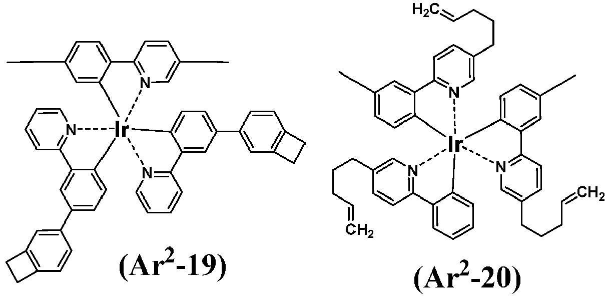

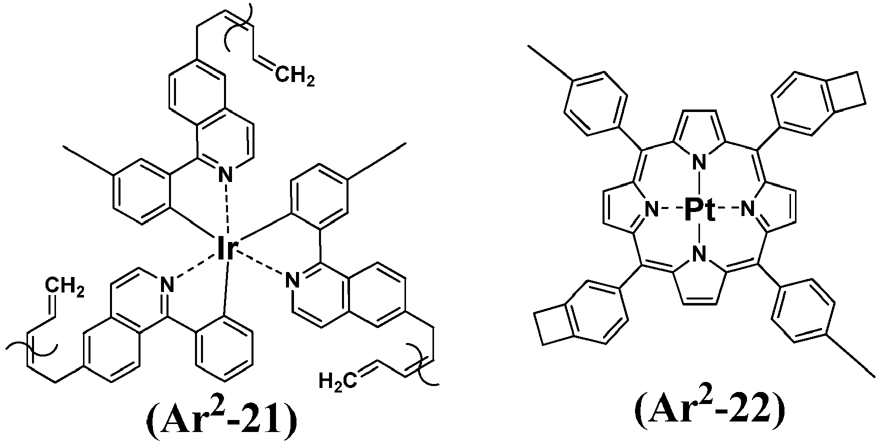

The above-described crosslinkable low molecular weight compound is preferably an aromatic hydrocarbon, from the standpoint of highly efficient fluorescence, and preferably a heterocyclic compound, from the standpoint of electron transportability. In the above-described crosslinkable low molecular weight compound, the aromatic hydrocarbon and the heterocyclic compound may have a substituent.

In the above-described crosslinkable low molecular weight compound, Ar² is preferably an arylene group, from the standpoint of highly efficient fluorescence, preferably a divalent heterocyclic group, from the standpoint of electron transportability, preferably a divalent aromatic amine residue, from the standpoint of hole transportability, preferably a divalent group having a metal complex structure, from the standpoint of highly efficient phosphorescence.

The above-described crosslinkable low molecular weight compound includes compounds represented by the following formulae.

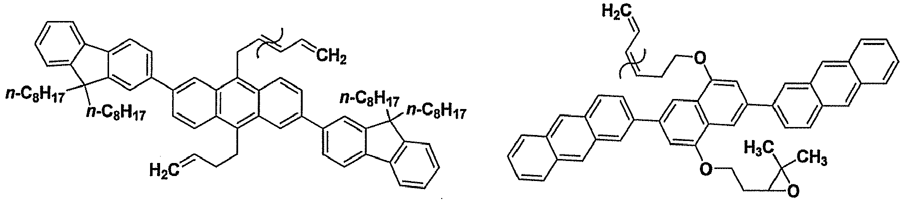

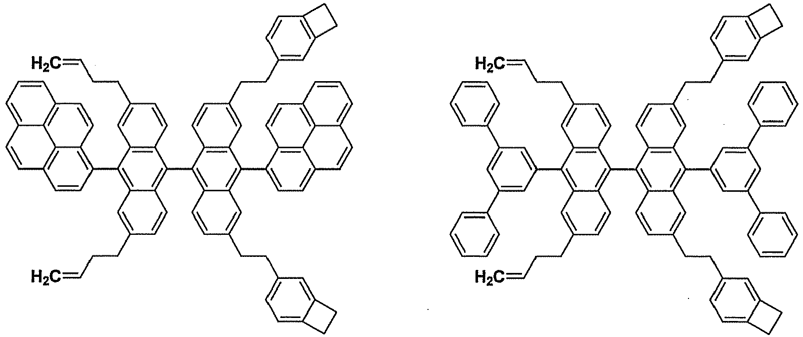

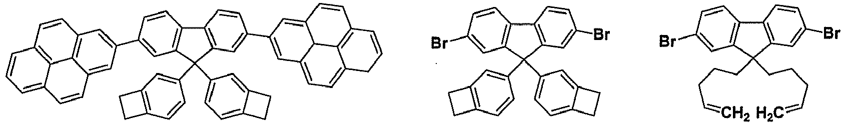

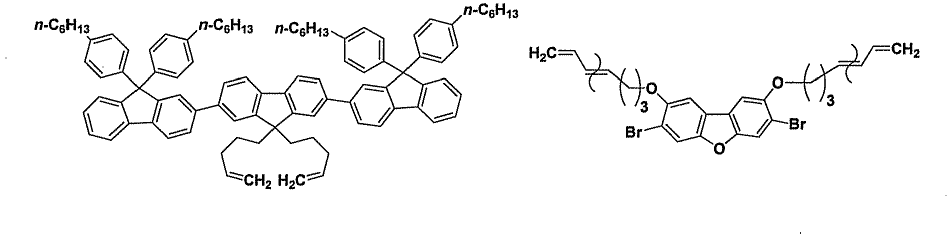

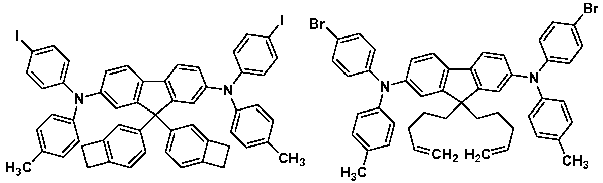

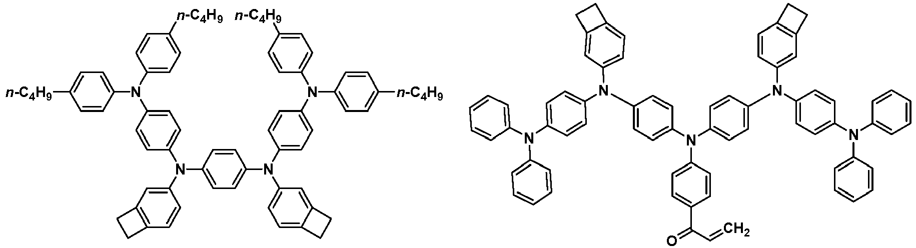

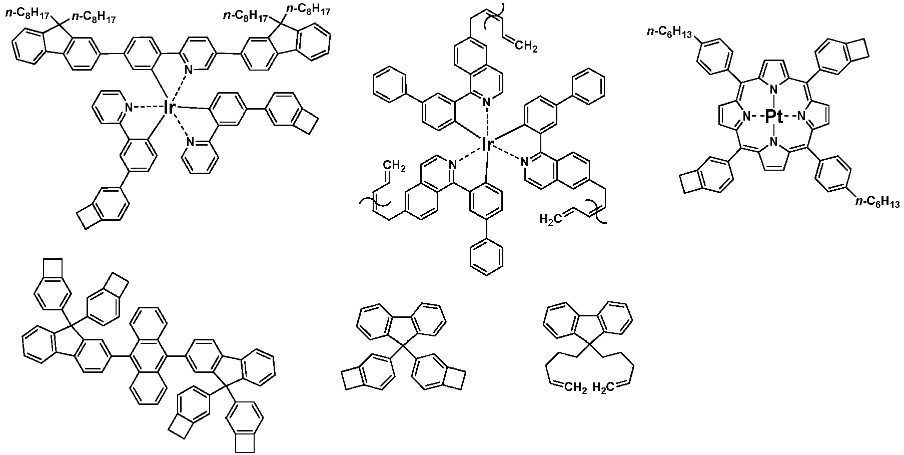

### -Common matter-

In the composition of the present invention, the crosslinkable polymer compound and the crosslinkable low molecular weight compound may each be used singly or in combination.

The weight ratio of the above-described crosslinkable polymer compound to the above-described crosslinkable low molecular weight compound is usually 99:1 to 50:50 (that is, 99/1 to 50/50), and from the standpoint of the curability of the composition, it is preferably 99:1 to 70:30, further preferably 99:1 to 90:10.

The number of moles of the crosslinkable group in one gram of the composition of the present invention is usually 1.0×10⁻⁶ to 1.0×10⁻² mol, and from the standpoint of the curability of the composition, it is preferably 1.0×10⁻⁴ to 1.0×10⁻² mol, more preferably 1.0×10⁻³ to 1.0×10⁻² mol.

The number of moles of the crosslinkable group in one gram of the above-described crosslinkable polymer compound is usually 1.0×10⁻⁶ to 5.0×10⁻³ mol, and from the standpoint of the curability of the composition, it is preferably 1.0×10⁻⁵ to 5.0×10⁻³ mol, more preferably 1.0×10⁻⁴ to 5.0×10⁻³ mol.

The number of moles of the crosslinkable group in one gram of the above-described crosslinkable low molecular weight compound is usually 1.0×10⁻⁶ to 1.0×10⁻² mol, and from the standpoint of the curability of the composition, it is preferably 1.0×10⁻⁵ to 5.0×10⁻³ mol, more preferably 1.0×10⁻⁴ to 5.0×10⁻³ mol.

### <Composition>

The composition of the present invention is capable of further containing a solvent (hereinafter, a composition containing a solvent is referred to as "liquid composition" in some cases).

The composition of the present invention is useful for fabrication of light emitting devices and organic transistors. The liquid composition of the present invention is in the liquid state in fabricating a device, and typically in the liquid state under normal pressure (that is, 1 atm) at 25°C.

The composition of the present invention may contain a low molecular weight light emitting material, a hole transporting material, an electron transporting material, an additive for regulating viscosity and/or surface tension, an antioxidant and the like. These optional components may each be used singly or in combination.

When the composition of the present invention contains an optional component, the proportion of the crosslinkable polymer compound and the crosslinkable low molecular weight compound is usually 80 to 99.9 wt%, preferably 85 to 99.9 wt% with respect to the weight of the composition excluding the solvent, from the standpoint of the curability of the composition.

The above-described low molecular weight light emitting material includes naphthalene derivatives, anthracene, anthracene derivatives, perylene, perylene derivatives, polymethine dyes, xanthene dyes, coumarin dyes, cyanine dyes, metal complexes having a 8-hydroxyquinoline metal complex as a ligand, metal complexes having a 8-hydroxyquinoline derivative as a ligand, other fluorescent metal complexes; phosphorescent metal complexes such as iridium complexes, platinum complexes and the like; phosphorescent metal complexes having a phenylpyridine derivative, a phenylisoquinoline derivative, a 2,2'-bipyridine derivative or the like as a ligand; fluorescent materials of low molecular weight compounds such as aromatic amines, tetraphenylcyclopentadiene, tetraphenylcyclopentadiene derivatives, tetraphenylcyclobutadiene, tetraphenylcyclobutadiene derivatives, stilbene compounds, silicon-containing aromatic compounds, oxazole compounds, furoxan compounds, thiazole compounds, tetraarylmethane compounds, thiadiazole compounds, pyrazole compounds, metacyclophane compounds, acetylene compounds and the like, and includes also materials described in JP-A No. 57-51781, JP-A No. 59-194393 and the like.

The above-described hole transporting material includes polyvinylcarbazole and derivatives thereof, polysilane and derivatives thereof, polysiloxane derivatives having an aromatic amine in the side chain or the main chain, pyrazoline derivatives, arylamine derivatives, stilbene derivatives, triphenyldiamine derivatives, polyaniline and derivatives thereof, polythiophene and derivatives thereof, polypyrrole and derivatives thereof, poly(p-phenylenevinylene) and derivatives thereof, poly(2,5-thienylenevinylene) and derivatives thereof and the like.

The above-described electron transporting material includes oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, metal complexes of 8-hydroxyquinoline and derivatives thereof, polyquinoline and derivatives thereof, polyquinoxaline and derivatives thereof, polyfluorene and derivatives thereof and the like.

As the above-described additive for regulating viscosity and/or surface tension, a high molecular weight compound (thickening agent) or a poor solvent for enhancing viscosity, a low molecular weight compound for lowering viscosity, a surfactant for lowering surface tension, and the like may be used, if necessary in combination.

The above-described high molecular weight compound may advantageously be one which does not disturb light emission and charge transportation, and usually a compound soluble in a solvent which the composition can contain. As the high molecular weight compound, high molecular weight polystyrene, high molecular weight polymethyl methacrylate and the like can be used. The above-described high molecular weight compound has a polystyrene-equivalent weight-average molecular weight of preferably 500000 or more, more preferably 1000000 or more. It is also possible to use a poor solvent as a thickening agent.

The above-described antioxidant may advantageously be one which does not disturb light emission and charge transportation, and when the composition contains a solvent, the antioxidant is usually a compound soluble in the solvent. The antioxidant includes phenol antioxidants, phosphorus antioxidants and the like. By use of the antioxidant, the storage stability of the above-described composition and the solvent can be improved.

When the composition of the present invention contains a hole transporting material, the proportion of the hole transporting material in the liquid composition is usually 1 to 80 wt%, preferably 5 to 60 wt%.

When the composition of the present invention contains an electron transporting material, the proportion of the electron transporting material in the liquid composition is usually 1 to 80 wt%, preferably 5 to 60 wt%.

In fabricating a light emitting device using the liquid composition of the present invention, a solvent has only to be removed by drying after coating of the composition of the present invention, and this is advantageous for production since the same method can be applied also in the case of mixing of a charge transporting material and a light emitting material. Drying may be performed under condition of heating at about 50 to 150°C, or drying may be performed under a reduced pressure of about 10⁻³ Pa.

For film formation using the composition of the present invention, coating methods such as a spin coat method, a casting method, a micro gravure coat method, a gravure coat method, a bar coat method, a roll coat method, a wire bar coat method, a dip coat method, a slit coat method, a cap coat method, a capillary coat method, a spray coat method, a screen printing method, a flexo printing method, an offset printing method, an inkjet print method, a nozzle coat method and the like can be used.

The proportion of a solvent in the liquid composition of the present invention is usually 1 to 99.9 wt%, preferably 60 to 99.9 wt%, more preferably 90 to 99.5 wt% with respect to the total weight of the liquid composition. The viscosity of the liquid composition varies depending on the printing method, and is preferably 0.5 to 500 mPa·s at 25°C, and when the liquid composition passes through a discharge apparatus such as in an inkjet print method and the like, the viscosity is preferably 0.5 to 20 mPa·s at 25°C for preventing curved flying and clogging in discharging.

As the solvent contained in the liquid composition, those capable of dissolving or dispersing components other than the solvent in the liquid composition are preferable. The solvent includes chlorine-based solvents such as chloroform, methylene chloride, 1,2-dichloroethane, 1,1,2-trichloroethane, chlorobenzene, o-dichlorobenzene and the like, ether solvents such as tetrahydrofuran, dioxane and the like, aromatic hydrocarbon solvents such as toluene, xylene, trimethylbenzene, mesitylene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone and the like, ester solvents such as ethyl acetate, butyl acetate, methyl benzoate, ethyl cellosolve acetate and the like, polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol and the like and derivatives thereof, alcohol solvents such as methanol, ethanol, propanol, isopropanol, cyclohexanol and the like, sulfoxide solvents such as dimethyl sulfoxide and the like, and amide solvents such as N-methyl-2-pyrrolidone, N,N-dimethylformamide and the like. These solvents may be used singly or in combination. Of the above-described solvents, at least one organic solvent having a structure containing at least one benzene ring and having a melting point of 0°C or lower and a boiling point of 100°C or higher is preferably contained, from the standpoint of viscosity, film formability and the like. From the standpoint of the solubility of components other than the solvent in the liquid composition in an organic solvent, uniformity in film formation, a viscosity property and the like, the solvent includes preferably aromatic hydrocarbon solvents, aliphatic hydrocarbon solvents, ester solvents and ketone solvents, and preferable are toluene, xylene, ethylbenzene, diethylbenzene, trimethylbenzene, mesitylene, n-propylbenzene, isopropylbenzene, n-butylbenzene, isobutylbenzene, sec-butylbenzene, anisole, ethoxybenzene, 1-methylnaphthalene, cyclohexane, cyclohexanone, cyclohexylbenzene, bicyclohexyl, cyclohexenylcyclohexanone, n-heptylcyclohexane, n-hexylcyclohexane, methyl benzoate, 2-propylcyclohexanone, 2-heptanone, 3-heptanone, 4-heptanone, 2-octanone, 2-nonanone, 2-decanone and dicyclohexylketone, and it is more preferable that at least one of xylene, anisole, mesitylene, cyclohexylbenzene and bicyclohexyl methyl benzoate is contained.

The number of the kind of the solvent contained in the liquid composition is preferably two or more, more preferably two to three, particularly preferably two, from the standpoint of film formability and from the standpoint of device properties and the like.

When two solvents are contained in the liquid composition, one of them may be in the solid state at 25°C. From the standpoint of film formability, it is preferable that one solvent has a boiling point of 180°C or higher and another solvent has a boiling point of lower than 180°C, it is more preferable that one solvent has a boiling point of 200°C or higher and another solvent has a boiling point of lower than 180°C. From the standpoint of viscosity, it is preferable that components of the liquid composition excluding the solvent are dissolved at a concentration of 0.2 wt% or more in solvents at 60°C, and it is preferable that components of the liquid composition excluding the solvent are dissolved at a concentration of 0.2 wt% or more in one of two solvents at 25°C.

When three solvents are contained in the liquid composition, one to two solvents among them may be in the solid state at 25°C. From the standpoint of film formability, it is preferable that at least one of three solvents has a boiling point of 180°C or higher and at least one of them has a boiling point of lower than 180°C, it is more preferable that at least one of three solvents has a boiling point of 200 to 300°C and at least one of them has a boiling point of lower than 180°C. From the standpoint of viscosity, it is preferable that components of the liquid composition excluding the solvent are dissolved at a concentration of 0.2 wt% or more in two of three solvents at 60°C, and it is preferable that components of the liquid composition excluding the solvent are dissolved at a concentration of 0.2 wt% or more in one of three solvents at 25°C.

When two or more solvents are contained in the liquid composition, the amount of a solvent having the highest boiling point is preferably 40 to 90 wt%, more preferably 50 to 90 wt% and further preferably 65 to 85 wt% with respect to the total weight of all solvents contained in the liquid composition, from the standpoint of viscosity and film formability.

### <Film>

The film of the present invention is obtained by using the composition of the present invention. The film includes luminous films, electric conductive films, organic semiconductor films and the like.

The first embodiment of the film of the present invention is a film in which a crosslinkable polymer compound and a crosslinkable low molecular weight compound contained in the composition of the present invention are retained. The second embodiment of the film of the present invention is a film in which a crosslinkable polymer compound and a crosslinkable low molecular weight compound contained in the composition of the present invention are crosslinked.

In the second embodiment of the film of the present invention, the above-described crosslinkage can be performed by an external stimulus such as heat, light and the like, and a film may be formed while crosslinking a crosslinkable polymer compound and a crosslinkable low molecular weight compound, or a film may be formed before crosslinkage thereof.

When the composition and the film of the present invention are cross-linked by heat, the heating temperature is usually from room temperature to 300°C. The upper limit of the heating temperature is preferably 250°C, further preferably 190°C and particularly preferably 170°C, from the standpoint of easiness of fabrication of a film, and the lower limit of the heating temperature is preferably 50°C, further preferably 70°C and particularly preferably 100°C, from the standpoint of easiness of handling of the composition at room temperature. The crosslinkage proportion can be regulated by the heating temperature and the heating time.

When the composition and the film of the present invention are cross-linked by light, the irradiation light includes preferably an ultraviolet ray, a near ultraviolet ray and a visible ray, more preferably an ultraviolet ray and a near ultraviolet ray. The crosslinkage proportion and the crosslinking speed can be regulated by the exposure wavelength and the irradiation time.

The luminous film shows a quantum yield of light emission of preferably 50% or more, more preferably 60% or more and further preferably 70% or more, from the standpoint of the device luminance, the light emission voltage and the like.

The electric conductive film has a surface resistance of preferably 1 KΩ/□ or less. By doping the film with a Lewis acid, an ionic compound or the like, the electric conductivity can be enhanced. The surface resistance is more preferably 100 Ω/□ or less, further preferably 10 Ω/□ or less.

For the organic semiconductor film, either larger one of electron mobility or hole mobility is preferably 10⁻⁵ cm²/V/s or more, more preferably 10⁻³ cm²/V/s or more, further preferably 10⁻¹ cm²/V/s or more. An organic transistor can be fabricated using the organic semiconductor film. Specifically, an insulation film made of SiO₂ and the like and a gate electrode are formed on a Si substrate, then, an organic semiconductor film is formed on the Si substrate, and a source electrode and a drain electrode are formed with Au or the like, thereby, an organic transistor can be obtained.

### <Organic transistor>

The organic transistor of the present invention is an organic transistor obtained by using the composition of the present invention. An electric field effect transistor which is one embodiment of the organic transistor will be illustrated below.

The composition of the present invention can be used suitably as the material of an electric field effect transistor, especially, as an active layer. Regarding the structure of an electric field effect transistor, it may be usually permissible that a source electrode and a drain electrode are disposed in contact with an active layer obtained by using the composition of the present invention, further, a gate electrode is disposed so as to sandwich an insulation layer in contact with the active layer.

The electric field effect transistor is usually formed on a supporting substrate. As the supporting substrate, use can be made of glass substrates and flexible film substrates and also plastic substrates.

The electric field effect transistor can be produced by known methods, for example, a method described in JP-A No. 5-110069.

In forming an active layer, it is advantageous and preferable to use the liquid composition of the present invention, from the standpoint of production. For film formation from the liquid composition of the present invention, coating methods can be used such as a spin coat method, a casting method, a micro gravure coat method, a gravure coat method, a bar coat method, a roll coat method, a wire bar coat method, a dip coat method, a slit coat method, a cap coat method, a capillary coat method, a spray coat method, a screen printing method, a flexo printing method, an offset printing method, an inkjet print method, a nozzle coat method and the like.

An encapsulated electric field effect transistor obtained by fabricating an electric field effect transistor and then encapsulating this is preferable. By this, an electric field effect transistor is blocked from atmospheric air and lowering of the property of an electric field effect transistor can be suppressed.

The encapsulation method includes a method of covering with an ultraviolet (UV) curable resin, a thermosetting resin or an inorganic SiONx film and the like, a method of pasting a glass plate or a film with an UV curable resin, a thermosetting resin and the like, and other methods. For effectively performing blocking from atmospheric air, it is preferable that a process from fabrication of an electric field effect transistor until encapsulation thereof is carried out without exposing to atmospheric air (for example, in a dried nitrogen atmosphere, or in vacuum).

### <Organic photoelectric conversion device>

The organic photoelectric conversion device of the present invention (for example, solar battery) is an organic photoelectric conversion device obtained by using the composition of the present invention.

The composition of the present invention is suitable as the material of an organic photoelectric conversion device, especially, as the material used in an organic semiconductor layer of a Schottky barrier type device utilizing an interface between an organic semiconductor and a metal, or as the material used in an organic semiconductor layer of a pn hetero-junction type device utilizing an interface between an organic semiconductor and an inorganic semiconductor or between organic semiconductors.

Further, the composition of the present invention can be suitably used as the electron donative material and the electron acceptive material in a bulk hetero-junction type device having an increased donor-acceptor contact area, or as the electron donative conjugated material (dispersion support) of an organic photoelectric conversion device using a polymer-low molecular weight composite system, for example, a bulk hetero-junction type organic photoelectric conversion device containing a fullerene derivative dispersed as an electron acceptor.

The structure of an organic photoelectric conversion device includes, for example, a structure in which a p-type semiconductor layer is formed on an ohmic electrode, for example, on ITO, further, an n-type semiconductor layer is laminated, and an ohmic electrode is disposed thereon, in the case of a pn hetero-junction type device.

The organic photoelectric conversion device is usually formed on a supporting substrate. As the supporting substrate, use can be made of a glass substrate, a flexible film substrate, a plastic substrate and the like.

The organic photoelectric conversion device can be produced by known methods, for example, a method described in Synth. Met., 102, 982 (1999) and a method described in Science, 270, 1789 (1995).

### <Light emitting device>

Next, the light emitting device of the present invention will be illustrated.

The light emitting device of the present invention is a light emitting device having electrodes consisting of an anode and a cathode and a layer which is disposed between the electrodes and obtained by using the composition of the present invention in which, preferably, the layer is a light emitting layer or a charge transporting layer. The light emitting device of the present invention includes (1) a light emitting device in which an electron transporting layer is disposed between a cathode and a light emitting layer, (2) a light emitting device in which a hole transporting layer is disposed between an anode and a light emitting layer, (3) a light emitting device in which an electron transporting layer is disposed between a cathode and a light emitting layer and a hole transporting layer is disposed between an anode and a light emitting layer; and other light emitting devices.

Examples thereof include the following structures a) to d).
a) anode/light emitting layer/cathode
b) anode/hole transporting layer/light emitting layer/cathode
c) anode/light emitting layer/electron transporting layer/cathode
d) anode/hole transporting layer/light emitting layer/electron transporting layer/cathode (here, "/" means adjacent lamination of layers. The same shall apply hereinafter.)

The above-described light emitting layer is a layer having a function of emitting light, the above-described hole transporting layer is a layer having a function of transporting holes, and the above-described electron transporting layer is a layer having a function of transporting electrons. The electron transporting layer and the hole transporting layer are collectively called a charge transporting layer. Two or more layers of the light emitting layer, two or more layers of the hole transporting layer and two or more layers of the electron transporting layer may be independently used, respectively. The hole transporting layer adjacent to the light emitting layer is called an interlayer layer in some cases.

The method of forming the light emitting layer includes methods of film formation from a solution. For film formation from a solution, coating methods such as a spin coat method, a casting method, a micro gravure coat method, a gravure coat method, a bar coat method, a roll coat method, a wire bar coat method, a dip coat method, a slit coat method, a cap coat method, a capillary coat method, a spray coat method, a screen printing method, a flexo printing method, an offset printing method, an inkjet print method, a nozzle coat method and the like can be used. This film formation from a solution is useful also for film formation of a hole transporting layer and an electron transporting layer described later.

In fabricating a light emitting device, a solvent has only to be removed by drying after coating using the liquid composition of the present invention, and this is advantageous for production since the same method can be applied also in the case of mixing of a charge transporting material and a light emitting material.

The thickness of the light emitting layer may be advantageously selected so as to give appropriate values of driving voltage and light emission efficiency, and is for example 1 nm to 1 µm, preferably 2 nm to 500 nm, further preferably 5 nm to 200 nm.

In the light emitting device of the present invention, also a light emitting material other than the composition of the present invention may be used in the light emitting layer. In the light emitting device of the present invention, the light emitting layer containing a light emitting material other than the composition of the present invention may be laminated with a light emitting layer obtained by using the composition of the present invention.

The light emitting material other than the composition of the present invention includes low molecular weight compounds such as naphthalene derivatives, anthracene and derivatives thereof, perylene and derivatives thereof, dyes such as polymethine dyes, xanthene dyes, coumarin dyes, cyanine dyes and the like, metal complexes of 8-hydroxyquinoline and derivatives thereof, aromatic amines, tetraphenylcyclopentadiene and derivatives thereof, tetraphenylbutadiene and derivatives thereof and the like, and also materials described in JP-A Nos. 57-51781 and 59-194393, and the like.

When the light emitting device of the present invention has a hole transporting layer, the hole transporting material to be used is the same as the above-described hole transporting material, and preferable are polymer hole transporting materials such as polyvinylcarbazole and derivatives thereof, polysilane and derivatives thereof, polysiloxane derivatives having an aromatic amine compound group in the side chain or the main chain, polyaniline and derivatives thereof, polythiophene and derivatives thereof, poly(p-phenylenevinylene) and derivatives thereof, poly(2,5-thienylenevinylene) and derivatives thereof and the like, more preferable are polyvinylcarbazole and derivatives thereof, polysilane and derivatives thereof and polysiloxane derivatives having an aromatic amine in the side chain or the main chain. In the case of a low molecular weight hole transporting material, use of the material dispersed in a polymer binder is preferable.

The method of forming a hole transporting layer includes a method of film formation from a mixed solution with a polymer binder in the case of a low molecular weight hole transporting material, and a method of film formation from a solution in the case of a polymer hole transporting material.

As the polymer binder to be mixed, those not extremely disturbing charge transportation are preferable, and those showing no strong absorption for a visible ray are suitably used. The polymer binder includes polycarbonates, polyacrylates, polymethyl acrylate, polymethyl methacrylate, polystyrene, polyvinyl chloride, polysiloxane and the like.

The thickness of the hole transporting layer may be advantageously selected so as to give suitable values of driving voltage and light emission efficiency, and is for example 1 nm to 1 µm, preferably 2 nm to 500 nm, further preferably 5 nm to 200 nm.

When the light emitting device of the present invention has an electron transporting layer, the electron transporting material to be used is the same as the above-described electron transporting material, and preferable are oxadiazole derivatives, benzoquinone and derivatives thereof, anthraquinone and derivatives thereof, metal complexes of 8-hydroxyquinoline and derivatives thereof, polyquinoline and derivatives thereof, polyquinoxaline and derivatives thereof and polyfluorene and derivatives thereof, more preferable are 2-(4-biphenylyl)-5-(4-tert-butylphenyl)-1,3,4-oxadiazole, benzoquinone, anthraquinone, tris(8-quinolinol)aluminum and polyquinoline.

The method of forming an electron transporting layer includes a vacuum vapor deposition method from a powder and a method of film formation from a solution or melted state in the case of a low molecular weight electron transporting material, and a method of film formation from a solution or melted state in the case of a polymer electron transporting material. In film formation from a solution or melted state, a polymer binder may be used together.

As the polymer binder to be mixed, those not extremely disturbing charge transportation are preferable, and those showing no strong absorption for a visible ray are suitably used. The polymer binder includes poly(N-vinylcarbazole), polyaniline and derivatives thereof, polythiophene and derivatives thereof, poly(p-phenylenevinylene) and derivatives thereof, poly(2,5-thienylenevinylene) and derivatives thereof, polycarbonates, polyacrylates, polymethyl acrylate, polymethyl methacrylate, polystyrene, polyvinyl chloride, polysiloxane and the like.

The thickness of the electron transporting layer may be advantageously selected so as to give suitable values of driving voltage and light emission efficiency, and is for example 1 nm to 1 µm, preferably 2 nm to 500 nm, further preferably 5 nm to 200 nm.

Among charge transporting layers disposed adjacent to an electrode, those having a function of improving charge injection efficiency from an electrode and having an effect of lowering the driving voltage of a device are, in particularly, called charge injection layers (hole injection layer, electron injection layer) in some cases.

Further, for improving close adherence with an electrode and improving charge injection from an electron, the above-described charge injection layer or insulation layer may be disposed adjacent to the electrode, alternatively, for improving close adherence of an interface and preventing mixing, a thin buffer layer may be inserted into an interface of a charge transporting layer and a light emitting layer.

The order and the number of layers to be laminated, and the thickness of each layer may advantageously be selected in view of light emission efficiency and device life.

In the present invention, the light emitting device having a charge injection layer includes a light emitting device having a charge injection layer disposed adjacent to a cathode and a light emitting device having a charge injection layer disposed adjacent to an anode.

Examples thereof include the following structures e) to p).
e) anode/charge injection layer/light emitting layer/cathode
f) anode/light emitting layer/charge injection layer/cathode
g) anode/charge injection layer/light emitting layer/charge injection layer/cathode
h) anode/charge injection layer/hole transporting layer/light emitting layer/cathode
i) anode/hole transporting layer/light emitting layer/charge injection layer/cathode
j) anode/charge injection layer/hole transporting layer/light emitting layer/charge injection layer/cathode
k) anode/charge injection layer/light emitting layer/charge transporting layer/cathode
l) anode/light emitting layer/electron transporting layer/charge injection layer/cathode
m) anode/charge injection layer/light emitting layer/electron transporting layer/charge injection layer/cathode
n) anode/charge injection layer/hole transporting layer/light emitting layer/charge transporting layer/cathode
o) anode/hole transporting layer/light emitting layer/electron transporting layer/charge injection layer/cathode
p) anode/charge injection layer/hole transporting layer/light emitting layer/electron transporting layer/charge injection layer/cathode

The charge injection layer includes a layer containing an electric conductive polymer, a layer disposed between an anode and a hole transporting layer and containing a material having ionization potential of a value between an anode material and a hole transporting material contained in the hole transporting layer, a layer disposed between a cathode and an electron transporting layer and containing a material having electron affinity of a value between a cathode material and an electron transporting material contained in the electron transporting layer, and the like.

When the above-described charge injection layer is a layer containing an electric conductive polymer, the electric conductivity of the electric conductive polymer is preferably 10⁻⁵ to 10³ S/cm, and for decreasing leak current between light emission picture elements, it is more preferably 10⁻⁵ to 10² S/cm, further preferably 10⁻⁵ to 10¹ S/cm. Usually, for the electric conductivity of the electric conductive polymer to be 10⁻⁵ to 10³ S/cm, the electric conductive polymer is doped with a suitable amount of ions.

As the kind of ions to be doped, an anion is used in the case of a hole injection layer and a cation is used in the case of an electron injection layer. Examples of the anion include a polystyrenesulfonic ion, an alkylbenzenesulfonic ion, a camphorsulfonic ion and the like, and examples of the cation include a lithium ion, a sodium ion, a potassium ion, a tetrabutylammonium ion and the like.

The thickness of the charge injection layer is, for example, 1 to 100 nm, preferably 2 to 50 nm.

The material to be used in the charge injection layer includes polyaniline and its derivatives, polythiophene and its derivatives, polypyrrole and its derivatives, polyphenylenevinylene and its derivatives, polythienylenevinylene and its derivatives, polyquinoline and its derivatives, polyquinoxaline and its derivatives, polymers containing an aromatic amine structure in the main chain or the side chain, and the like (electric conductive polymers), and metal phthalocyanines (copper phthalocyanine and the like), carbon and the like.

The insulation layer has a function of making charge injection easy. The average thickness of this insulation layer is usually 0.1 to 20 nm, preferably 0.5 to 10 nm, more preferably 1 to 5 nm. As the material of the insulation layer, metal fluorides, metal oxides, organic insulating materials and the like are mentioned. The light emitting device having an insulation layer includes a light emitting device having an insulation layer disposed adjacent to a cathode and a light emitting device having an insulation layer disposed adjacent to an anode.

Examples thereof include the following structures q) to ab).
q) anode/insulation layer/light emitting layer/cathode
r) anode/light emitting layer/insulation layer/cathode
s) anode/insulation layer/light emitting layer/insulation layer/cathode
t) anode/insulation layer/hole transporting layer/light emitting layer/cathode
u) anode/hole transporting layer/light emitting layer/insulation layer/cathode
v) anode/insulation layer/hole transporting layer/light emitting layer/insulation layer/cathode
w) anode/insulation layer/light emitting layer/electron transporting layer/cathode
x) anode/light emitting layer/electron transporting layer/insulation layer/cathode
y) anode/insulation layer/light emitting layer/electron transporting layer/insulation layer/cathode
z) anode/insulation layer/hole transporting layer/light emitting layer/electron transporting layer/cathode
aa) anode/hole transporting layer/light emitting layer/electron transporting layer/insulation layer/cathode
ab) anode/insulation layer/hole transporting layer/light emitting layer/electron transporting layer/insulation layer/cathode

The substrate for forming a light emitting device of the present invention may advantageously be one which does not chemically change in forming an electrode and forming an organic layer, and examples thereof include substrates made of glass, plastic, polymer film, silicon and the like. In the case of an opaque substrate, it is preferable that the opposite electrode is transparent or semi-transparent.

In the light emitting device of the present invention, it is usually preferable that at least one of electrodes consisting of an anode and a cathode is transparent or semi-transparent, and the anode is transparent or semi-transparent.

As the material of the anode, an electric conductive metal oxide film, a semi-transparent metal film and the like are used, and films fabricated using electric conductive inorganic compounds such as indium oxide, zinc oxide, tin oxide, and composite thereof: indium•tin•oxide (ITO), indium•zinc•oxide and the like, and NESA, gold, platinum, silver, copper and the like are used, and preferable are ITO, indium•zinc•oxide and tin oxide. The fabrication method includes a vacuum vapor deposition method, a sputtering method, an ion plating method, a plating method and the like. As the anode, organic transparent electric conductive films obtained by using polyaniline and its derivatives, polythiophene and its derivatives and the like may be used.

The thickness of the anode is for example 10 nm to 10 µm, preferably 20 nm to 1 µm, further preferably 50 nm to 500 nm, in view of light transmission and electric conductivity.

For making charge injection easy, a layer made of a phthalocyanine derivative, an electric conductive polymer, carbon and the like or a layer made of a metal oxide, a metal fluoride, an organic insulation material and the like may be provided on the anode.

As the material of a cathode, materials of small work function are preferable, and use is made of metals such as lithium, sodium, potassium, rubidium, cesium, beryllium, magnesium, calcium, strontium, barium, aluminum, scandium, vanadium, zinc, yttrium, indium, cerium, samarium, europium, terbium, ytterbium and the like, alloys composed of two or more of them, or alloys composed of at least one of them and at least one of gold, silver, platinum, copper, manganese, titanium, cobalt, nickel, tungsten and tin, and graphite or graphite intercalation compounds and the like. Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, a calcium-aluminum alloy and the like. The cathode may take a lamination structure composed of two or more layers.

The thickness of the cathode is for example 10 nm to 10 µm, preferably 20 nm to 1 µm, more preferably 50 nm to 500 nm in view of electric conductivity and durability.

The cathode fabrication method includes a vacuum vapor deposition method, a sputtering method, a laminate method of thermally compression-bonding a metal film, and the like. A layer made of an electric conductive polymer, or a layer made of a metal oxide, a metal fluoride, an organic insulation material and the like, may be provided between a cathode and an organic layer, and after fabrication of a cathode, a protective layer for protecting the light emitting device may be installed. For use of the light emitting device stably for a long period of time, it is preferable to install a protective layer and/or a protective cover, for protecting a device from outside.

For the protective layer, resins, metal oxides, metal fluorides, metal borides and the like can be used. As the protective cover, a glass plate, and a plastic plate having a surface which has been subjected to a low water permeation treatment, and the like can be used, and a method in which the cover is pasted to a device substrate with a thermosetting resin or a photo-curing resin to attain sealing is suitably used. When a space is kept using a spacer, blemishing of a device can be prevented easily. If an inert gas such as nitrogen, argon and the like is filled in this space, oxidation of a cathode can be prevented, further, by placing a drying agent such as barium oxide and the like in this space, it becomes easy to suppress moisture adsorbed in a production process from imparting damage to the device. It is preferable to adopt at least one strategy of them.

The light emitting device of the present invention can be used for surface light sources, displays such as segment displays, dot matrix displays, liquid crystal displays (for example, backlight and the like), flat panel displays and the like; and other apparatuses.

For obtaining light emission in the form of plane using a light emitting device of the present invention, it may be advantages to place a surface anode and a surface cathode so as to overlap. For obtaining light emission in the form of pattern, there are a method in which a mask having a window in the form of pattern is placed on the surface of the above-described surface light emitting device, a method in which an organic layer at no light emission parts is formed with extremely large thickness to cause substantially no light emission, and a method in which either an anode or a cathode, or both electrodes are formed in the form pattern. By forming a pattern by any of these methods, and placing several electrodes so that ON/OFF is independently possible, a display device of segment type is obtained which can display digits, letters, simple marks and the like. Further, for providing a dot matrix device, it may be permissible that both an anode and a cathode are formed in the form of stripe, and placed so as to cross. By a method in which compounds showing different emission colors are painted separately or a method in which a color filter or a fluorescence conversion filter is used, partial color display and multi-color display are made possible. In the case of a dot matrix device, passive driving is possible, and active driving may also be carried out in combination with TFT and the like. These display devices can be used as a display of computers, televisions, portable terminals, cellular telephones, car navigations, video camera view finders, and the like.

Further, the above-described surface light emitting device is of self emitting and thin type, and can be suitably used as a surface light source for back light of a liquid crystal display, or as a surface light source for illumination. Examples of illumination light sources include light emission colors such as white light emission, red light emission, green light emission, blue light emission and the like. If a flexible substrate is used, it can also be used as a curved light source or a display.

### EXAMPLES

Examples for illustrating the present invention in detail will be shown below.

In examples, for the number-average molecular weight and the weight-average molecular weight, the polystyrene-equivalent number-average molecular weight and weight-average molecular weight were measured by size exclusion chromatography (SEC) (manufactured by Shimadzu Corp., trade name: LC-10Avp). SEC using an organic solvent as the mobile phase is called gel permeation chromatography (GPC). A polymer to be measured was dissolved in tetrahydrofuran at a concentration of about 0.5 wt%, and 30 µL of the solution was injected into GPC. Tetrahydrofuran (THF) was used as the mobile phase of GPC, and flowed at a flow rate of 0.6 mL/min. As the column, two columns of TSKgel SuperHM-H (manufactured by Tosoh Corp.) and one column of TSKgel SuperH2000 (manufactured by Tosoh Corp.) were serially connected. As the detector, a differential refractive index detector (manufactured by Shimadzu Corp., trade name: RID-10A) was used. The measurement was carried out at 40°C.

### <Synthesis Example 1> (Synthesis of compound M-1)

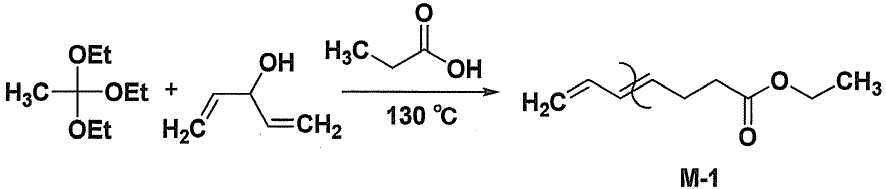

Under an argon atmosphere, divinylcarbinol (25.24 g), triethyl orthoacetate (340 g) and propionic acid (0.20 g) were mixed, and heated at 130°C for 4 hours while removing ethanol using a Dean-Stark tube. After completion of the reaction, the resultant reaction liquid was cooled, and to this was added hexane (300 ml) and ion exchanged water (300 ml) and the mixture was stirred at 60°C for 3 hours. After liquid separation, the organic layer was washed with ion exchanged water (300 ml, three times), and dried over sodium sulfate. The resultant organic layer was passed through an alumina flash column, and concentrated. To the resultant oil was added, again, hexane (300 ml), ion exchanged water (300 ml) and propionic acid (0.20 g), and the mixture was stirred at 60°C for 8 hours. After liquid separation, the organic layer was washed with ion exchanged water (300 ml, three times), and dried over sodium sulfate. The resultant organic layer was passed through an alumina flash column and concentrated, to obtain 28 g of a compound M-1.

¹H-NMR (270 MHz, CDCl₃): δ=1.25 (t, 3H), 2.07 (q, 2H), 2.41 (m, 4H), 5.05 (dd, 2H), 5.70 (m, 1H), 6.09 (dd, 1H), 6.29 (m, 1H) ppm.

### <Synthesis Example 2> (Synthesis of compound M-2)

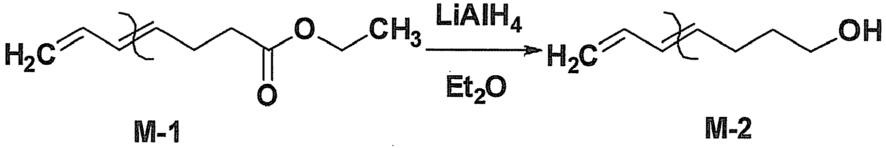

Under an argon atmosphere, the compound M-1 (14.65 g) and diethyl ether (770 ml) were mixed, and cooled down to 0°C. Into the resultant mixed liquid, a 1M lithium aluminum hydride diethyl ether solution (50 ml) was dropped over a period of 1 hour, and the mixture was stirred for 1 hour while maintaining 0°C. Into the reaction solution, a 5 wt% sodium hydroxide aqueous solution (100 ml) was dropped slowly to stop the reaction, then, the organic layer was washed with water (100 ml, three times), and the organic layer after washing was dried over sodium sulfate. The resultant organic layer was passed through an alumina flash column and concentrated, to obtain 8.0 g of a compound M-2.

¹H-NMR (270 MHz, CDCl₃): δ=1.67 (tt, 2H), 2.13-2.28 (m, 2H), 3.63 (q, 2H), 5.04 (dd, 2H), 5.72 (dd, 1H), 6.07 (dd, 1H), 6.30 (m, 1H) ppm.

### <Synthesis Example 3> (Synthesis of compound M-3)

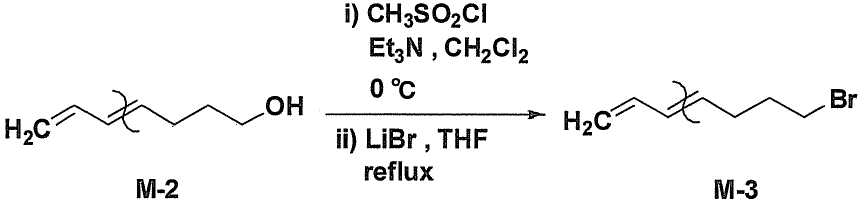

Under an argon atmosphere, the compound M-2 (18.98 g) and dichloromethane (730 ml) were mixed and cooled down to 0°C. Into the resultant mixed liquid, triethylamine (58 ml) was dropped, then, methanesulfonyl chloride (24 ml) was dropped, and the mixture was stirred for 2 hours while maintaining 0°C. Water was added to the resultant reaction solution to stop the reaction, then, the mixture was extracted with diethyl ether and dried over sodium sulfate, to obtain 32 g of a yellow oil.

Under an argon atmosphere, this yellow oil (32 g), lithium bromide (36 g) and THF (400 ml) were mixed and refluxed for 7 hours. The resultant reaction solution was cooled, and ion exchanged water (200 ml) and toluene(500 ml) were added, and the mixture was subjected to liquid separation, and the organic layer was washed with ion exchanged water (100 ml, five times) and dried over sodium sulfate. The resultant organic layer was concentrated, and hexane (100 ml) was added, then, the mixture was passed through alumina flash column and concentrated. The resultant oil was subjected to fractional distillation (3 mmHg, 27°C), to obtain 15.1 g of a compound M-3.

¹H-NMR (270 MHz, CDCl₃): δ=1.96 (tt, 2H), 2.22-2.29 (m, 2H), 3.41 (t, 2H), 5.05 (dd, 2H), 5.65 (m, 1H), 6.10 (dd, 1H), 6.30 (m, 1H) ppm.

### <Synthesis Example 4> (Synthesis of compound M-4)

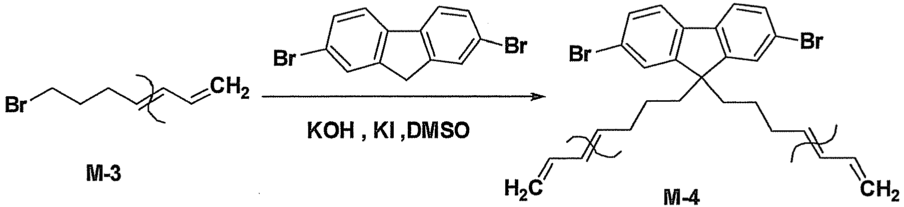

In a 300 ml four-necked flask under an argon atmosphere, the compound M-3 (5.29 g), 2,7-dibromofluorene (4.67 g) and dimethyl sulfoxide (35 ml) were mixed. To the resultant mixed liquid were added potassium hydroxide (3.43 g) and potassium iodide (0.17 g) ground in a mortar, and the mixture was heated at 85°C for 45 minutes. To the resultant mixed liquid were added ion exchanged water (50 ml) and ethyl acetate (100 ml), and the mixture was subjected to liquid separation, then, the organic layer was washed with saturated saline (100 ml, ten times) and dried over sodium sulfate, then, concentrated. The resultant oil was purified by silica gel column chromatography (developing solvent: hexane), to obtain 4.9 g of a compound M-4 as a white solid.

¹H-NMR (270 MHz, CDCl₃): δ=0.68 (m, 4H), 1.81-1.96 (m, 8H), 4.99 (dd, 4H), 5.44 (m, 2H), 5.89 (dd, 2H), 6.22 (td, 2H), 7.47 (m, 6H) ppm.
MS (APCI-MS: Positive) m/z: 512 ([M]⁺).

### <Synthesis Example 5> (Synthesis of compound M-6)

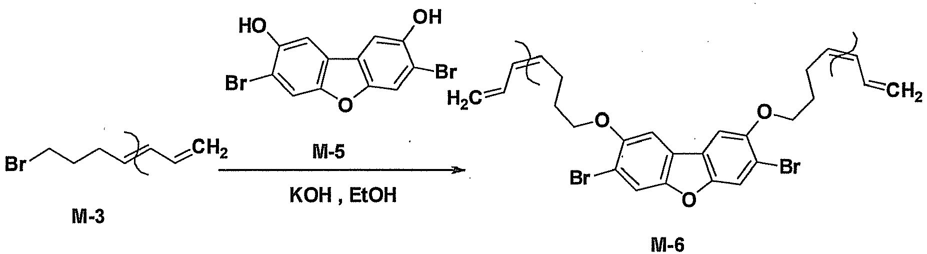

In a 100 ml four-necked flask under an argon atmosphere, the compound M-3 (1.63 g), the compound M-5 (1.63 g) and ethanol (7 ml) were mixed. To the resultant mixed liquid was added potassium hydroxide (0.97 g) ground in a mortar, and the mixture was heated at 60°C for 40 hours. After completion of the reaction, to the resultant reaction liquid were added ion exchanged water (50 ml) and toluene (50 ml), and the mixture was subjected to liquid separation, then, the organic layer was washed with ion exchanged water (40 ml, three times) and dried over sodium sulfate, then, concentrated. The resultant oil was purified by silica gel column chromatography (developing solvent: toluene/hexane=1:1), to obtain 1.1 g of a compound M-6 as a white solid. The compound M-5 was synthesized referring to EP1344788.

¹H-NMR (270 MHz, CDCl₃): δ= 1.97-2.06 (m, 4H), 2.36-2.43 (m, 4H), 4.10 (t, 4H), 5.04 (dd, 4H), 5.78 (m, 2H), 6.14 (m, 2H), 6.32 (m, 2H), 7.32 (s, 2H), 7.73 (s, 2H) ppm.

### <Synthesis Example 6> (Synthesis of compound M-7)

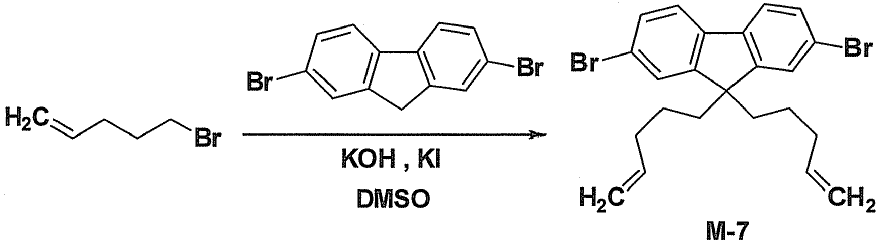

In a 500 ml four-necked flask under an argon atmosphere, 2,7-dibromofluorene (22.7 g), 5-bromo-1-pentene (21.9 g), potassium hydroxide (16.7 g), potassium iodide (1.2 g) and dimethyl sulfoxide (170 ml) were mixed and the mixture was heated at 80°C for 4 hours. After completion of the reaction, the mixture liquid was cooled down to room temperature, and this was mixed with water (300 ml) and toluene (300 ml) and the mixture was subjected to liquid separation. Then, the organic layer was washed with a sodium chloride saturated aqueous solution (300 ml) five times. The resultant organic layer was dried over sodium sulfate, then, purified by column chromatography using hexane as a developing solvent and using silica gel as a filler, to obtain a compound M-7 (25.2 g).

ESI-MS: 460 [M]⁺
¹H-NMR (270 MHz, CDCl₃); δ=0.69 (t, 4H), 1.83 (m, 4H), 1.93 (m, 4H), 4.85 (d, 4H), 5.56 (m, 2H), 7.44-7.53 (m, 6H).

### <Synthesis Example 6> (Synthesis of compound M-8)

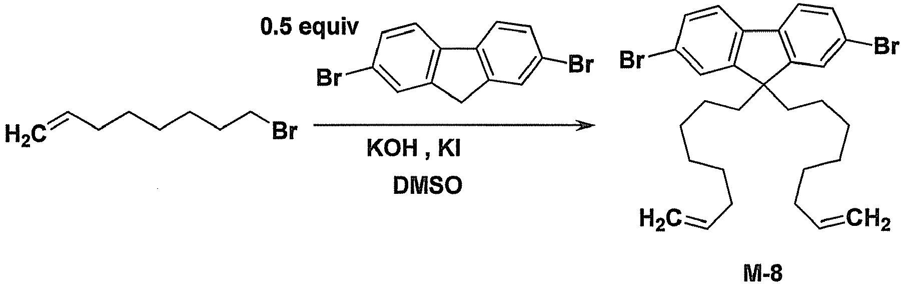

In a 300 ml three-necked flask under an argon atmosphere, 2,7-dibromofluorene (8.1 g), 8-bromo-1-octene (10.0 g), potassium hydroxide (6.0 g), potassium iodide (0.42 g) and dimethyl sulfoxide (60 ml) were mixed and the mixture was heated at 80°C for 4 hours. After completion of the reaction, the mixture was cooled down to room temperature, and this was mixed with water (100 ml) and toluene (100 ml) and the mixture was subjected to liquid separation, then, the resultant organic layer was washed with a sodium chloride saturated aqueous solution (100 ml) five times. The organic layer after washing was dried over sodium sulfate, then, purified by column chromatography using hexane as a developing solvent and using silica gel as a filler, to obtain a compound M-8 (12.8 g).

ESI-MS: 544 [M]⁺
¹H-NMR (270 MHz, CDCl₃); δ=0.58 (m, 4H), 1.06 (m, 8H), 1.18 (m, 4H), 1.92 (m, 8H), 4.90 (d, 4H), 5.73 (m, 2H), 7.43-7.52 (m, 6H).

### <Synthesis Example 7> (Synthesis of compound M-10)

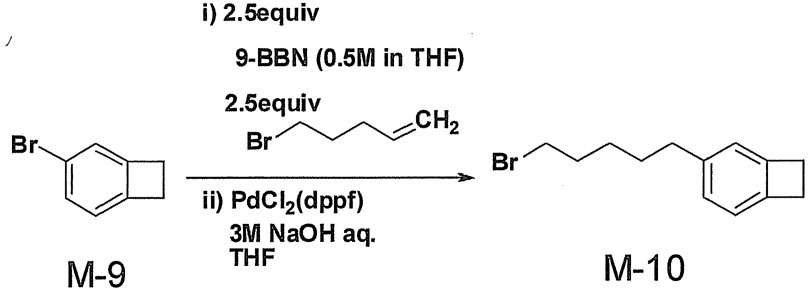

In a 300 ml four-necked flask under an argon atmosphere, 5-bromo-1-pentene (7.45 g) and THF (20 ml) were mixed, and into the resultant solution, a 0.5M 9-BBN/THF solution (100 ml) was dropped over a period of 1 hour, then, the mixture was stirred at room temperature for 12 hours. The reaction liquid was mixed with a compound M-9 (3.66 g), (diphenylphosphinoferrocene)palladium dichloride (PdCl₂ (dppf)) (0.82 g), THF (32 ml) and a 3M sodium hydroxide aqueous solution (27 ml), and the mixture was refluxed for 4 hours. After completion of the reaction, the resultant solution was cooled down to room temperature and hexane (40 ml) was added, then, 20 wt% hydrogen peroxide water (6 ml) was slowly dropped into this while cooling with ice, and the mixture was stirred for 1 hour. The reaction liquid was subjected to liquid separation, then, the organic layer was washed with ion exchanged water (50 ml) five times. The resultant organic layer was dried over sodium sulfate, then, purified by column chromatography using hexane as a developing solvent and using silica gel as a filler, to obtain a compound M-10 (2.6 g).

The compound M-9 was synthesized referring to WO1987/001383.
GC-MS: 253 [M]⁺

### <Synthesis Example 8> (Synthesis of compound M-11)

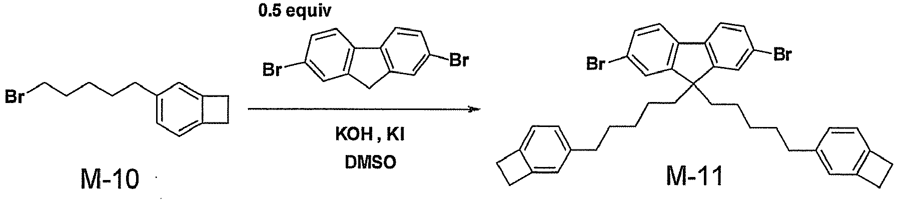

In a 50 ml three-necked flask under an argon atmosphere, 2,7-dibromofluorene(0.65 g), the compound M-10 (1.1 g), potassium hydroxide (0.48 g), potassium iodide (0.03 g) and dimethyl sulfoxide (5 ml) were mixed and the mixture was heated at 80°C for 4 hours. After completion of the reaction, the mixture was cooled down to room temperature, this was mixed with water (10 ml) and toluene (10 ml) and the mixture was subjected to liquid separation, then, the resultant organic layer was washed with a sodium chloride saturated aqueous solution (10 ml) five times. The resultant organic layer was dried over sodium sulfate, then, purified by column chromatography using hexane as a developing solvent and using silica gel as a filler, to obtain a compound M-11 (0.61 g).

LC-MS (APPI-MS (posi)): 668 [M]⁺
¹H-NMR (270 MHz, CDCl₃); δ=0.59 (tt, 4H), 1.08 (tt, 4H), 1.35 (tt, 4H), 1.89 (t, 4H), 2.38 (t, 4H), 3.11 (t, 8H), 6.77 (s, 2H), 6.89 (dd, 4H), 7.41-7.52 (m, 6H).

### <Synthesis Example 9> (Synthesis of polymer compound P-1)

First, a compound M-12 represented by the following formula: 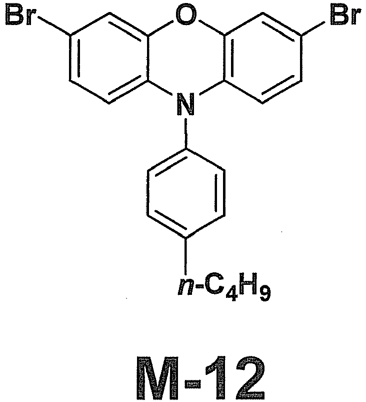 was synthesized by a method described in US 2004-0127666.

Next, under an inert atmosphere, 2,7-bis(1,3,2-dioxaborolan-2-yl)-9,9-dioctylfluorene (1.06 g), 2,7-dibromo-9,9-dioctylfluorene (0.66 g), the compound M-6 (0.10 g), the compound M-12 (0.28 g), palladium acetate (0.4 mg), tris(o-methoxyphenyl)phosphine (2.8 mg), trioctylmethylammonium chloride (trade name: Aliquat336, manufactured by Aldrich)(0.25 g) and toluene (40 ml) were mixed and heated at 105°C. Into the reaction liquid, a 2M sodium carbonate aqueous solution (11 ml) was dropped, and the mixture was refluxed for 4 hours. After the reaction, to this was added phenylboric acid (240 mg), and the mixture was further refluxed for 4 hours. Then, to this was added a 1.8M sodium diethyldithiacarbamate aqueous solution (10 ml), and the mixture was stirred at 80°C for 4 hours. After cooling down to room temperature, the mixture was washed with water (30 ml) three times, with a 3 wt% acetic acid aqueous solution (30 ml) three times and with water (30 ml) three times, and purified by passing through an alumina column and a silica gel column. The resultant toluene solution was dropped into methanol (300 ml), and the mixture was stirred for 1 hour, then, and the resultant solid was filtrated and dried, to obtain 0.9 g of a polymer compound P-1 represented by the following formula: 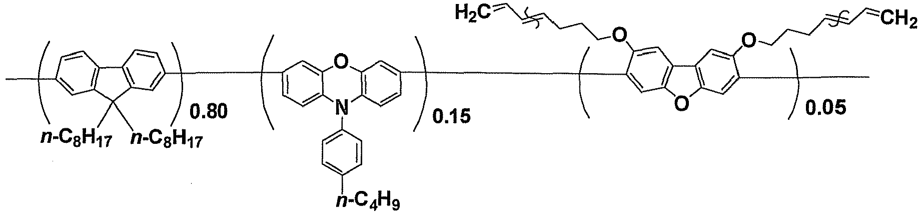 (wherein suffixes appended to the outside of parentheses represent the molar ratio of repeating units).
The polymer compound P-1 had a polystyrene-equivalent number-average molecular weight of 1.0×10⁵ and a polystyrene-equivalent weight-average molecular weight of 3.9×10⁵.

### <Synthesis Example 10> (Synthesis of polymer compound P-2)

Under an inert atmosphere, 2,7-bis(1,3,2-dioxaborolan-2-yl)-9,9-dioctylfluorene (1.06 g), bis(4-bromophenyl)-(4-sec-butylphenyl)-amine (0.37 g), the compound M-8 (0.44 g), the compound M-11(0.27 g), bistriphenylphosphinepalladium dichloride (1.4 mg), trioctylmethylammonium chloride (trade name: Aliquat336, manufactured by Aldrich) (0.25 g) and toluene (40 ml) were mixed and heated at 105°C. Into the reaction liquid, a 2M sodium carbonate aqueous solution (6 ml) was dropped, and the mixture was refluxed for 5 hours. After the reaction, phenylboric acid (240 mg) was added, and the mixture was further refluxed for 4 hours. Then, a 1.8M sodium diethyldithiacarbamate aqueous solution (10 ml) was added and the mixture was stirred at 80°C for 4 hours. After cooling down to room temperature, the mixture was washed with water (30 ml) three times, with a 3 wt% acetic acid aqueous solution(30 ml) three times and with water (30 ml) three times, and purified by passing through an alumina column and a silica gel column. The resultant toluene solution was dropped into methanol (300 ml), and the mixture was stirred for 1 hour, then, and the resultant solid was filtrated and dried, to obtain 0.8 g of a polymer compound P-2 represented by the following formula: 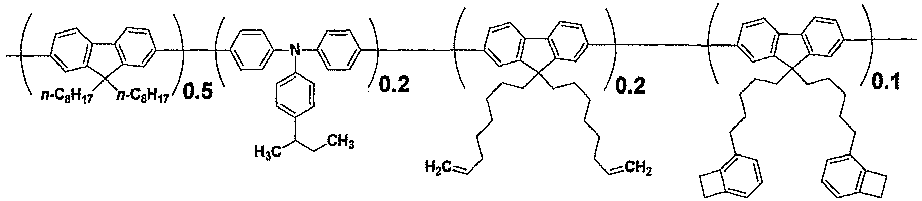 (wherein suffixes appended to the outside of parentheses represent the molar ratio of repeating units.).
The polymer compound P-2 had a polystyrene-equivalent number-average molecular weight of 4.3×10⁴ and a polystyrene-equivalent weight-average molecular weight of 2.1×10⁵.

### <Synthesis Example 11> (Synthesis of polymer compound P-3)

Under an inert atmosphere, 2,7-bis(1,3,2-dioxaborolan-2-yl)-9,9-dioctylfluorene (1.05 g), 2,7-dibromo-9,9-dioctylfluorene (0.77 g), the compound M-4 (0.31 g), bistriphenylphosphinepalladium dichloride (1.4 mg), trioctylmethylammonium chloride (trade name: Aliquat336, manufactured by Aldrich) (0.25 g) and toluene(40 ml) were mixed and heated at 105°C. Into the reaction liquid, a 2M sodium carbonate aqueous solution (6 ml) was dropped, and the mixture was refluxed for 20 hours. After the reaction, to this was added phenylboric acid (240 mg), and the mixture was further refluxed for 4 hours. Then, to this was added a 1.8M sodium diethyldithiacarbamate aqueous solution (10 ml), and the mixture was stirred at 80°C for 4 hours. The resultant reaction liquid was cooled down to room temperature, then, washed with water (30 ml) three times, with a 3 wt% acetic acid aqueous solution (30 ml) three times and with water (30 ml) three times, and purified by passing through an alumina column and a silica gel column. The resultant toluene solution was dropped into methanol (300 ml), and the mixture was stirred for 1 hour, then, the resultant solid was filtrated and dried, to obtain 0.8 g of a polymer compound P-3 represented by the following formula: 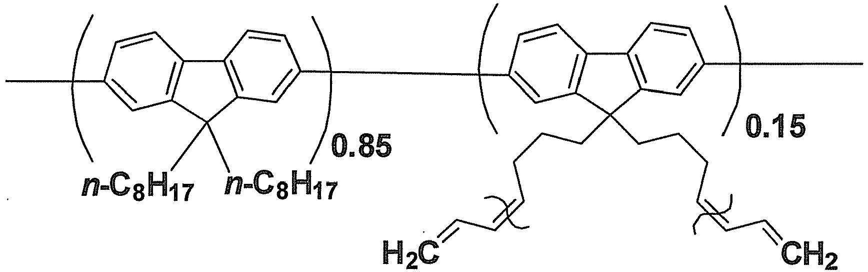 (wherein suffixes appended to the outside of parentheses represent the molar ratio of repeating units.).
The polymer compound P-3 had a polystyrene-equivalent number-average molecular weight of 4.1×10⁴ and a polystyrene-equivalent weight-average molecular weight of 1.3×10⁵.

### <Synthesis Example 12> (Synthesis of polymer compound CP-1)

First, a compound M-13 represented by the following formula: 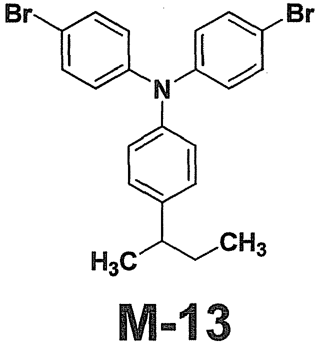 was synthesized by a method described in WO 2002-045184 and a compound M-15 represented by the following formula: 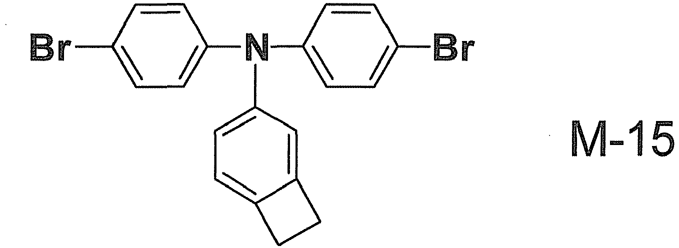 was synthesized by a method described in US 2004/035221.

Next, under an inert atmosphere, 2,7-bis(1,3,2-dioxaborolan-2-yl)-9,9-dioctylfluorene (1.06 g), the compound M-13 (0.87 g), the compound M-15 (0.04 g), bistriphenylphosphinepalladium dichloride (1.4m g), trioctylmethylammonium chloride (trade name: Aliquat336, manufactured by Aldrich) (0.25 g) and toluene(40 ml) were mixed and heated at 105°C. Into the resultant reaction liquid, a 2M sodium carbonate aqueous solution (6 ml) was dropped, and the mixture was refluxed for 7 hours. After the reaction, to this was added phenylboric acid (240 mg), and the mixture was further refluxed for 4 hours. Then, to this was added a 1.8M sodium diethyldithiacarbamate aqueous solution (10 ml), and the mixture was stirred at 80°C for 4 hours. After cooling down to room temperature, the mixture was washed with water (30 ml) three times, with a 3 wt% acetic acid aqueous solution (30 ml) three times and with water (30 ml) three times, and purified by passing through an alumina column and a silica gel column. The resultant toluene solution was dropped into methanol (300 ml), and the mixture was stirred for 1 hour, then, and the resultant solid was filtrated and dried, to obtain 0.8 g of a polymer compound CP-1 represented by the following formula: 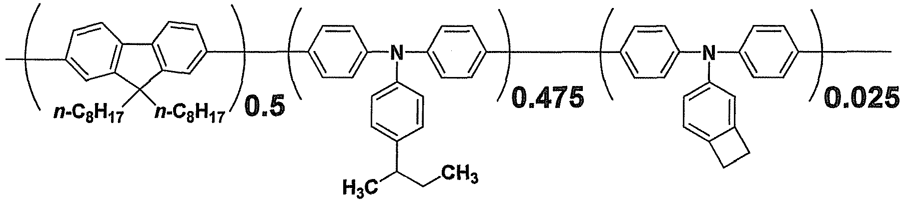 (wherein suffixes appended to the outside of parentheses represent the molar ratio of repeating units.).
The polymer compound CP-1 had a polystyrene-equivalent number-average molecular weight of 3.4×10⁴ and a polystyrene-equivalent weight-average molecular weight of 6.7×10⁴.

### <Synthesis Example 13> (Synthesis of polymer compound CP-2)

First, a compound M-14 represented by the following formula: 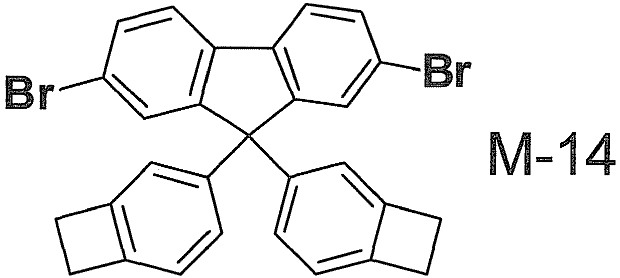 was synthesized by a method described in JP-A No. 2008-106241.

Next, under an inert atmosphere, 2,7-bis(1,3,2-dioxaborolan-2-yl)-9,9-dioctylfluorene (1.06 g), 2,7-dibromo-9,9-dioctylfluorene (0.22 g), the compound M-13 (0.55 g), the compound M-14 (0.21 g), bistriphenylphosphinepalladium dichloride (1.4m g), trioctylmethylammonium chloride (trade name: Aliquat336, manufactured by Aldrich) (0.25 g) and toluene (40 ml) were mixed and heated at 105°C. Into the reaction liquid, a 2M sodium carbonate aqueous solution (6 ml) was dropped, and the mixture was refluxed for 7 hours. After the reaction, to this was added phenylboric acid (240 mg), and the mixture was further refluxed for 4 hours. Then, to this was added a 1.8M sodium diethyldithiacarbamate aqueous solution (10 ml), and the mixture was stirred at 80°C for 4 hours. After cooling down to room temperature, the mixture was washed with water (30 ml) three times, with a 3 wt% acetic acid aqueous solution (30 ml) three times and with water (30 ml) three times, and purified by passing through an alumina column and a silica gel column. The resultant toluene solution was dropped into methanol (300 ml), and the mixture was stirred for 1 hour, then, and the resultant solid was filtrated and dried, to obtain a polymer compound CP-2 with a yielded amount of 0.9 g represented by the following formula: 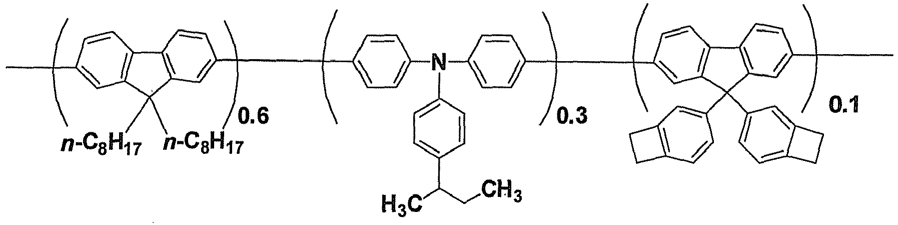 (wherein suffixes appended to the outside of parentheses represent the molar ratio of repeating units.).
The polymer compound CP-2 had a polystyrene-equivalent number-average molecular weight of 8.4×10⁴ and a polystyrene-equivalent weight-average molecular weight of 2.0×10⁵.

### <Synthesis Example 14> (Synthesis of polymer compound CP-3)

Under an inert atmosphere, 2,7-bis(1,3,2-dioxaborolan-2-yl)-9,9-dioctylfluorene (1.06 g), the compound M-8 (0.22 g), N,N-di(4-bromophenyl)aniline (0.73 g), bistriphenylphosphinepalladium dichloride (1.4 mg), trioctylmethylammonium chloride (trade name: Aliquat336, manufactured by Aldrich) (0.25 g) and toluene(40 ml) were mixed and heated at 105°C. Into the reaction solution, a 2M sodium carbonate aqueous solution (6 ml) was dropped, and the mixture was refluxed for 20 hours. After the reaction, to this was added phenylboric acid (240 mg), and the mixture was further refluxed for 4 hours. Then, to this was added a 1.8M sodium diethyldithiacarbamate aqueous solution (10 ml), and the mixture was stirred at 80°C for 4 hours. After cooling down to room temperature, the mixture was washed with water (30 ml) three times, with a 3 wt% acetic acid aqueous solution (30 ml) three times and with water (30 ml) three times, and purified by passing through an alumina column and a silica gel column. The resultant toluene solution was dropped into methanol (300 ml), and the mixture was stirred for 1 hour, then, and the resultant solid was filtrated and dried, to obtain 0.8 g of a polymer compound CP-3 represented by the following formula : 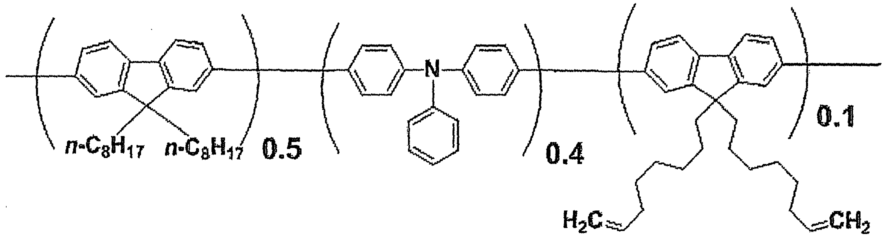 (wherein suffixes appended to the outside of parentheses represent the molar ratio of repeating units.).
The polymer compound CP-3 had a polystyrene-equivalent number-average molecular weight of 5.3×10⁴ and a polystyrene-equivalent weight-average molecular weight of 1.9×10⁵.

### <Synthesis Example 15> (Synthesis of polymer compound P-4)

Under an inert atmosphere, a compound MM-1 (7.28 g) represented by the following formula: 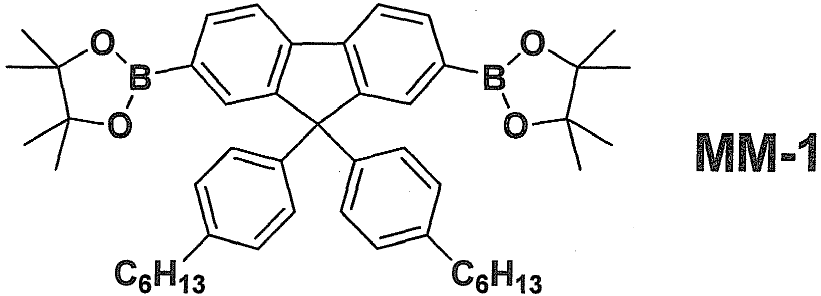 , 2,7-dibromo-9,9-dioctylfluorene (4.94 g), a compound MM-2 (0.74 g) represented by the following formula: 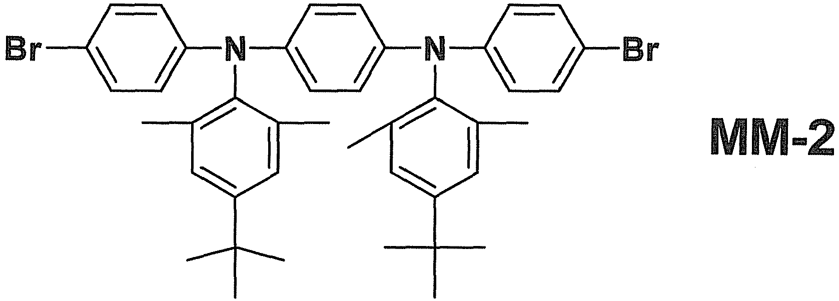 , bistriphenylphosphinepalladium dichloride (7.0 mg), trioctylmethylammonium chloride (trade name: Aliquat336, manufactured by Aldrich) (1.30 g) and toluene (100 ml) were mixed and heated at 105°C. Into the reaction solution, a 2M sodium carbonate aqueous solution (27 ml) was dropped, and the mixture was refluxed for 2 hours. After the reaction, to this was added phenylboric acid (120 mg), and the mixture was further refluxed for 4 hours. Then, to this was added a 1.8M sodium diethyldithiacarbamate aqueous solution (60 ml), and the mixture was stirred at 80°C for 4 hours. After cooling down to room temperature, the mixture was washed with water (130 ml) three times, with a 3 wt% acetic acid aqueous solution (130 ml) three times and with water (130 ml) three times, and purified by passing through an alumina column and a silica gel column. The resultant toluene solution was dropped into methanol (1.5 L), and the mixture was stirred for 1 hour, then, the resultant solid was filtrated and dried, to obtain 8.0 g of a polymer compound P-4 represented by the following formula: 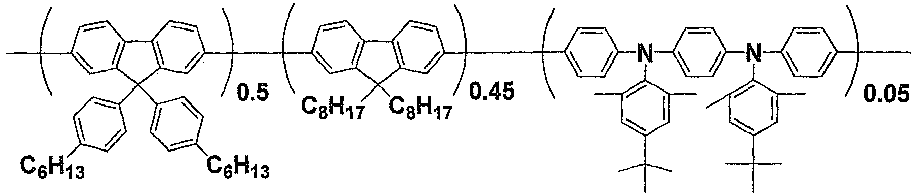 (wherein suffixes appended to the outside of parentheses represent the molar ratio of repeating units.).
The polymer compound P-4 had a polystyrene-equivalent number-average molecular weight of 5.1×10⁴ and a polystyrene-equivalent weight-average molecular weight of 1.4×10⁵. The compound MM-1 was synthesized by a method described in WO 2008/111658, and the compound MM-2 was synthesized by a method described in EP 1394188.

### <Example 1> (Preparation of liquid composition L-1)

The polymer compound P-1 (70 mg) and the compound M-14 (30 mg) were mixed and dissolved in xylene (10 g), to prepare a liquid composition L-1 having a composition concentration of about 1 wt%.

### <Example 2> (Preparation of liquid composition L-2)

The polymer compound P-2 (90 mg) and the compound M-6 (10 mg) were mixed and dissolved in xylene (10 g), to prepare a liquid composition L-2 having a composition concentration of about 1 wt%.

### <Reference Example 3> (Preparation of liquid composition L-3)

The polymer compound P-3 (50 mg) and the compound M-7 (50 mg) were mixed and dissolved in xylene (10 g), to prepare a liquid composition L-3 having a composition concentration of about 1 wt%.

### <Comparative Example 1> (Preparation of liquid composition CL-1)

The polymer compound CP-1 (100 mg) was dissolved in xylene (10 g), to prepare a liquid composition CL-1 having a composition concentration of about 1 wt%.

### <Comparative Example 2> (Preparation of liquid composition CL-2)

The polymer compound CP-2 (100 mg) was dissolved in xylene (10 g), to prepare a liquid composition CL-2 having a composition concentration of about 1 wt%.

### <Comparative Example 3> (Preparation of liquid composition CL-2)

The polymer compound CP-3 (100 mg) was dissolved in xylene (10 g), to prepare a liquid composition CL-3 having a composition concentration of about 1 wt%.

### <Measurement of film remaining ratio and evaluation thereof>

### •Evaluation of film remaining ratio on glass substrate

Any of liquid compositions L-1 to L-3 and CL-1 to CL-3 was dropped on a glass substrate, and coated by using a spin coater (trade name: MS-A100 type, manufactured by Misawa) to form a film under conditions of 1000 rpm and 15 seconds. The thickness of the resultant film (H₁) was measured by using a profiler (trade name: P-16+, manufactured by KLA-Tencor).

Next, in a glove box of which internal gas had been purged with nitrogen, the film on the above-described glass substrate was baked for 20 minutes at a baking temperature shown in Table 1 using a high power hot plate (trade name: HP-ISA, manufactured by AS ONE Corporation). The resultant film on the glass substrate was cooled down to room temperature, then, immersed in a xylene solution, then, rinsed by using a spin coater (trade name: MS-A100 type, manufactured by Misawa) under conditions of 1000 rpm and 15 seconds. The thickness of the fabricated film (H₂) was measured by using a profiler (trade name: P-16+, manufactured by KLA-Tencor).

The film remaining ratio was represented by (H₂)/(H₁), and the resultant results are shown in Table 1.

**Table 1**

| | liquid composition | number of moles of crosslinkable group in 1 g of composition | film remaining ratio | |
|---|---|---|---|---|
| | | | baked at 150°C | baked at 170°C |
| Example 1 | L-1 | 1.55×10⁻³ mol | 38% | 52% |
| Example 2 | L-2 | 1.47×10⁻³ mol | 89% | 94% |
| Reference Example 3 | L-3 | 2.63×10⁻³ mol | 41% | 57% |
| Comparative Example 1 | CL-1 | 1.74×10⁻⁴ mol | 0% | 0% |
| Comparative Example 2 | CL-2 | 6.59×10⁻⁴ mol | 0% | 0% |
| Comparative Example 3 | CL-3 | 4.20×10⁻⁴ mol | 0% | 38% |

### •Evaluation

The films fabricated using the liquid compositions L-1 to L-3 showed higher film remaining ratios as compared with the films fabricated using the liquid compositions CL-1 to CL-3, thus confirming excellent curability in a low temperature range (150°C). Further, it was confirmed that the films fabricated using the liquid compositions L-1 to L-3 had excellent curability also at 170°C.

### <Measurement of PL quantum yield of polymer compound >

(1) The polymer compound P-1 was dissolved in xylene, to prepare a 1.2 wt% solution PL-1.
(2) The polymer compound P-2 was dissolved in xylene, to prepare a 1.2 wt% solution PL-2.
(3) The polymer compound P-3 was dissolved in xylene, to prepare a 1.2 wt% solution PL-3.
(4) The low molecular weight compound M-14 was dissolved in xylene, to prepare a 3 wt% solution ML-1.
(5) The low molecular weight compound M-6 was dissolved in xylene, to prepare a 3 wt% solution ML-2.
(6) The low molecular weight compound M-7 was dissolved in xylene, to prepare a 3 wt% solution ML-3.
(7) Any of the solutions PL-1 to PL-3 and ML-1 to ML-3 was dropped on a glass substrate, and coated by using a spin coater (trade name: MS-A100 type, manufactured by Misawa) to form a film under conditions of 1000 rpm and 15 seconds, obtaining a film having a thickness of about 40 nm.

The PL quantum yields of the polymer compounds P-1 to P-3 and the low molecular weight compounds M-14, M-6 and M-7 were measured at an excitation wavelength of 325 nm using the films obtained from the solutions PL-1 to PL-3. The PL quantum yields of the films obtained from the solutions ML-1 to ML-3 were measured using an excitation wavelength of 250 nm. For measurement of the PL quantum yield, PL Quantum Yield Measurement System (c9920-02) manufactured by Hamamatsu Photonics, Japan was used. The resultant results are shown in Table 2.

**Table 2**

| solution | compound | PL quantum yield |
|---|---|---|
| PL-1 | polymer compound P-1 | 41% |
| PL-2 | polymer compound P-2 | 38% |
| PL-3 | polymer compound P-3 | 18% |
| ML-1 | low molecular weight compound M-14 | 8% |
| ML-2 | low molecular weight compound M-6 | 7% |
| ML-3 | low molecular weight compound M-7 | 6% |

### <Reference Example 4> (Fabrication of electroluminescent device and evaluation thereof)

### • Preparation of polymer compound P-4 solution

The polymer compound P-4 was dissolved in xylene, to prepare a solution PL-4 having a polymer concentration of 1.2 wt%.

### • Fabrication of electroluminescent device

On a glass substrate carrying thereon an ITO film having a thickness of 150 nm formed by a sputtering method, a solution obtained by filtrating a suspension of poly(3,4)ethylenedioxythiophene/polystyrenesulfonic acid (manufactured by Bayer, trade name: Baytron PAI4083) through a 0.2 µm membrane filter was spin-coated to form a film having a thickness of 70 nm which was then dried on a hot plate at 200°C for 10 minutes. Next, the liquid composition L-1 was spin-coated at a rotating speed of 1000 rpm to form a film, and the film was cured by heating on a hot plate at 170°C for 20 minutes. The thickness of the film after curing was about 30 nm. Further, the solution PL-4 was spin-coated at a rotating speed of 3000 rpm to form a film. The thickness of the film after curing was about 55 nm. Further, this was dried under reduced pressure at 130°C for 10 minutes, then, as a cathode, sodium fluoride was vapor-deposited with a thickness of about 5 nm, then, aluminum was vapor-deposited with a thickness of about 60 nm, fabricating an electroluminescent device. After the degree of vacuum reached 1×10⁻⁴ Pa or less, metal vapor deposition was initiated.

### •Evaluation of performance electroluminescent device

Voltage was applied on the resultant electroluminescent device, to obtain EL light emission having a peak at 450 nm. The maximum light emission efficiency of this electroluminescent device was 8.45 cd/A.

### INDUSTRIAL APPLICABILITY

The composition of the present invention is a composition showing excellent curability in a low temperature range (150°C).

## Claims

1. A composition comprising
(i) a crosslinkable polymer compound comprising a crosslinkable group and being capable of showing at least one of a light emitting property and charge transportability, and
(ii) a crosslinkable low molecular weight compound having a single molecular weight which is 1x10² or more and less than 1x10⁴, comprising a crosslinkable group and being capable of showing at least one of a light emitting property and charge transportability,
**characterised in that** one of said crosslinkable polymer compound and said crosslinkable low molecular weight compound has a group represented by any of formulae (Z-1) and (Z-2) as said crosslinkable group, and the other has a group represented by formula (Z-5) as said crosslinkable group: 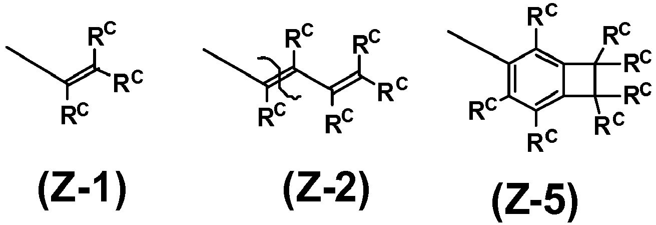 in the formulae (Z-1), (Z-2) and (Z-5), each R^{C} represents a hydrogen atom, an alkyl group, an alkoxy group, an alkylthio group, an aryl group, an aryloxy group, an arylthio group, an arylalkyl group, an arylalkoxy group, an arylalkylthio group, an amino group, a silyl group, a halogen atom, an acyl group, an acyloxy group, an imine residue, a carbamoyl group, an acid imide group, a monovalent heterocyclic group, a carboxyl group, a cyano group or a nitro group, and the group represented by R^{C} may have a substituent, wherein the plurality of R^{C} may be the same or different.

2. The composition according to claim 1, wherein said crosslinkable low molecular weight compound is an aromatic hydrocarbon.

3. The composition according to claim 1, wherein said crosslinkable low molecular weight compound is a heterocyclic compound.

4. The composition according to any one of claims 1 to 3, wherein said crosslinkable polymer compound has, as a repeating unit, at least one member selected from the group consisting of an arylene group comprising 1 to 4 groups represented by the following formula (I) and optionally comprising a substituent, a divalent heterocyclic group comprising 1 to 4 groups represented by the following formula (I) and optionally comprising a substituent and a divalent aromatic amine residue comprising 1 to 4 groups represented by the following formula (I) and optionally comprising a substituent: 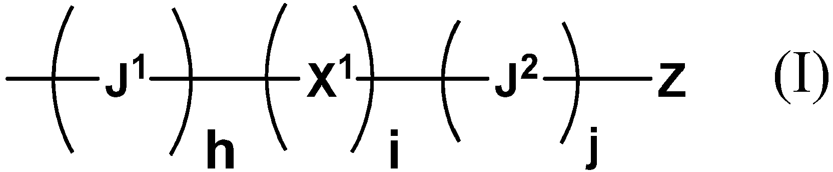 in the formula (I), Z represents a group represented by any of said formulae (Z-1), (Z-2) and (Z-5), J¹ represents a phenylene group optionally comprising a substituent, J² represents an alkylene group optionally comprising a substituent, X¹ represents an oxygen atom or a sulfur atom, h and i are each independently 0 or 1, and j is an integer of 0 to 3.

5. The composition according to any one of claims 1 to 4, wherein the weight ratio of said crosslinkable polymer compound to said crosslinkable low molecular weight compound is 99:1 to 50:50.

6. The composition according to any one of claims 1 to 5, wherein the total amount of said crosslinkable groups contained in one gram of the composition is 1.0×10⁻⁶ to 1.0×10⁻² mol.

7. The composition according to any one of claims 1 to 6, wherein the composition further comprises a solvent.

8. A film; or
an organic transistor; or
an organic photoelectric device; or
a light emitting device comprising electrodes consisting of an anode and a cathode and a layer that is disposed between the electrodes;
wherein the film, the organic transistor, the organic photoelectric device, or the layer comprise the composition according to any one of claims 1 to 7.

9. A surface light source or a display comprising the light emitting device according to claim 8.

## Patentansprüche

1. Eine Zusammensetzung, umfassend:
(i) eine vernetzbare Polymerverbindung, welche eine vernetzbare Gruppe umfasst und dazu in der Lage ist, mindestens eine von einer lichtemittierenden Eigenschaft sowie Ladungstransport aufzuweisen und
(ii) eine vernetzbare Verbindung mit niedrigem Molekulargewicht, welche ein Einzelmolekulargewicht von 1×10² oder mehr und weniger als 1×10⁴ aufweist, und welche eine vernetzbare Gruppe aufweist und dazu in der Lage ist, mindestens eine von einer lichtemittierenden Eigenschaft sowie Ladungstransport aufzuweisen,
**dadurch gekennzeichnet, dass** eine der vernetzbaren Polymerverbindung und der Verbindung mit einem niedrigen Molekulargewicht eine Gruppe dargestellt durch eine der Formeln (Z-1) und (Z-2) als vernetzbare Gruppe aufweist und die andere eine Gruppe dargestellt durch Formel (Z-5) als vernetzbare Gruppe aufweist: 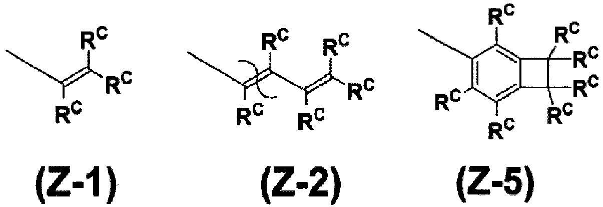 in den Formeln (Z-1), (Z-2) und (Z-5) stellt jedes R^{C}
ein Wasserstoffatom, eine Alkylgruppe, eine Alkoxygruppe, eine Alkylthiogruppe, eine Arylgruppe, eine Aryloxygruppe, eine Arylthiogruppe, eine Arylalkylgruppe, eine Arylalkoxygruppe, eine Arylalkylthiogruppe, eine Aminogruppe, eine Silylgruppe, ein Halogenatom, eine Acylgruppe, eine Acyloxygruppe, einen Iminrest, eine Carbamoylgruppe, eine Säureimidgruppe, eine monovalente heterocyclische Gruppe, eine Carboxylgruppe, eine Cyanogruppe oder eine Nitrogruppe dar, und die Gruppe dargestellt durch R^{C} kann einen Substituenten aufweisen, wobei die Vielzahl von R^{C} gleich oder verschieden sein kann.

2. Die Zusammensetzung gemäß Anspruch 1, wobei die vernetzbare Verbindung mit einem niedrigen Molekulargewicht ein aromatischer Kohlenwasserstoff ist.

3. Die Zusammensetzung gemäß Anspruch 1, wobei die vernetzbare Verbindung mit einem niedrigen Molekulargewicht eine heterocyclische Verbindung ist.

4. Die Zusammensetzung gemäß einem der Ansprüche 1 bis 3, wobei die vernetzbare Polymerverbindung, als eine Wiederholungseinheit mindestens ein Mitglied aufweist, ausgewählt aus der Gruppe bestehend aus einer Arylengruppe, welche 1 bis 4 Gruppen umfasst, die durch nachstehende Formel (I) dargestellt sind, und die gegebenenfalls einen Substituenten umfasst, einer divalenten heterocyclischen Gruppe, welche 1 bis 4 Gruppen umfasst, die durch nachstehende Formel (I) dargestellt sind, und die gegebenenfalls einen Substituenten umfasst, und einem divalenten aromatischen Aminrest, welcher 1 bis 4 Gruppen umfasst, die durch nachstehende Formel (I) dargestellt sind, und der gegebenenfalls einen Substituenten umfasst: 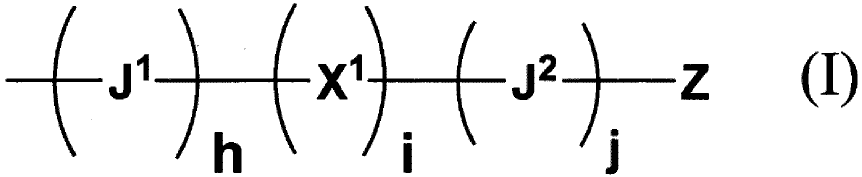 in der Formel (I) stellt Z eine Gruppe dar, dargestellt durch eine der Formeln (Z-1), (Z-2) und (Z-5), J¹ stellt eine Phenylengruppe dar, welche gegebenenfalls einen Substituenten umfasst, J² stellt eine Alkylengruppe dar, welche gegebenenfalls einen Substituenten umfasst, X¹ stellt ein Sauerstoffatom oder ein Schwefelatom dar, h und i sind jeweils unabhängig 0 oder 1 und j ist eine ganze Zahl von 0 bis 3.

5. Die Zusammensetzung gemäß einem der Ansprüche 1 bis 4, wobei das Gewichtsverhältnis der vernetzbaren Polymerverbindung zur vernetzbaren Verbindung mit einem niedrigen Molekulargewicht 99:1 zu 50:50 beträgt.

6. Die Zusammensetzung gemäß einem der Ansprüche 1 bis 5, wobei die Gesamtmenge an vernetzbaren Gruppen, welche in einem Gramm der Zusammensetzung enthalten sind, 1,0×10⁻⁶ bis 1,0×10⁻² Mol beträgt.

7. Die Zusammensetzung gemäß einem der Ansprüche 1 bis 6, wobei die Zusammensetzung ferner ein Lösungsmittel umfasst.

8. Ein Film; oder
ein organischer Transistor; oder
ein organisches photoelektrisches Element; oder
ein lichtemittierendes Element, welches Elektroden, bestehend aus einer Anode und einer Kathode und einer Schicht, welche zwischen den Elektroden angeordnet ist, umfasst;
wobei der Film, der organische Transistor, das organische photoelektrische Element oder die Schicht die Zusammensetzung gemäß einem der Ansprüche 1 bis 7 umfassen.

9. Eine Oberflächenlichtquelle oder ein Display, welche das lichtemittierende Element gemäß Anspruch 8 umfassen.

## Revendications

1. Composition comprenant
(i) un composant de polymère réticulable comprenant un groupe réticulable et capable de présenter au moins l'une d'une propriété électroluminescente et d'une capacité de transport de charge, et
(ii) un composé réticulable de poids moléculaire faible présentant un poids moléculaire unitaire égal ou supérieur à 1x10² et inférieur à 1x10⁴, comprenant un groupe réticulable et capable de présenter au moins l'une d'une propriété électroluminescente et d'une capacité de transport de charge,
**caractérisé en ce que** l'un desdits composé de polymère réticulable et composé réticulable de poids moléculaire faible présente un groupe représenté par l'une quelconque des formules (Z-1) et (Z-2) comme ledit groupe réticulable, et l'autre présente un groupe représenté par la formule (Z-5) comme ledit groupe réticulable : 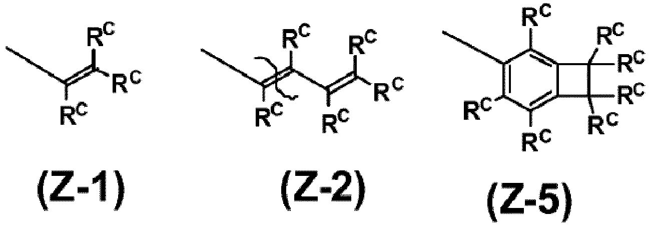 dans les formules (Z-1), (Z-2) et (Z-5), chaque R^{c} représente un atome d'hydrogène, un groupe alkyle, un groupe alcoxy, un groupe aklylthio, un groupe aryle, un groupe aryloxy, un groupe arylthio, un groupe arylalkyle, un groupe arylalcoxy, un groupe arylalkylthio, un groupe amino, un groupe silyle, un atome halogène, un groupe acyle, un groupe acyloxy, un résidu imine, un groupe carbamoyle, un groupe acide imide, un groupe hétérocyclique monovalent, un groupe carboxyle, un groupe cyano ou un groupe nitro, et le groupe représenté par R^{c} peut avoir un substituant, la pluralité de R^{c} pouvant être similaires ou différents.

2. Composition selon la revendication 1, dans laquelle ledit composé réticulable de poids moléculaire faible est un hydrocarbone aromatique.

3. Composition selon la revendication 1, dans laquelle ledit composé réticulable de poids moléculaire faible est un composé hétérocyclique.

4. Composition selon l'une quelconque des revendications 1 à 3, dans laquelle ledit composé de polymère réticulable présente, en tant qu'unité répétitive, au moins un élément choisi dans le groupe constitué par un groupe arylène comprenant 1 à 4 groupes représentés par la formule (I) suivante et comprenant éventuellement un substituant, un groupe hétérocyclique divalent comprenant 1 à 4 groupes représentés par la formule (I) suivante et comprenant éventuellement un substituant et un résidu amine aromatique divalent comprenant 1 à 4 groupes représentés par la formule (I) suivante et comprenant éventuellement un substituant : 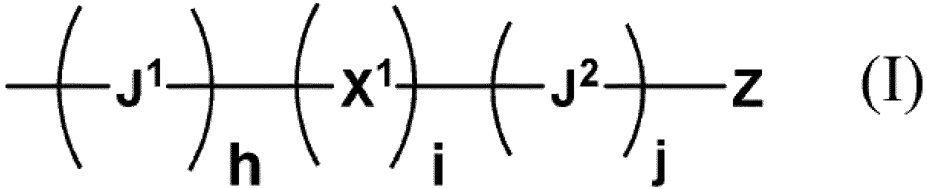 dans la formule (I), Z représente un groupe représenté par l'une quelconque desdites formules (Z-1), (Z-2) et (Z-5), J¹ représente un groupe phénylène comprenant éventuellement un substituant, J² représente un groupe alkylène comprenant éventuellement un substituant, X¹ représente un atome d'oxygène ou un atome de soufre, h et i valent chacun indépendamment 0 ou 1, et j est un entier de 0 à 3.

5. Composition selon l'une quelconque des revendications 1 à 4, dans laquelle le rapport pondéral dudit composé de polymère réticulable au composé réticulable de poids moléculaire faible est de 99:1 à 50:50.

6. Composition selon l'une quelconque des revendications 1 à 5, dans laquelle la quantité totale desdits groupes réticulables contenus dans un gramme de la composition est de 1,0 x 10⁻⁶ à 1,0 x 10⁻² mol.

7. Composition selon l'une quelconque des revendications 1 à 6, laquelle composition comprend en outre un solvant.

8. Film ; ou
transistor organique ; ou
dispositif photoélectrique organique ; ou
dispositif électroluminescent comprenant des électrodes constitué d'une anode et d'une cathode et d'une couche placée entre les électrodes ;
le film, le transistor organique, le dispositif photoélectrique organique ou la couche comprenant la composition selon l'une quelconque des revendications 1 à 7.

9. Source de lumière de surface ou affichage comprenant le dispositif électroluminescent selon la revendication 8.
